(19) Europäisches Patentamt
European Patent Office
Office européen des brevets

(11) Publication number: **0 170 073**
**A1**

(12) **EUROPEAN PATENT APPLICATION**

(21) Application number: 85108132.3

(22) Date of filing: 01.07.85

(51) Int. Cl.[4]: C 07 D 487/04
A 61 K 31/40, C 07 D 519/00

(30) Priority: 02.07.84 US 626821

(43) Date of publication of application:
05.02.86 Bulletin 86/6

(84) Designated Contracting States:
AT BE CH DE FR GB IT LI LU NL SE

(71) Applicant: MERCK & CO. INC.
126, East Lincoln Avenue P.O. Box 2000
Rahway New Jersey 07065(US)

(72) Inventor: Christensen, Burton G.
770 Anderson Avenue, Apt. 19H
Cliffside Park New Jersey 07010(US)

(72) Inventor: Johnston, David B.R.
53 Round Top Road
Warren New Jersey 07060(US)

(72) Inventor: Schmitt, Susan M.
50 M Southwyck Village
Scotch Plains New Jersey 07076(US)

(74) Representative: Abitz, Walter, Dr.-Ing. et al,
Abitz, Morf, Gritschneder, Freiherr von Wittgenstein
Postfach 86 01 09
D-8000 München 86(DE)

(54) **1-Methylcarbapenems having an externally alkylated mono- or bicyclic 2-quaternary heteroarylalkylthio substituent.**

(57) Carbapenems having the formula:

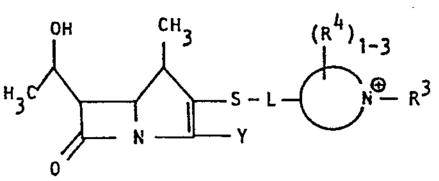

wherein

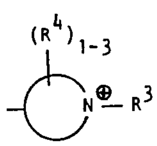

is a mono-or bicyclic quaternary heteroaryl, their preparation and antibiotic use are disclosed.

TITLE OF THE INVENTION

1-METHYLCARBAPENEMS HAVING AN EXTERNALLY ALKYLATED MONO- OR BICYCLIC 2-QUATERNARY HETEROARYLALKYLTHIO SUBSTITUENT

BACKGROUND OF THE INVENTION

The present invention is concerned with carbapenems antibiotics having a quaternary mono- or bicyclic heteroaryl containing group in the 2-position and a methyl group in the 1-position.

Thienamycin is a known carbapenem, broad spectrum antibiotic of the formula:

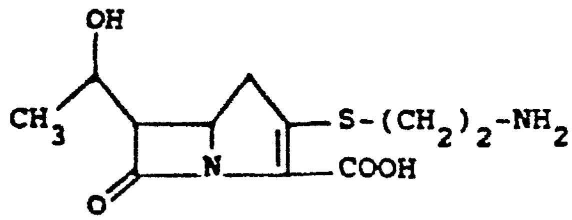

A

Other derivatives of A are also known.

The present externally alkylated mono- or bicyclic 2-quaternary heteroarylalkylthio substituted carbapenems have an antibiotic spectrum equal to or better than A. The present carbapenems also are more resistant than A to degradation by the dehydropeptidase enzyme DHP-I. The present carbapenems are also more resistant to DHP-I mediated degradation and are chemically more stable, both in solution and in the solid state, than the corresponding carbapenems lacking the 1-methyl substituent.

SUMMARY OF THE INVENTION

Carbapenems having the formula:

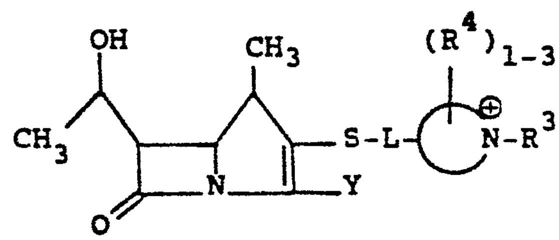

I

wherein $R^3$ is a quaternizing substitutent, $R^4$ is a ring hydrogen or substituent, L is a covalent bond or a bridging group, 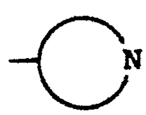 is mono- or bicyclic heteroaryl, and

Y is a carboxy containing substituent.

DETAILED DESCRIPTION OF THE INVENTION

The invention is embodied in a compound having the formula:

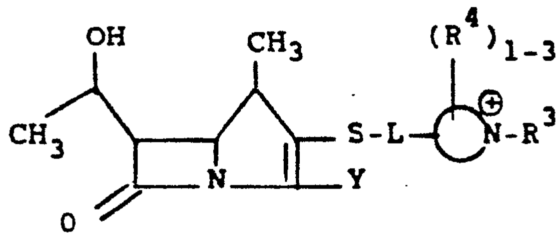

I

wherein:

L is a covalent bond or a bridging group selected from $-(CH_2)_{1-4}S-$; $-(CH_2)_{1-4}-O-$; $-(CH_2)_{1-4}-X-(CH_2)_{1-4}$ where X=O, S, NH, or $N(C_1-C_6)$alkyl; substituted or unsubstituted $C_1-C_4$ straight, $C_1-C_6$ branched or $C_3-C_7$ cycloalkyl groups wherein the substituents are selected from $C_1-C_6$ alkyl, $O-C_1-C_6$ alkyl, $S-C_1-C_6$ alkyl, halo, OH, $CF_3$, CN, $NH_2$, $NHC_1-C_6$ alkyl, $N(C_1-C_6$ alkyl$)_2$, $CO_2H$, $CONH_2$, CONH ($C_1-C_6$ alkyl), and $CON(C_1-C_6$ alkyl$)_2$;

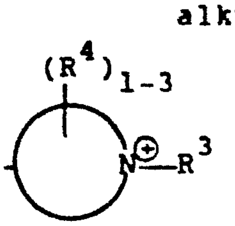
is a mono- or bicyclic heteroarylium group containing from 5-11 ring atoms of which up to 5 are heteroatoms wherein $R^3$ is:

1) an unsubstituted or substituted $C_1-C_6$ alkyl radical;

2) an unsubstituted or substituted $C_1-C_6$ alkenyl radical;

3) an unsubstituted or substituted $C_1$-$C_6$ alkynyl radical;

4) a $C_3$-$C_7$ cycloalkyl radical in which the ring is substituted or unsubstituted and one or more atoms may be replaced by a heteroatom;

5) a $C_3$-$C_7$ cycloalkyl methyl radical in which the ring may be substituted and one or more atoms may be replaced by a heteroatom;

6) an unsubstituted or substituted $C_5$-$C_7$ cycloalkenyl radical;

7) an unsubstituted or substituted bivalent $C_2$-$C_6$ alkylidene radical, optionally interrupted by a heteroatom, and joined to the heteroarylium group to form a ring which is carbocyclic or in which one or more atoms is replaced by a heteroatom. The new ring may contain one or more double bonds;

8) an unsubstituted or substituted phenyl or heteroaryl radical;

9) an unsubstituted or substituted phenyl ($C_1$-$C_4$ alkyl) or heteroaryl ($C_1$-$C_4$ alkyl) radical;

10) a cyano ($C_1$-$C_4$ alkyl) radical;

11) a carbamoyl ($C_1$-$C_4$ alkyl) radical;

12) a hydroxy ($C_1$-$C_4$ alkyl) radical;

13) an amino ($C_1$-$C_4$ alkyl) radical in which the nitrogen atom is unsubstituted or substituted with one to three $C_1$-$C_4$ alkyl groups;

14) an acidic side-chain of the structure

$-(CH_2)_n-X-(CH_2)_m-Y-A$ where:

n = 0-4

m = 0-4

X = $CHR^5$, CH=CH, phenylene ($-C_6H_4-$), NH, N(C1-C4 alkyl), O, S, S=O, C=O, $SO_2$, $SO_2NH$, $CO_2$, CONH, $OCO_2$, OC=O, NHC=O;

$R^5$ = H, O(C1-C4 alkyl), $NH_2$, NH(C1-C4 alkyl), N(C1-C4 alkyl)$_2$, CN, $CONH_2$, CON(C1-C4 alkyl)$_2$, $CO_2H$, $SO_2NH_2$, $SO_2$NH(C1-C4 alkyl);

Y = single bond, NH, N(C1-C4 alkyl), O, S;

A = an acidic function such as carboxy ($CO_2H$), phosphono [$P=O(OH)_2$], alkylphosphono {P=O(OH)-[C($C_1$-$C_4$ alkyl)]}, alkylphosphinyl [P=O(OH)-($C_1$-$C_4$ alkyl)], substituted phosphoramido [P=O(OH)NH($C_1$-$C_4$ alkyl) and $P=O(OH)NHR^x$], sulfino ($SO_2H$), sulfo ($SO_3H$), 5-tetrazolyl ($CN_4H$), arylsulfonamido ($SO_2NHR^x$) and acylsulfonamides represented by the structures $CONHSO_2$($C_1$-$C_4$ alkyl), $CONHSO_2N$($C_1$-$C_4$alkyl)$_2$ - $SO_2NHCO$($C_1$-$C_4$ alkyl) and $SO_2NHCOR^x$;

$R^x$ = aryl or heteroaryl as defined above;

wherein the substituents in the above definitions of $R^3$ are independently selected from the group consisting of the definitions of $R^4$ set out below;

$R^4$ is independently selected from:

a) a trifluoromethyl group;

b) a halogen atom;

c) an unsubstituted or substituted $C_1$-$C_4$ alkoxyl radical;

d) a hydroxy group;

e) an unsubstituted or substituted ($C_1$-$C_6$ alkyl) carbonyloxy radical;

f) a carbamoyloxy radical which is unsubstituted or substituted on nitrogen with one or two $C_1$-$C_4$ alkyl groups;

g) a $C_1$-$C_6$ alkylthio radical, $C_1$-$C_6$ alkylsulfinyl radical or $C_1$-$C_6$ alkylsulfonyl radical, each of which is unsubstituted or substituted on the alkyl group;

h) a sulfamoyl group which is unsubstituted or substituted on nitrogen by one or two $C_1$-$C_4$ alkyl groups;

i) an amino group;

j) a mono ($C_1$-$C_4$ alkyl) amino or di($C_1$-$C_4$ alkyl)amino group, each of which is unsubstituted or substituted on the alkyl group;

k) a formylamino group;

l) an unsubstituted or substituted ($C_1$-$C_6$ alkyl)carbonylamino radical;

m) a ($C_1$-$C_4$ alkoxyl) carbonylamino radical;

n) a ureido group in which the terminal nitrogen is unsubstituted or substituted with one or two $C_1$-$C_4$ alkyl groups;

o) a ($C_1$-$C_6$ alkyl)sulfonamido group;

p) a cyano group;

q) a formyl or acetalized formyl radical;

r) an unsubstituted or substituted ($C_1$-$C_6$ alkyl)carbonyl radical wherein the carbonyl is free or acetalized;

s) an unsubstituted or substituted phenylcarbonyl or heteroarylcarbonyl radical;

t) a hydroximinomethyl radical in which the oxygen or carbon atom is optionally substituted by a $C_1$-$C_4$ alkyl group;

u) a ($C_1$-$C_6$ alkoxy)carbonyl radical;

v) a carbamoyl radical which is unsubstituted or substituted on nitrogen by one or two $C_1$-$C_4$ alkyl groups;

w) an N-hydroxycarbamoyl or N($C_1$-$C_4$ alkoxy)carbamoyl radical in which the nitrogen atom may be additionally substituted by a $C_1$-$C_4$ alkyl group;

x) a thiocarbamoyl group;

y) an amidino group 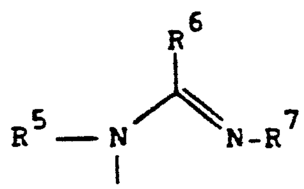

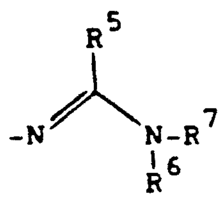

where $R^5$, $R^6$ and $R^7$ are independently hydrogen, $C_1$-$C_4$alkyl or wherein two of the alkyl groups together form a $C_2$-$C_6$alkylidene radical optionally interrupted by a heteroatom and joined together to form a ring;

z) a carboxamidino group 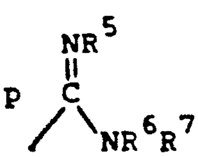 , where $R^5$, $R^6$ and $R^7$ are as defined above;

aa) a guanidinyl group where $R^6$ in ab) above is $NR^8R^9$ and $R^8$ and $R^9$ are as defined for $R^5$ through $R^7$ above.

ab) hydrogen;

ac) an unsubstituted or substituted $C_1$-$C_6$ alkyl radical;

ad) an unsubstituted or substituted $C_1$-$C_6$ alkenyl radical;

ae) an unsubstituted or substituted $C_1$-$C_6$ alkynyl radical;

af) a $C_3$-$C_7$ cycloalkyl radical in which the ring is substituted or unsubstituted and one or more atoms may be replaced by a heteroatom;

ag) a $C_3$-$C_7$ cycloalkyl methyl radical in which the ring may be substituted and one or more atoms may be replaced by a heteroatom;

ah) an unsubstituted or substituted $C_5$-$C_7$ cycloalkenyl radical;

ai) an unsubstituted or substituted phenyl or heteroaryl radical;

aj) an unsubstituted or substituted phenyl ($C_1$-$C_4$ alkyl) or heteroaryl ($C_1$-$C_4$ alkyl) radical; and

ak) an acidic side-chain of the structure

$-A$ or $-(CH_2)_n-X-(CH_2)_m-Y-A$ where:

n = 0-4

m = 0-4

X = $CHR^5$, CH=CH, phenylene ($-C_6H_4-$), NH, N(C1-C4 alkyl), O, S, S=O, C=O, $SO_2$, $SO_2NH$, $CO_2$, CONH, $OCO_2$, OC=O, NHC=O;

$R^5$ = H, O(C1-C4 alkyl), $NH_2$, NH(C1-C4 alkyl), N(C1-C4 alkyl)$_2$, CN, $CONH_2$, CON(C1-C4 alkyl)$_2$, $CO_2H$, $SO_2NH_2$, $SO_2NH$(C1-C4 alkyl);

Y = single bond, NH, N(C1-C4 alkyl), O, S;

A = an acidic function such as carboxy ($CO_2H$), phosphono [$P=O(OH)_2$], alkylphosphono {$P=O(OH)-[C(C_1-C_4$ alkyl)]}, alkylphosphinyl [$P=O(OH)-(C_1-C_4$ alkyl)], substituted phosphoramido [$P=O(OH)NH(C_1-C_4$ alkyl) and $P=O(OH)NHR^x$], sulfino ($SO_2H$), sulfo ($SO_3H$), 5-tetrazolyl ($CN_4H$), arylsulfonamido ($SO_2NHR^x$) and acylsulfonamides represented by the structures $CONHSO_2(C_1-C_4$ alkyl), $CONHSO_2N(C_1-C_4alkyl)_2$ - $SO_2NHCO(C_1-C_4$ alkyl) and $SO_2NHCOR^x$;

$R^x$ = aryl or heteroaryl as defined above;

Y is selected from:
i) COOH or a pharmaceutically acceptable ester or salt thereof,
ii) $COOR^1$ wherein $R^1$ is a removable carboxy protecting group,
iii) COOM wherein M is an alkali metal, or
iv) $COO^{\ominus}$; provided that when Y is other than iv) a counterion $Z^{\ominus}$ is provided.

As used herein, the term "heteroatom" means nitrogen, oxygen, or sulfur, independently selected where more than one heteroatom is involved.

Representative L groups are $-CH_2-$, $-CH(CH_3)-$, $-CH(C_2H_5)-$, $-(CH_2)_{2-4}$, $-CH(CH_3)-CH_2-$, $CH_2-CH(OCH_3)-$, $-CH(CH_3)-(CH_2)_2-$, $-CH(CH_2OH)-CH_2-$, $-CH(CF_3)-CH_2-$, $-CH_2-CH_2-S$, $-CH_2-CH_2-O$, $-(CH_2)_2-S-CH_2-$, $-(CH_2)_2-O-CH_2$, a single covalent bond, and the like.

A preferred L group is a substituted or unsubstituted $C_1$-$C_6$ linear or branched chain alkyl. A more preferred L group is $-CH_2-$, $-CH(CH_3)-$ or $(CH_2)_2-$.

Examples of useful $R^3$ groups are $-CH_3$, $-(CH_2)_{1-3}-CH_3$, $-CH_2-$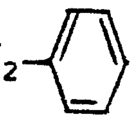, $-(CH_2)_{1-3}-O-CH_3$, $-CH_2-CN$, $CH_2-COOC_1-C_3$ alkyl, $-(CH_2)_2-N(C_1-C_3\text{ alkyl})_2$, $-CH_2-COOH$, $-(CH_2)_2-SO_3H$, $-CH_2$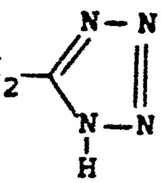, $-(CH_2)_2-\overset{\oplus}{N}(CH_3)_3$ and the like.

Preferred $R^3$ groups are the $C_1$-$C_6$ alkyls, both substituted and unsubstituted.

Preferred substituents are CN, $CON(CH_3)_2$, $CONH_2$, $SOCH_3$, $SO_2CH_3$, $CO_2H$, $SO_3H$, $SO_2NH_2$ and

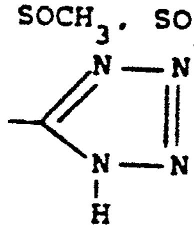

Examples of useful $R^4$ groups are OH, $NH_2$, $N(C_1-C_3\text{alkyl})$, $OC_1-C_4$alkyl, $C_1-C_4$alkyl, CN, $CF_3$, $CH_2OH$ and the like.

Preferred $R^4$ groups are $CO_2H$, $CH_2CO_2H$, $SO_3H$, $CH_2SO_3H$, $CONH_2$, $CH_2CONH_2$, CN, $CH_2CN$, $SO_2NH_2$

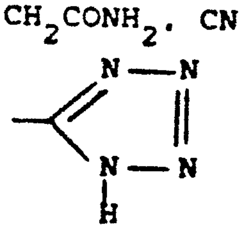 , $CH_2P(O)(OCH_3)$ and the like.
HO

The 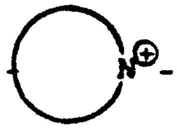- moiety is mono- or bicyclic quaternary heteroaryl group having 5-11 ring atoms of which, in addition to the quaternary $N^{\oplus}$, up to four can be heteroatoms.

Of particular interest and the most preferred group are compounds of the present invention wherein the substituent on the N-containing mono- or bicyclic quaternary heteroaryl group in the 2-position is an acidic function as defined above and the Y substituent in the 3-position is $COO^{\ominus}$ as defined above, thus forming a zwitterion with the positive charge of the quaternary nitrogen. The acidic function is anionic and the compounds are thus anionic zwitterions, i.e., they have a net negative charge. This novel characteristic has been found to result in at least one surprising and important improvement in the biological properties of the compounds reduced CNS side-effects. A more particular group of the compounds, those wherein the acidic function is a sulfoalkyl group of the formula $(C_{1-4}$ alkyl$)SO_3^{\ominus}$, have been found to have the additional surprising and important biological property of enhanced potency against <u>Pseudomonas</u> species, an especially important nosocomial pathogen. In this most preferred group of compounds, it is preferred that the N-containing mono- or bicyclic quaternary heteroaryl group in the 2-position is pyridinium.

Examples of useful -S-L-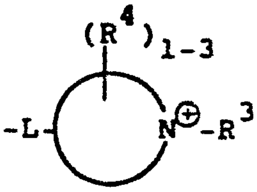 groups are:

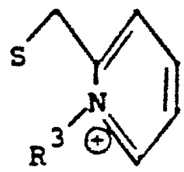
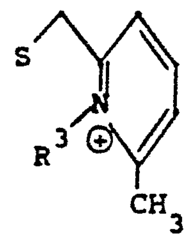
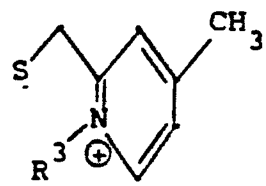
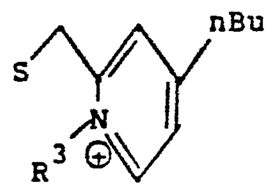

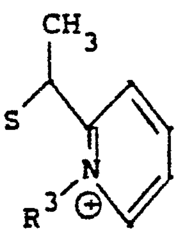
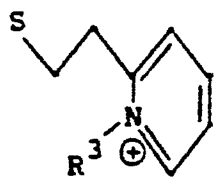
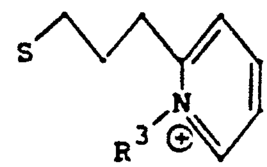

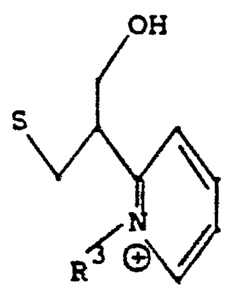
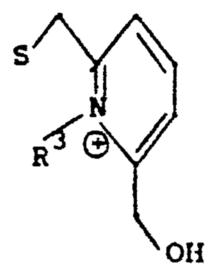
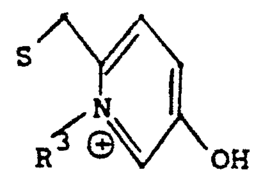
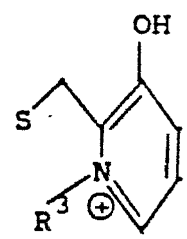

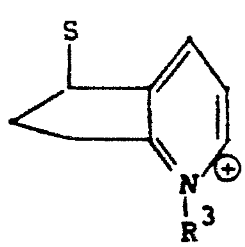
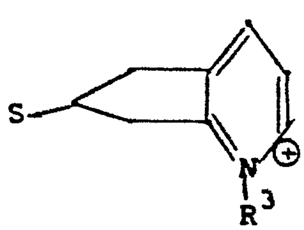
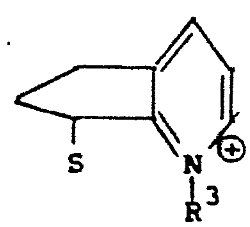

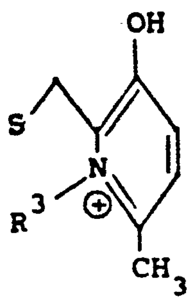
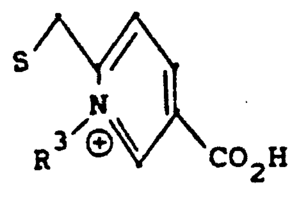
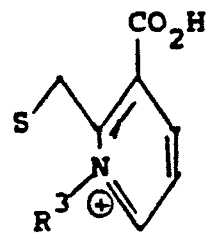
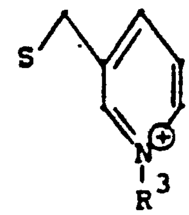

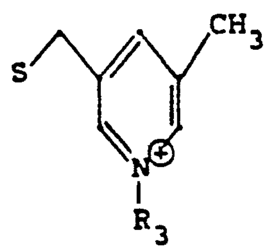
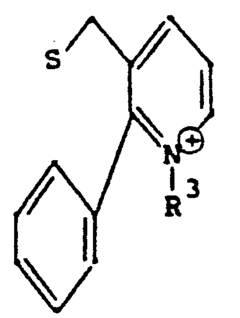
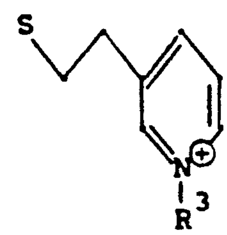
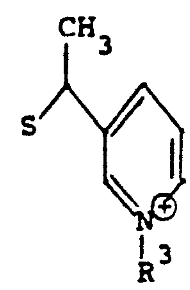

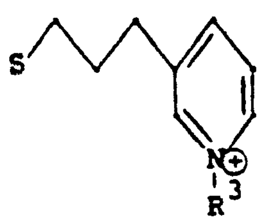
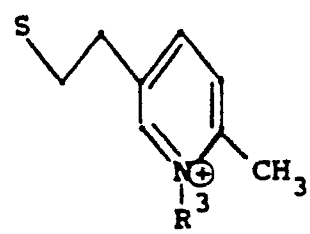
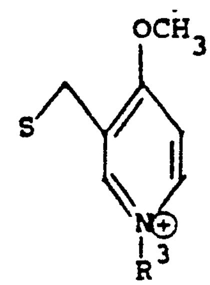

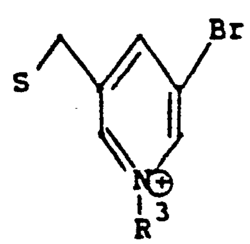
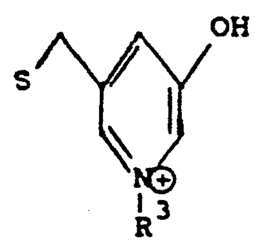
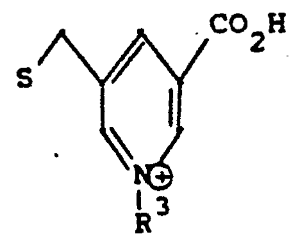

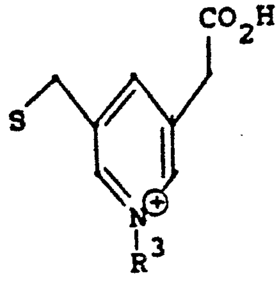
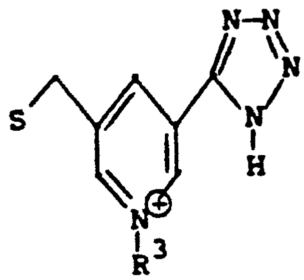
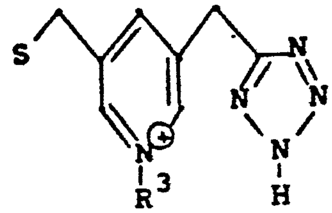

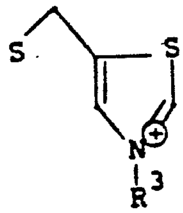
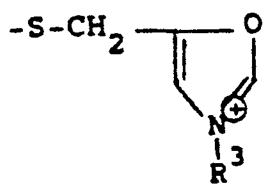
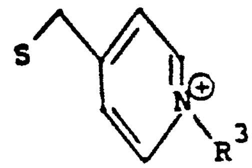

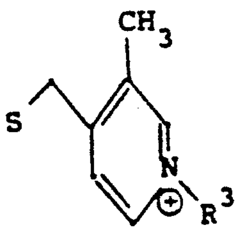
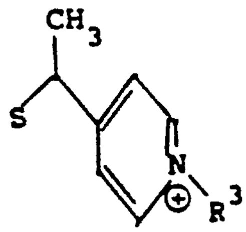
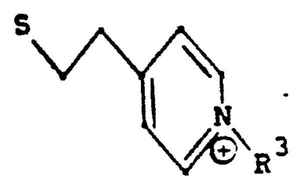

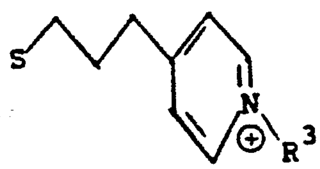
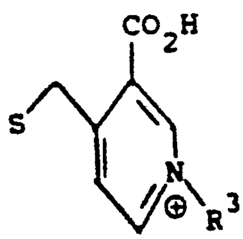
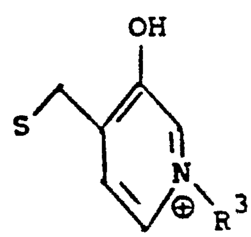

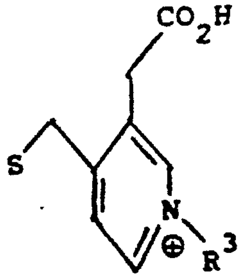
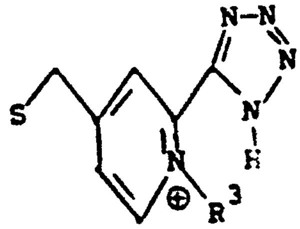

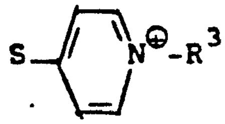
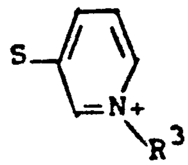
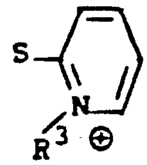
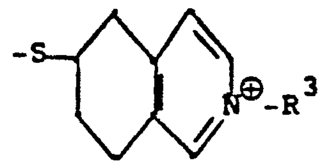

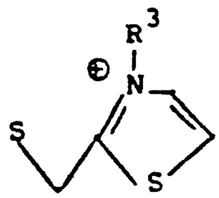
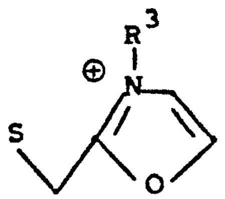
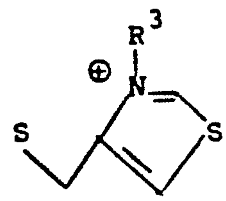

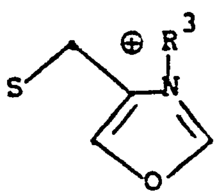
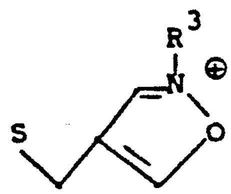
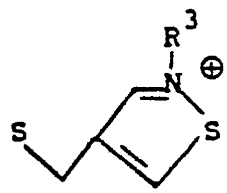

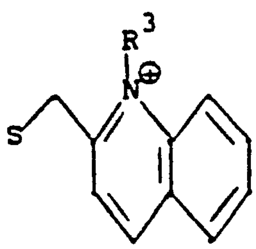
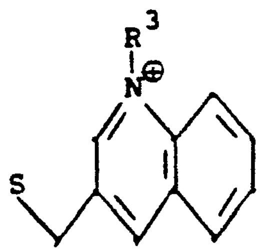
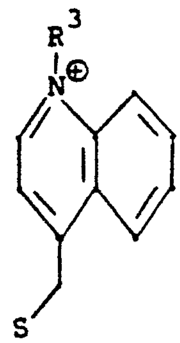

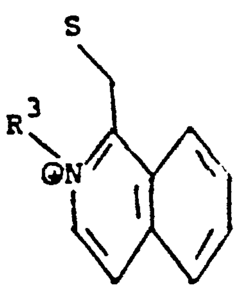
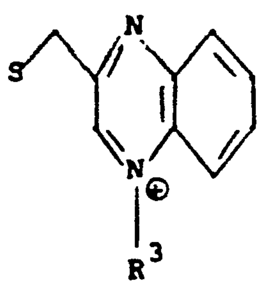
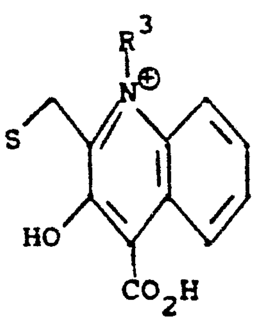

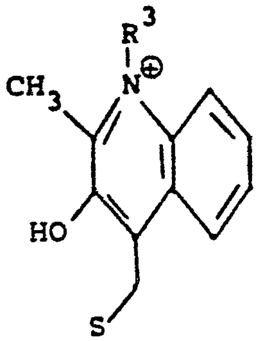 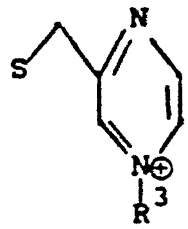 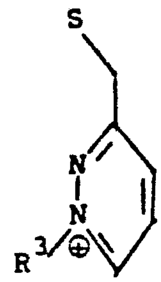

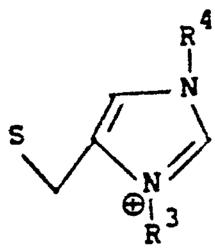 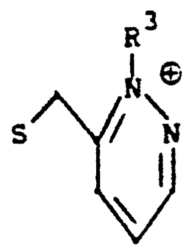

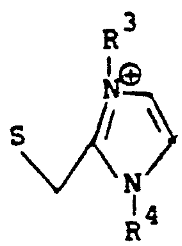 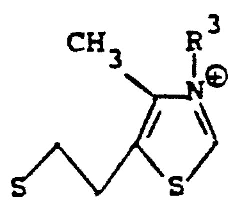 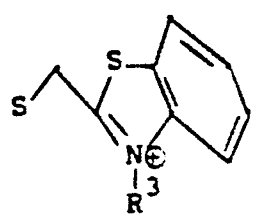 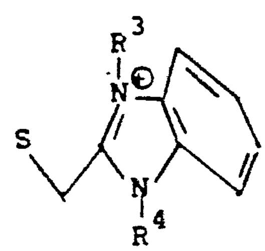

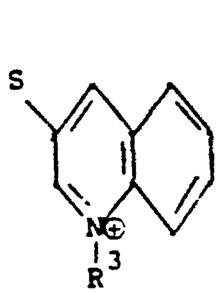 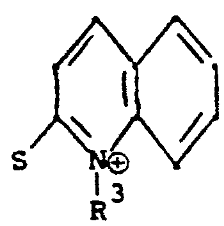 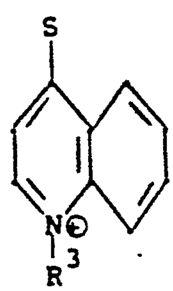 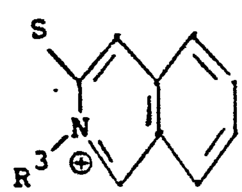

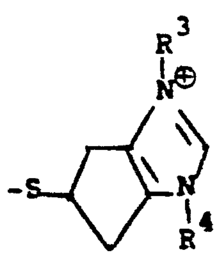 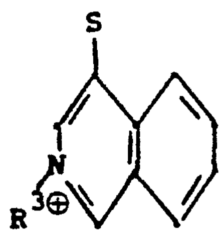 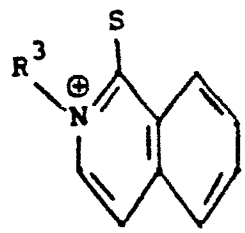

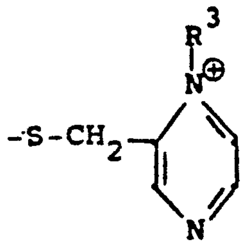
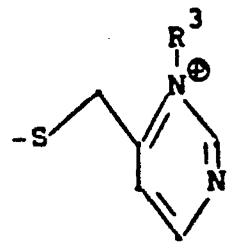
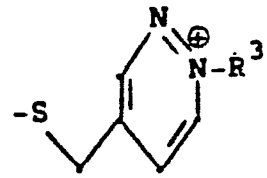

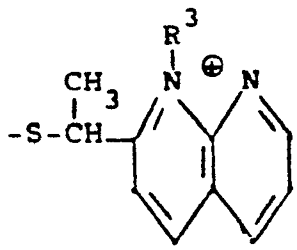
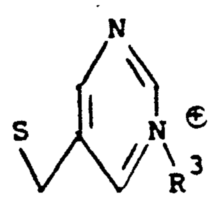

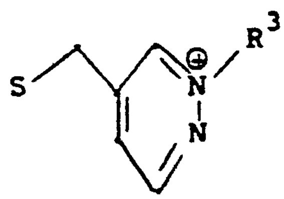
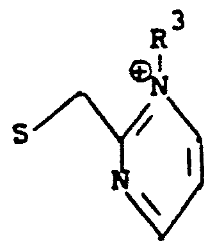
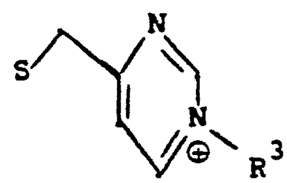

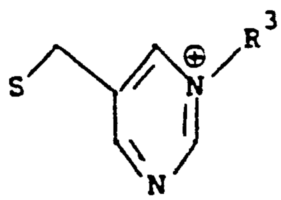
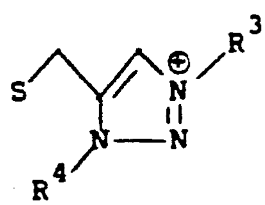
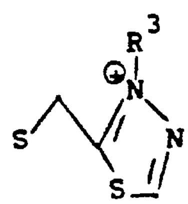

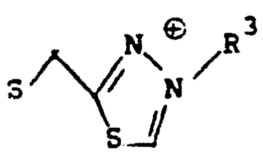
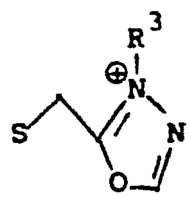

and the like.

A preferred S-L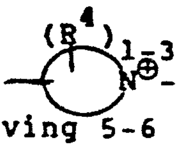- $R^3$ group is monocyclic heteroaryl having 5-6 ring atoms and optionally one heteroatom additional to the N atom already present, e.g.,

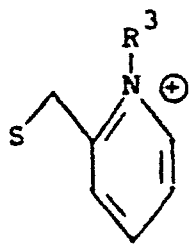 , 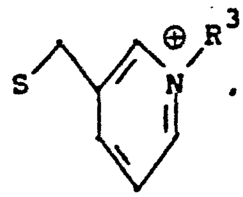 , 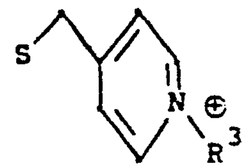 ,

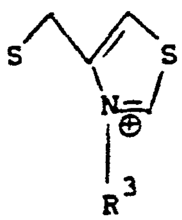 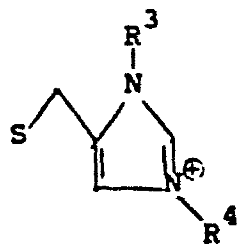 and 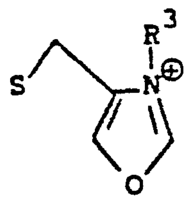

where $R^3$ and $R^4$ are as defined in the preferred list above.

A more preferred subclass includes the nuclei shown above where $R^3$ is $CH_3$ and $R^4$ is $CH_3$.

The compounds of Formula I include inner (Zwitterion) salts when Y is $COO^{\ominus}$ e.g.

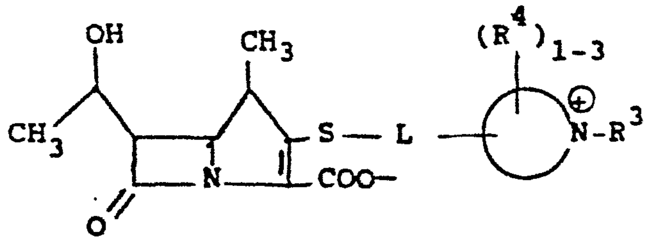

or, when Y is other than $COO^{\ominus}$, salts with an external, physiologically acceptable counterion $Z^{\ominus}$ such as $Cl^{\ominus}$, $Br^{\ominus}$, $I^{\ominus}$, $OCH_3^{\ominus}$, $OSO_2CF_3^-$, $\phi P(O)(O\ phenyl)_2^{\ominus}$ and the like.

The inner salts are preferred.

Again, the compounds of Formula I include the stereoisomers as mixtures and as separate isomers.

A preferred isomer configuration is:

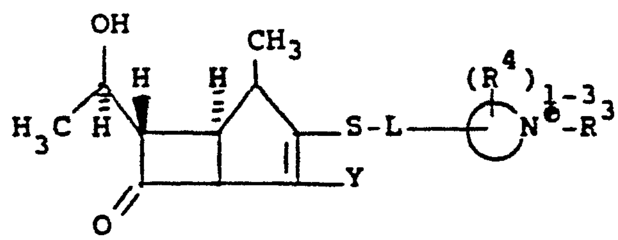

Ia

The compounds of the present invention (I) are valuable antibiotics active against various Gram-positive and Gram-negative bacteria and accordingly find utility in human and veterinary medicine. Representative pathogens which are sensitive to antibiotics I include: Staphylococcus aureus, Escherichia coli, Klebsiella Pneumoniae, Bacillus subtilis, Salmonella typhosa, Pseudomonas and Bacterium proteus. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of

antibiotic per million parts of solution in order to destroy or inhibit the growth of harmful bacteria on medical and dental equipment and as bactericides in industrial applications, for example in waterbased paints and in the white water of paper mills to inhibit the growth of harmful bacteria.

The compounds of this invention may be used in any of a variety of pharmaceutical preparations. They may be employed in capsule, powder form, in liquid solution, or in suspension. They may be administered by a variety of means; those of principal interest include: topically or parenterally by injection (intravenously or intramuscularly).

Compositions for injection, a preferred route of delivery, may be prepared in unit dosage form in ampules, or in multidose containers. The compositions may take such forms as suspensions, solutions, or emulsions in oily or aqueous vehicles, and may contain formulatory agents. Alternatively, the active ingredient may be in powder form for reconstitution, at the time of delivery, with a suitable vehicle, such as sterile water. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized infections. Such matters, however, are left to the routine discretion of the therapist according to

principles of treatment well known in the antibiotic art. In general, a daily dosage consists of from about 5 to about 600 mg of active ingredient per kg of body weight of the subject in one or more treatments per day. A preferred daily dosage for adult humans lies in the range of from about 10 to 240 mg of active ingredient per kg of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10-60%. The composition will generally contain from about 15 mg to about 1500 mg of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution.

The preferred method of administration of the formula I antibiotic is parenteral by i.v. infusion, i.v. bolus, or i.m. injection.

For adults, 5-50 mg of Formula I antibiotic per kg of body weight given 2, 3, or 4 times per day is preferred. Preferred dosage is 250 mg to 1000 mg of the Formula I antibiotic given two (b.i.d.) three (t.i.d.) or four (q.i.d.) times per day. More specifically, for mild infections, and particularly

urinary tract infections, a dose of 250 mg t.i.d. or q.i.d. is recommended. For moderate infections against highly susceptible gram positive and gram negative organisms, a dose of 500 mg t.i.d. or q.i.d. is recommended. For severe, life-threatening infections against organisms at the upper limits of sensitivity to the antibiotic, a dose of 1000 t.i.d. or q.i.d. is recommended.

For children, a dose of 5-25 mg/kg of body weight given 2, 3, or 4 times per day is preferred; a dose of 10 mg/kg t.i.d. or q.i.d. is usually recommended.

Antibiotic compounds of Formula I are of the broad class known as carbapenems or 1-carbadethiapenems. Certain of these carbapenems are susceptible to attack by a renal enzyme known as dehydropeptidase (DHP). This attack or degradation may reduce the efficacy of the carbapenem antibiotic. Inhibitors of DHP and their use with carbapenem antibiotics are disclosed in the prior art [see published European Patent Applications No. 79102616.4 filed July 24, 1979 (Patent Number 10573); 79102615.6, filed July 24, 1979 (application no. 15573); and No. 82107174.3, filed August 9, 1980 (application no. 72014)].

The present I compounds may, where DHP inhibition is desired or necessary, be combined or used with the appropriate DHP inhibitor as described in the aforesaid published applications. Thus, to the extent that the cited European patent applications 1.) define the procedure for determining DHP susceptibility of the present carbapenems and 2.)

disclose suitable inhibitors, combination compositions and methods of treatment, they are incorporated herein by reference. A preferred weight ratio of I compound:DHP inhibitor in the combination compositions is about 1:1. A preferred DHP inhibitor is 7-(L-2-amino-2-carboxyethylthio)-2-(2,2-dimethylcyclopropanecarboxamide)-2-heptenoic acid or a useful salt thereof.

These combination compositions and their use is another embodiment of the present invention.

The compounds of Formula I may be prepared by any convenient process.

<u>A.</u> One such process is illustrated in the following reaction equations:

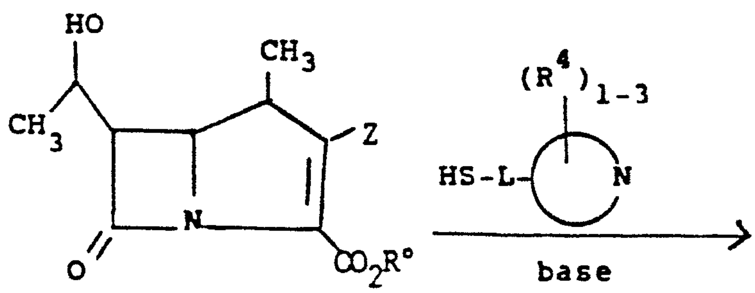


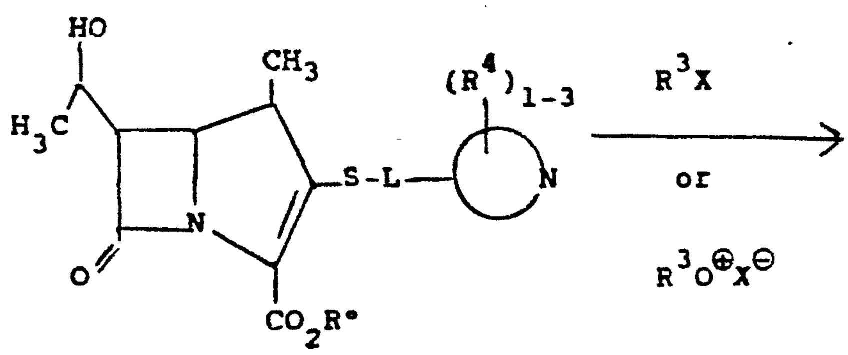

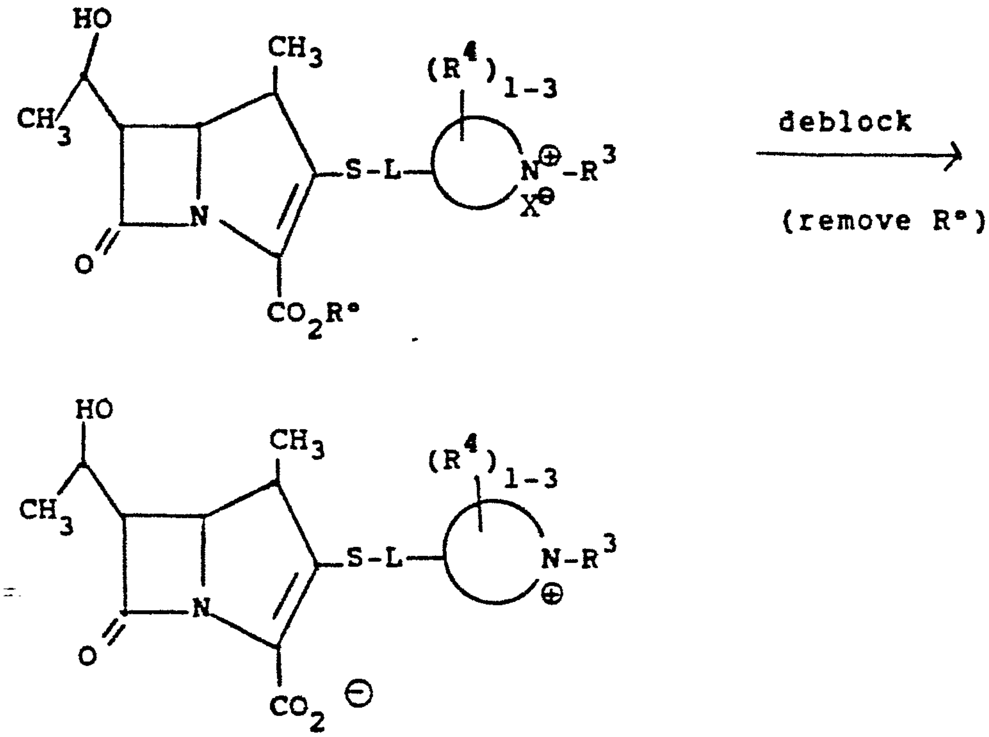


I

wherein Z is a leaving group such as $-OPO(O\emptyset)_2$,

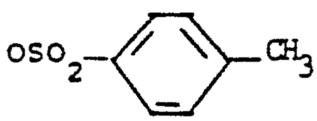 , $OSO_2CF_3$

and the like, X is a leaving group such as Br, I, $OSO_2CF_3$, $-OSO_2F$, 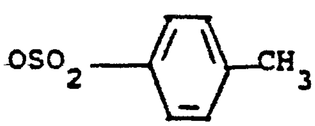 or X is an anionic group such as $BF_4$, $SbF_5$, $PF_6$ and the like; and R° is a protecting group such as p-nitrobenzyl or allyl. $R^3$, L and $R^4$ are as defined above.

The side chain addition reaction is carried out in a solvent such as acetonitrile, dimethylformamide, dimethylacetamide or N-ethylpyrrolidinone in the presence of a base such as

N,N-diisopropylethylamine, triethylamine or 4-dimethylaminopyridine at a temperature of from -40°C to 25°C for a period of five minutes to ten hours. The alkylation reaction is conducted in a solvent such as dichloromethane, dimethylformamide, acetonitrile or dimethylacetamide at a temperature of from -20°C to 25°C for a period of 1 to 24 hours. The deblocking reaction wherein R° is p-nitrobenzyl is usually conducted in an aqueous system containing cosolvents such as tetrahydrofuran, ethanol, n-butanol, i-amyl alcohol, or ethyl acetate and a pH 6.8 to 7.0 aqueous buffer. Suitable buffers include phosphate buffers and buffers derived from non-nucleophilic amines such as N-methylmorpholine or morpholinopropane sulfonic acid. The reaction is conducted at 0°C to 40°C for 0.5 to 5 hours under 1-100 atmospheres of hydrogen in the presence of a catalyst such as 10% palladium on carbon or 20% palladium hydroxide on carbon. The final products are purified by ion exchange chromatography and/or reverse phase chromatography. When a pharmaceutically acceptable ester of the final product is desired, the deblocking step is omitted and the appropriate R° group is incorporated into the starting material.

B. A second process is illustrated by the following set of equations:

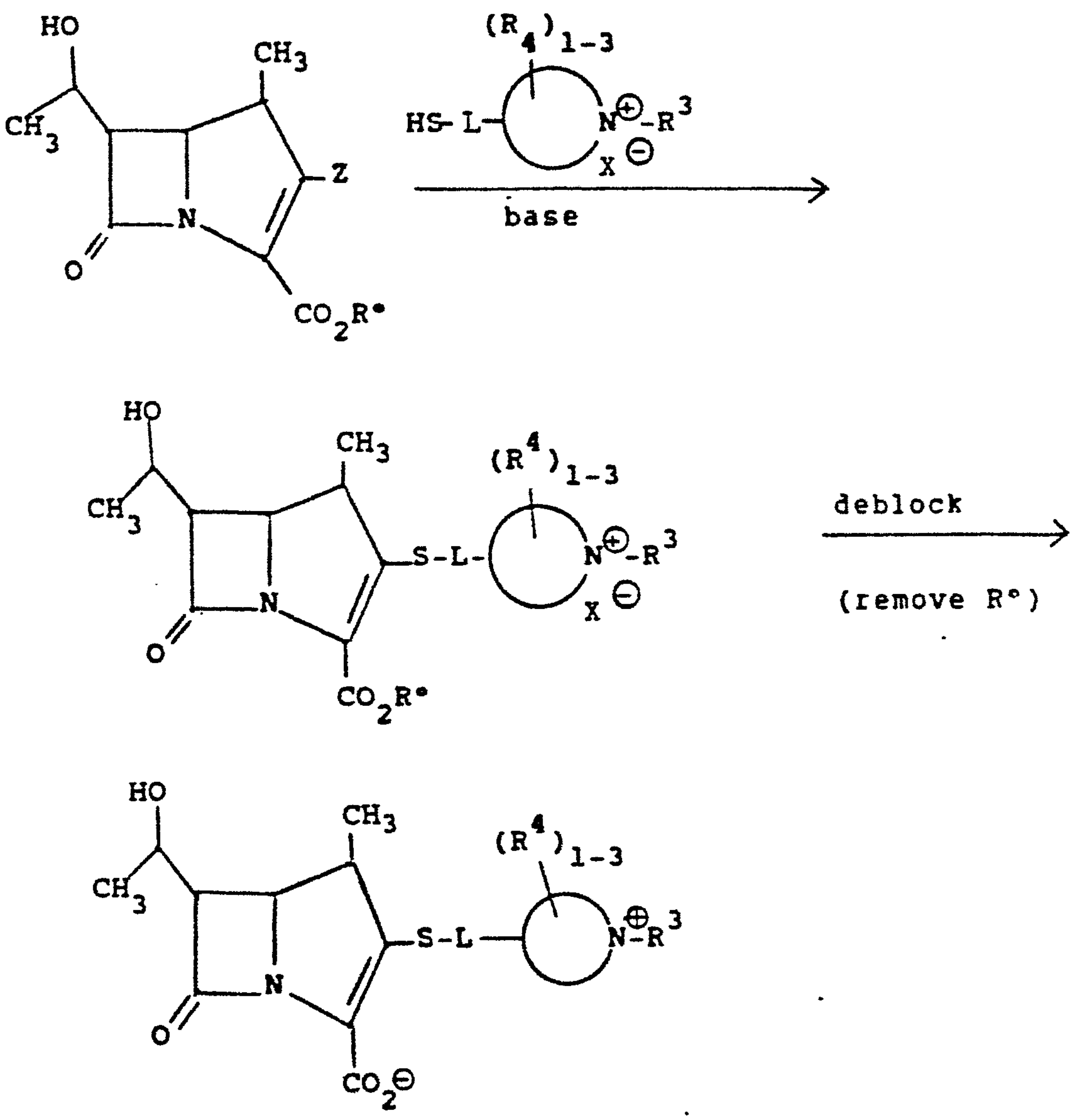


wherein Z, X, L, R°, $R^3$ and and $R^4$ are as defined above.

The difference between the above process and that earlier described is that the side chain moiety is alkylated with the group $R^3$ prior to addition to the carbapenem nucleus. The side chain addition step and deblocking are conducted as described above.

C. A third process is illustrated by the following set of equations:

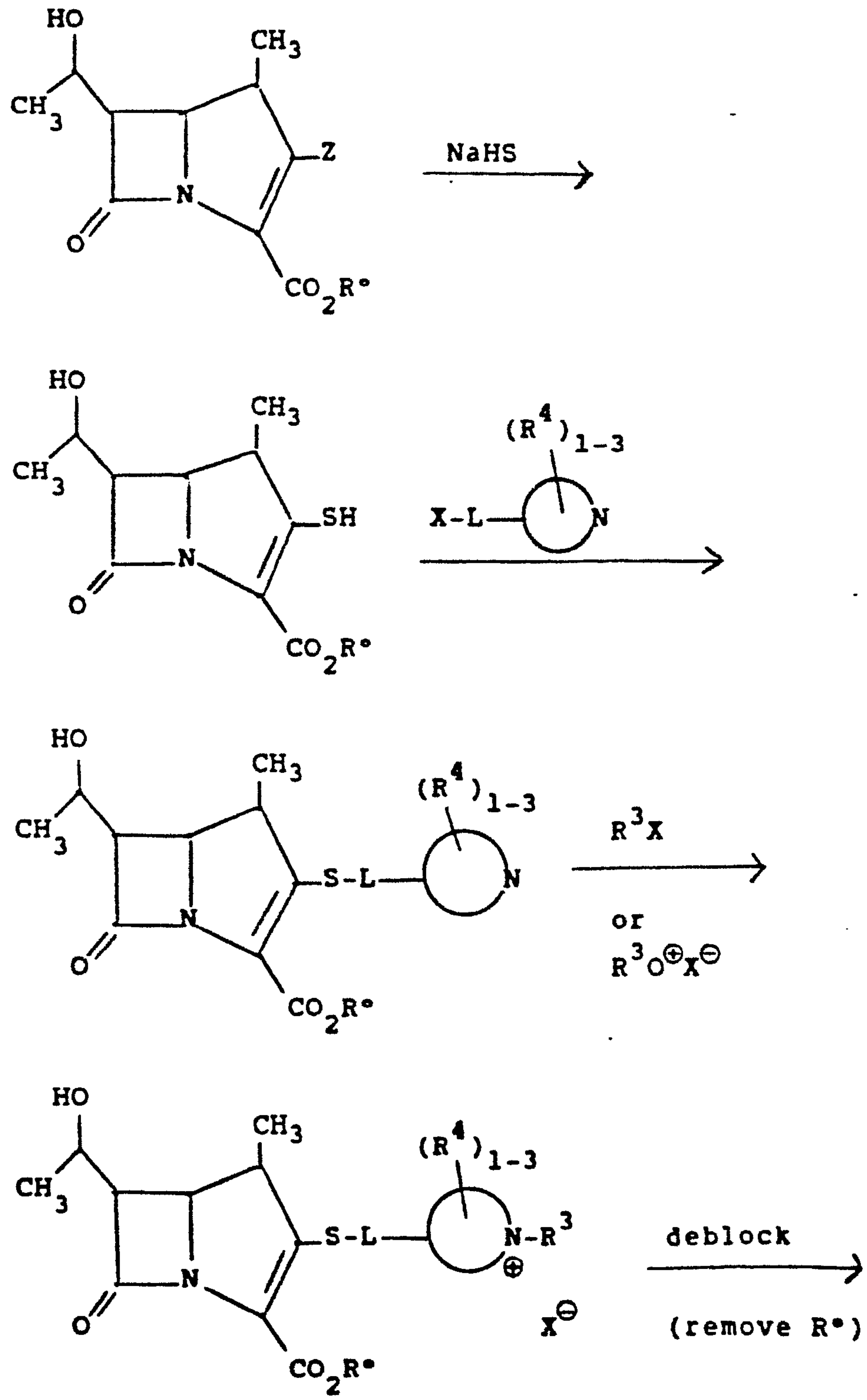

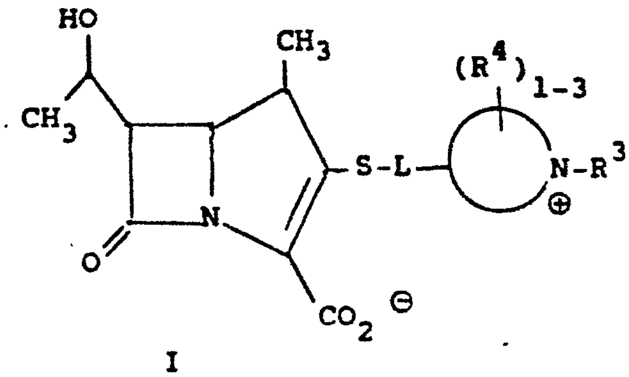

I

wherein Z, X, L, R°, $R^3$ and $R^4$ are as previously defined.

In this case the 2-mercapto intermediate is generated from the activated carbapenem upon exposure to sodium hydrosulfide in dimethylformamide or dimethylacetamide at a temperature of from -50°C to -20°C for a period of five minutes to one hour. The sulfur atom is alkylated in a solvent such as acetonitrile, dimethylformamide, dimethylacetamide or the like in the presence of a base such as N,N-diisopropylethylamine, triethylamine, 4-dimethylaminopyridine or the like at a temperature of from -40°C to 25°C for a period of from ten minutes to eight hours. The side chain alkylation, removal of R° and purification of I is conducted as described above.

D. A fourth process is illustrated by the following set of equations:

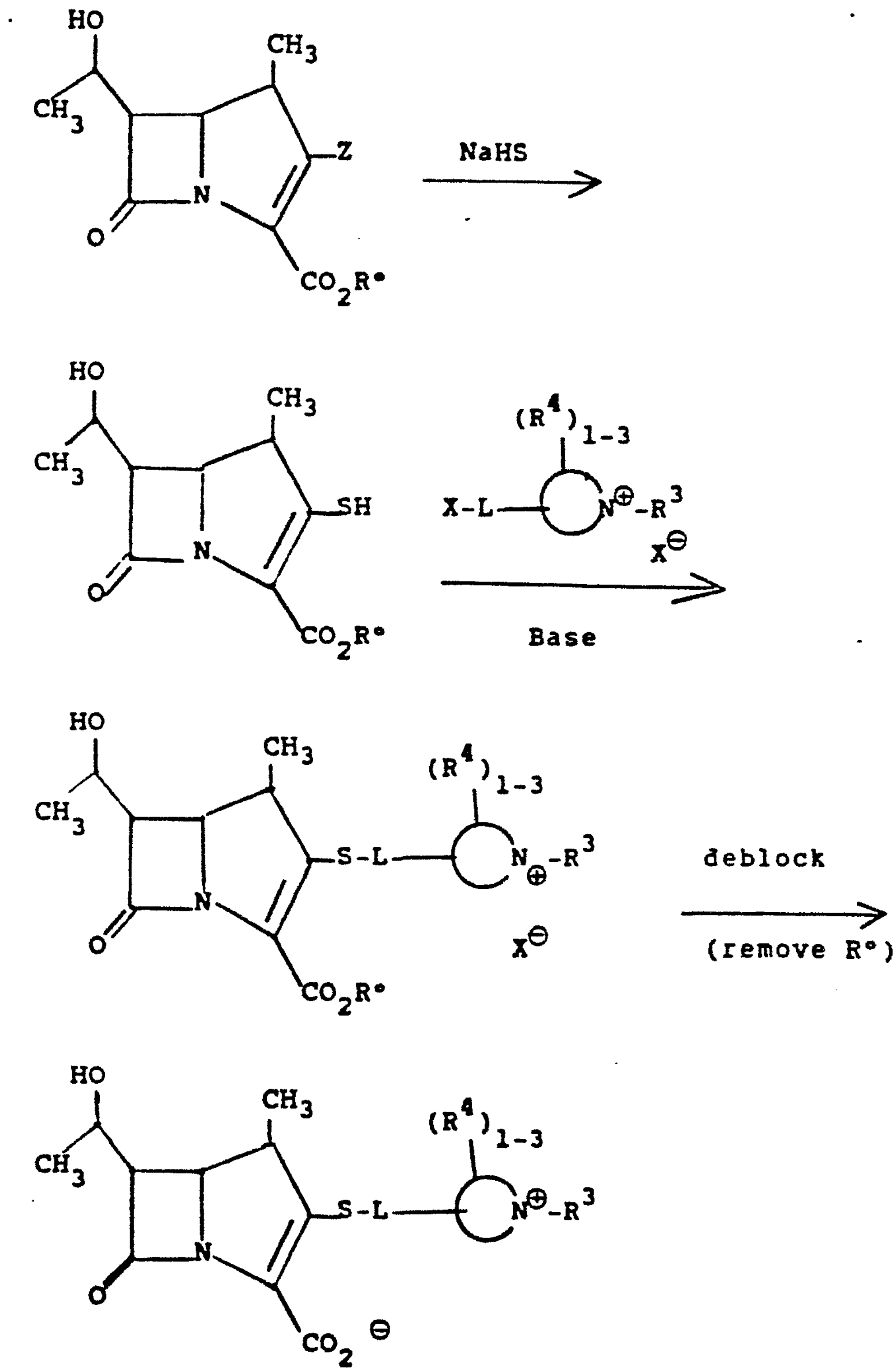


I

wherein Z, X, R°, $R^3$, and $R^4$ are as previously defined.

The difference between this process and that described in process C is that the side chain moiety is alkylated with the group $R^3$ prior to addition to the carbapenem nucleus. The side chain addition step and the deblocking are conducted as described above.

The following examples illustrate the preparation of compounds of Formula I. The temperature is in degrees Celsius unless otherwise indicated.

EXAMPLE 1

Step A.

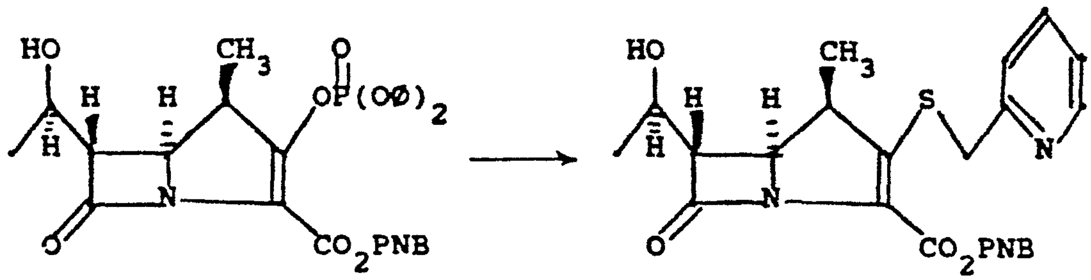

1 2

Preparation of p-Nitrobenzyl 1R,5S,6S-6-(1-1R-hydroxyethyl)-1-methyl-2-(2-pyridylmethylthio)-carbapen-2-em carboxylate

To a suspension of vinylphosphate (399 mg, 0.672 mmol) in 3 ml dry acetonitrile is added 127 µl (0.732 mmol) of N,N-diisopropylethylamine and 94.7 µl (0.84 mmol) of 2-pyridylmethyl mercaptan while the temperature is maintained at 0°. After stirring 3 hours at 0° the resulting homogenous solution is diluted with ethyl acetate and washed with 3 x 15 ml of 5% aqueous sodium bicarbonate. The organic phase is dried over sodium sulfate and concentrated to crude product in vacuo which is purified by preparative layer chromatography (Rf=0.14 in 9:1 ethyl acetate/hexanes). The desired ester 2 is obtained as a yellow foam (235 mg). nmr δ ($CDCl_3$): 1.22 (d), 1.31 (d), 3.22 (q), 3.73 (qt), 4.04 (d), 4.12 (q), 4.24 (d), 5.17 (d), 7.1-8.6 (m).

Step B.

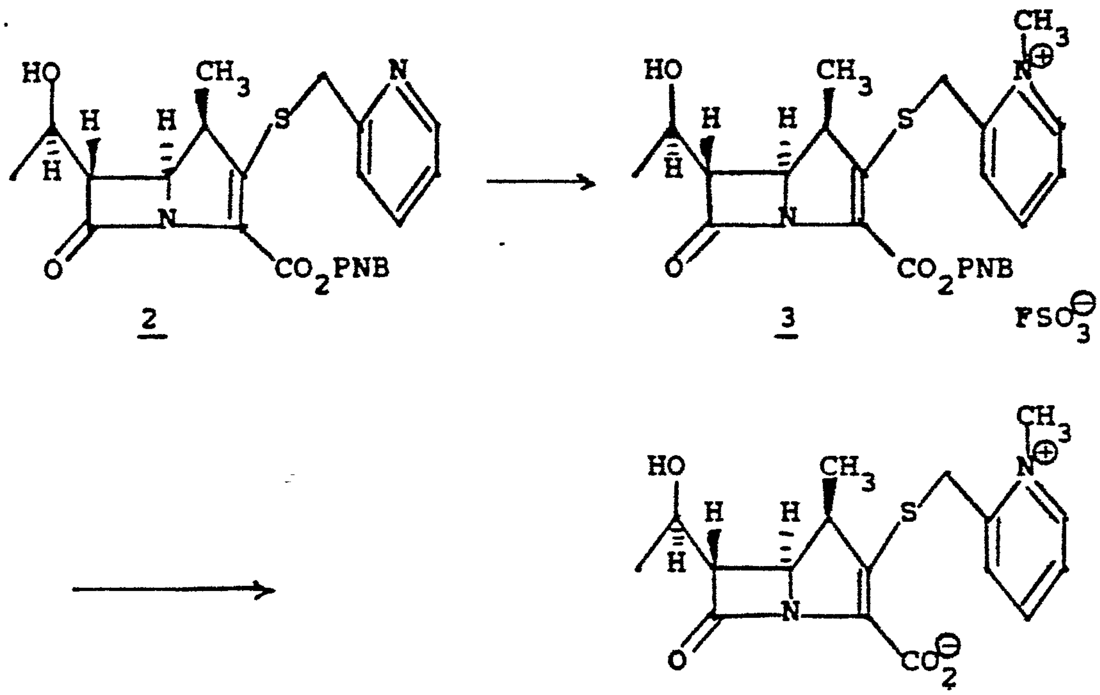

2 3

4

Preparation of 1R,5S,6S-6-(1-1R-Hydroxyethyl-1-methyl-2-(2-N-methylpyridiniummethylthio)carbapen-2-em carboxylate

To a solution of 105.3 mg (0.224 mmol) ester 2 in 1.0 ml dichloromethane is added 20 µl (0.246 mmol) of methyl fluorosulfonate. The mixture is stirred 3.5 hours at room temperature and then the resulting white precipitate of 3 is collected by filtration The crude salt is dissolved in a mixture of 4.4 ml water, 4.4 ml n-butanol, 2.2 ml ethyl acetate, 2.2 ml 0.5M N-methylmorpholine hydrochloride (pH 6.8) and 32.9 mg of 20% palladium hydroxide on carbon is added. The mixture is hydrogenated at 35 psi for 1.6 hours at then the organic and aqueous phases separated. The aqueous phase is filtered to

remove catalyst, washed with ethyl acetate and concentrated to about 2 ml. Purification is effected by chromatography on Dowex 50x4 ($Na^+$) at 5° with elution with water. Fractions containing 4 by UV are pooled, concentrated then lyophilized to yield 14.6 mg. UV$\lambda_{max}^{H_2O}$ 295 nm. nmr ($D_2O$):$\delta$ 1.13 (d), 1.25 (d), 3.36 (m), 3.47 (q), 4.21 (m), 4.4 (s), 7.89 (m), 8.45 (t), 8.77 (d).

EXAMPLE 2

Step A.

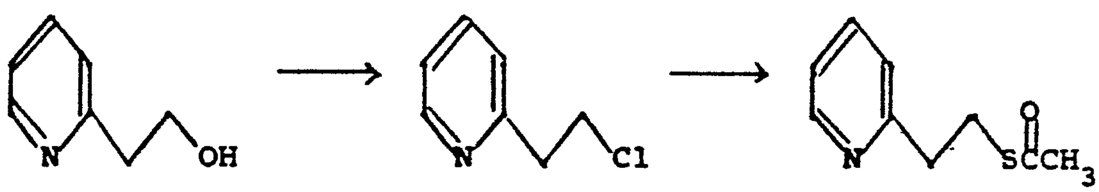

5 6

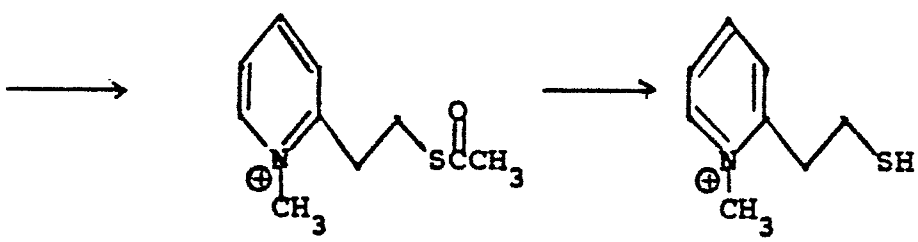

7 8

Preparation of 2-(N-Methyl-2-pyridinium)ethanethiol 8

2-ß-Hydroxyethyl)pyridine (1.00 g, 8.12 mmol) is charged into a 25 ml round-bottom flask under nitrogen and cooled to 0°. Thionyl chloride (650 µl, 8.93 mmol) is added in small portions over 10 minutes with some anhydrous ether (about 200 µl) to facilitate stirring. When the addition is complete the bath is removed and the mixture stirred 17 hours at room temperature. The resulting suspension is triturated with ether and the remaining solid is partitioned between aqueous potassium carbonate and ether (75 ml). The aqueous layer is extracted twice more with 75 ml portions of ether and the combined organics are dried over magnesium sulfate and evaporated to 1.15 g of 5 as a pale yellow oil. nmr ($CDCl_3$): $\delta$ 3.25 (t), 3.94 (t), 7.24 (m), 7.67 (tt), 8.6 (dd).

Crude 5 (1.00 g, 7.06 mmol) and 0.968 g (847 mmol) of potassium thioacetate are dissolved in 11.6 ml N,N-dimethylformamide and heated to 80° for 1.3 hours. The solvent is then removed in vacuo and the residue is taken up in ethyl acetate, washed with water and brine, dried over magnesium sulfate and concentrated to a dark red oil 6 (1.00 g, 5.52 mmol). nmr ($CDCl_3$): $\delta$ 2.34 (s), 3.08 (t), 3.32 (t), 7.2 (m), 7.64 (tt), 8.58 (dd).

The crude thioester 6 (1.00 g, 5.52 mmol) is dissolved in 4.6 ml N,N-dimethylformamide and 3.45 (55.2 mmol) of methyl iodide is added. The mixture is allowed to age at room temperature for 17 hours then concentrated to a brown solid. Trituration with dichloromethane followed by filtration yields 1.31 g (4.06 mmol) of 7 as a brown solid. nmr ($D_2O$): $\delta$ 2.35 (s), 3.26-3.4 (m), 4.44 (s), 7.96 (m), 8.48 (t), 8.8 (d).

A suspension of 100 mg (0.31 mmol) 7 in 590 µl of 2N perchloric acid in methanol is stirred at room temperature for 18 hours. Pyridinium salt 8 is precipitated as a dark oil upon dilution of this mixture with anhydrous ether.

Step B.

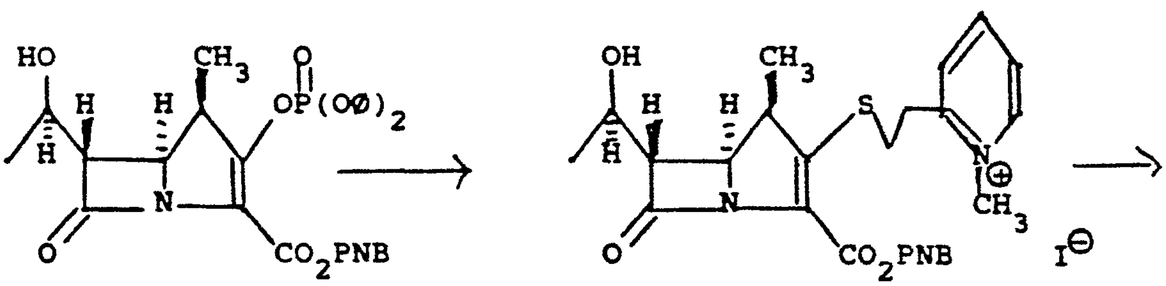

1 9

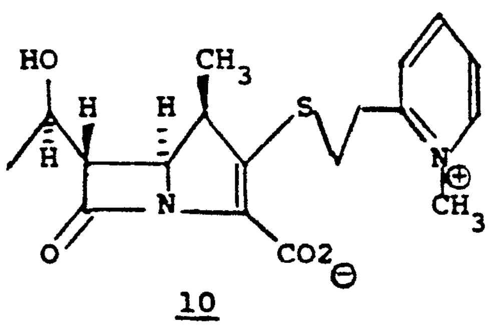

10

Preparation of 1R,5S,6S-6-(1-1R-hydroxyethyl)-1-methyl-2-(2-N-methylpyridinium-2-ethylthio)carbapen-2-em carboxylate

Vinylphosphate 1 (181 mg, 0.30 mmol) and mercaptan 11 are combined in 1.26 ml of N,N-dimethylacetamide and cooled to -20°. N,N-diisopropylethylamine (106 µl, 0.60 mmol) is added and the solution aged to -20° for 35 minutes to give 9. The resulting solution is transferred to a hydrogenation vessel

with 5.86 ml isopropanol, 3 ml ethyl acetate, 5.86 ml water and 3.0 ml 0.1M pH 7 phosphate buffer. Palladium hydroxide (44 mg, 20% Pd) is added and the mixture hydrogenated to 41 psi for 2 hours. The resulting suspension is filtered to remove catalyst, diluted with ethyl acetate and worked up as in Example I to yield 2.9 mg of 10.

EXAMPLE 3

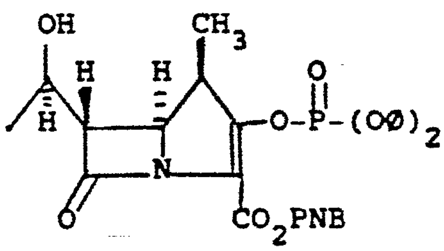

1

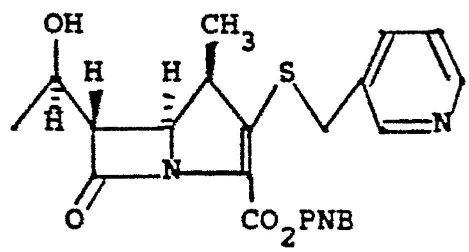

11

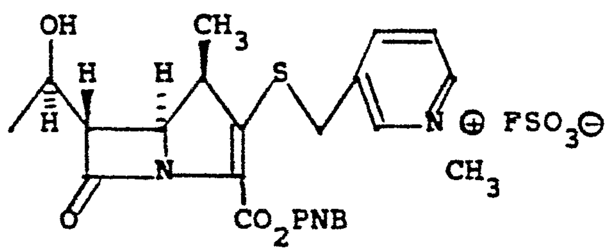

12

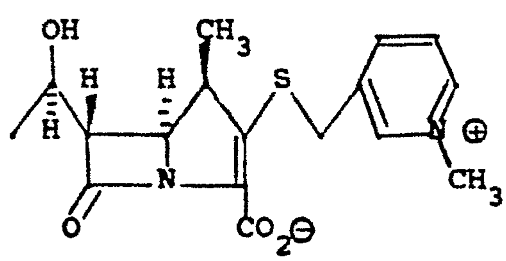

13

Preparation of 1R,5S,6S-6-(1-R-hydroxyethyl)-2-(3-N-methylpyridiniummethylthio)-1-methylcarbapen-2-em-3-carboxylate

Following the procedure in Example Ib, above, the β-methylvinylphosphate (1) (100 mg) is treated with 3-mercaptomethylpyridine (20.2 μl) and diisopropylethylamine (32.5 μl) in acetonitrile (0.5 ml). The product (11) is obtained crystalline from ethyl acetate yield 51 mg (65%).

To a suspension of 11 (47.8 mg) in methylene chloride (1 ml) is added methyl fluorosulfonate (9 μl) at 0°. The solid changes during 10 minutes to a heavy oil. The methylene chloride is evaporated in a stream of nitrogen. The residue is hydrogenated in THF-buffer solution and the product isolated via Dowex 50, $Na^+$ chromatography to give 20 mg of the title compound 13. $\lambda_{max}$ 265 nm, E% 221, 296 nm, E% 221 (85% $NH_2OH$ ext.). 200 MHz, NMR, $\delta$ 1.19 (d, J=7.5 Hz), 1.27 (d, J=6Hz), 3.3 (d, q, J=7.5 and 9 Hz), 3.46 (dd, J=2.8 and 6Hz), 4.14 (dd, J=2.8 and 9Hz), 4.24 (abq, J=15Hz and m (hidden)), 8.01 (dd, J=6 and 8.5 Hz), 8.51 (d, J=8.5 Hz), 8.69 (d, J=6Hz), 8.83 (s).

EXAMPLE 4

Step A.

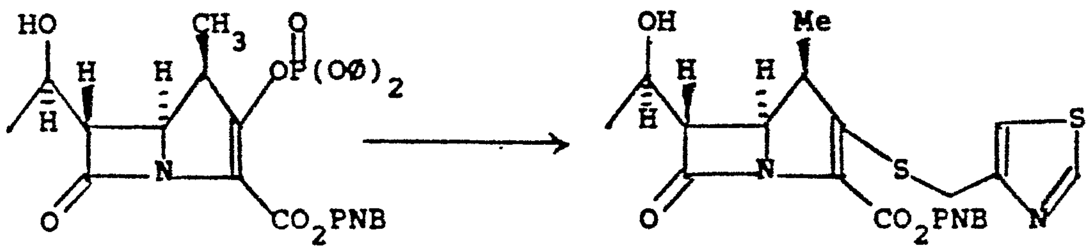

1 14

p-Nitrobenzyl(1R,5S,6S)-6-(1-R-hydroxyethyl)-2-(4-thiazolylmethylthio)-1-methylcarbapen-2-em-3-carboxylate 14

To an ice-cooled suspension of p-nitrobenzyl (1R,5S,6S)-6-(1-R-hydroxyethyl)-2-diphenylphosphoroxy-1-methylcarbapen-2-em 3-carboxylate (100 mg, 0.168 mmoles) in acetonitrile (0.5 ml) is added diisopropylethylamine (31 µl, 0.179 mmoles) and 4-thiomethylthiazole (17 µl, 0.17 mmoles). After 2 hours the mixture is diluted wtih methylene chloride, washed twice wth pH 7 phosphate buffer, dried over anhydrous magnesium sulfate, evaporated and placed on a reverse phase preparative tlc plate. 5% ethanol/chloroform ($R_f$=0.5) the title comound 46 mg is isolated as a light yellow oil. 58% yield.

Step B.

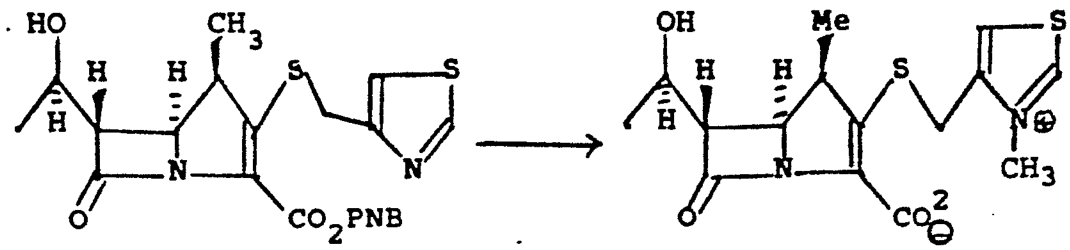

14 15

1R,5S,6S-6-(1-R-hydroxyethyl)-2-(4-N-methylthiazolium-methylthio)-1-methylcarbapen-2-em 3-carboxylate 18

p-Nitrobenzyl (1R,5S,6S)-6-(1-R-hydroxyethyl)-2-(4-thiazolylmethylthio)-1-methylcarbapen-2-em 3-carboxylate 14 (0.04 g, 0.091 mmoles) in acetonitrile (1.0 cc) is treated with methyl fluorosulfonate (8.0 µl, 0.091 mmoles) at room temperature for 2 hours. The solvent is evaporated by a stream of nitrogen and the residue is hydrogenated in THF-pH7 buffer solution. The isolation of the title compound is accomplished via Dowex 50 sodium cycle chromatography to give 10 mg of a powder 15.

uv $(H_2O)\lambda_{max}$. 295 nm

NMR (selected resonances) $D_2O,\delta$1.28 (3H, d, J=6.5Hz), 3.48 (1H, dd, J=2.8, 6.0Hz), 1.18 (3H, d, J=7.5Hz), $\overset{\oplus}{N}$-methyl, 4.26 (3H, S).

EXAMPLE 5

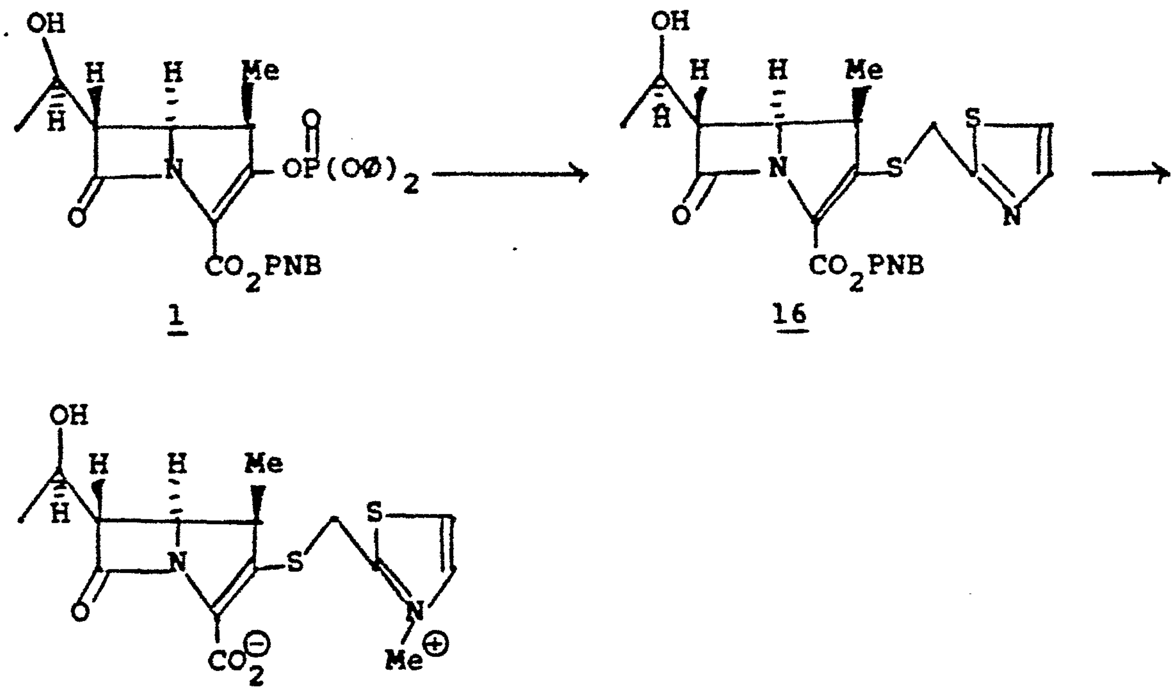

1

16

17

1R,5S,6S-6-(1-R-hydroxyethyl)-2-(2-N-methylthiazolium-methylthio)-1-methylcarbapen-2-em 3-carboxylate 17

Following the procedures in Example IV, the β-methylvinylphosphate (1) (150 mg, 0.252 mmoles) is treated with 2-mercaptomethylthiazole (27 μl, 0.316 mmoles) and diisopropylethylamine (55 μl, 0.316 mmoles) in acetonitrile (1 cc). The product 16 is obtained as a colorless oil (82 mg) from a tlc plate. 71% yield.

To a suspension of 16 (0.05 g, 0.108 mmoles) in acetonitrile (1.5 cc) is added methyl fluorosulfonate (9.0 μl, 0.108 mmoles). The solvent is evaporated by a stream of nitrogen and the residue is hydrogenated in THF- pH 7 phosphate buffer solution. The isolation of the title compound is accomplished via Dowex 50 sodium cycle chromatography to give 17 as 10 mg of a light yellow powder.

uv $(H_2O) \lambda_{max}$ = 296 nm

NMR (selected resonances) $(D_2O) \delta$ 1.26 (3H, d, J=6.5Hz), 3.45 (1H, dd, J=2.8, 6.0Hz), 1.18 (3H, d, J=7.5Hz), $\overset{\oplus}{N}$-methyl, 4.16 (3H, s).

## EXAMPLE 6

Step A.

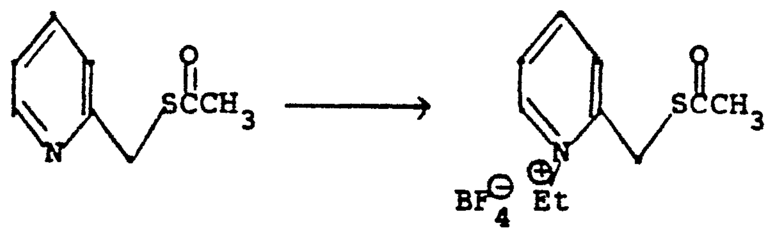

18

Preparation of N-Ethyl-2-pyridiniummethylthioacetate tetrafluoroborate 18

2-Picolyl thioacetate (200 mg) is treated with triethyloxonium tetrafluoroborate (227 mg) in methylene chloride (1 ml) at 0°C for 5 hours. The mixture is diluted with ether (20 ml) to separate product 18 as oil (210 mg). 60 MHz NMR $(CDCl_3)$: $\delta$ 1.57 (t), 2.48 (s), 4.66 (s), 4.72 (q), 8.00-9.20 (m).

Step B.

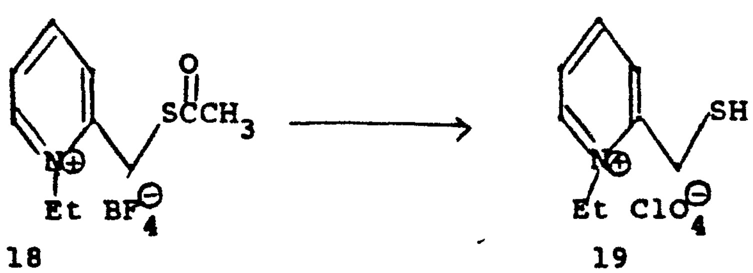

18 19

Preparation of N-ethyl-2-pyridiniummethylmercapta-perchlorate

Tetrafluoroborate salt 18 (210 mg) is treated with 2N $HClO_4$/methanol (1.54 ml) at room temperature for 2 days. The reaction mixture is then diluted with ether to separate oily product 19. 200 MHz NMR (DMSO-$d_6$):$\delta$ 1.55 (t), 4.64 (s), 4.70 (q), 8.00-9.18 (m).

Step C.

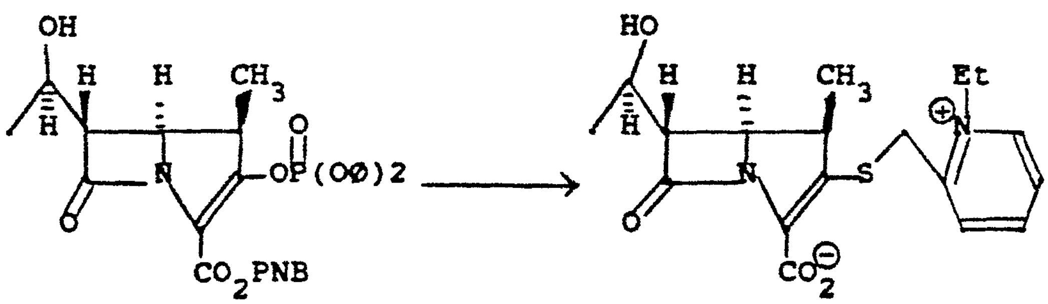

1 20

Preparation of 1R,5S,6S-6-(1-1R-hydroxyethyl)-2-(N-ethyl-2-pyridiniummethylthio)-1-methylcarbapen-2-em carboxylate

At -20°C, the 1-ß-methylvinylphosphate I (100 mg) is treated with N-ethyl-2-pyridiniummethylmercaptan perchlorate 19 (70 mg) and diisopropylethylamine (66 µl) in N,N-dimethylacetamide (1 ml) for 20 minutes. The reaction mixture is then transferred into a hydrogenation flask which contains isopropanol (3.3 ml), 0.1M pH 7.0 phosphate buffer (3.3 ml) and 20% Pd/C (25 mg) and hydrogenated at 50 psi for 2 hours.

The mixture is filtered from catalyst, then diluted with ethyl acetate (20 ml) to separate

aqueous layer. The aqueous layer is charged to a Dowex-50x4 ($Na^+$) column which is eluted with D.I. water. The fractions containing product 23 are combined, concentrated, then lyophilized to give solid 20 (4.7 mg), uv$\lambda_{max}^{H_2O}$ 292 nm ($NH_2OH$ extinguishable) and 268 nm, 200MHz NMR ($D_2O$):$\delta$1.21 (d), 1.33 (d), 1.70 (t), 3.45 (m), 3.55 (q), 4.30 (m), 4.82 (q), 7.9-8.95 (m).

## EXAMPLE 7

Step A.

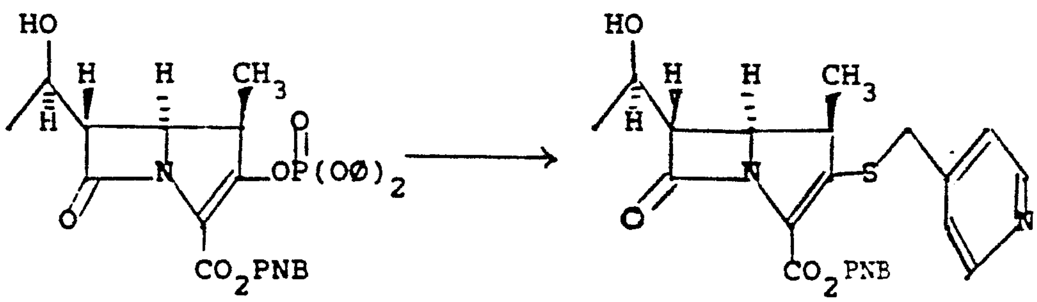

1 21

Preparation of p-Nitrobenzyl 1R,5S,6S-6-(1-1R-hydroxyethyl)-1-methyl-2-(4-pyridylmethylthio)carbapen-2-em carboxylate

Vinylphosphate 1 (225 mg, 0.378 mmol) and 4 pyridylmethanethiol hydrochloride (64 mg, 0.397 mmol) are dissolved/suspended in 1.8 ml acetonitrile and cooled to 0°. N,N-Diisopropylethylamine (145 µl, 0.832 mmol) is added and the resulting homogenous solution stirred 45 minutes. The mixture is diluted with ethylacetate, washed with aqueous bicarbonate and brine, dried over magnesium sulfate and evaporated to yield 202 mg of 21 as a yellow foam.

Step B.

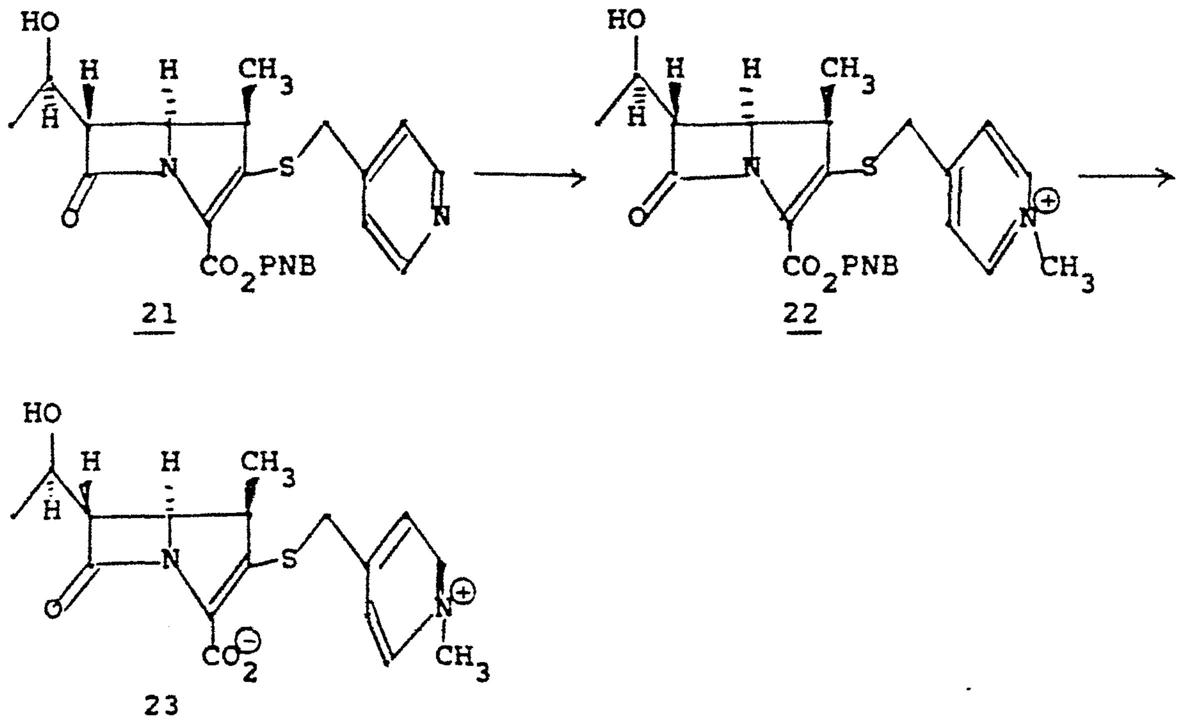

21 22

23

Preparation of 1R,5S,6S-6-(1-1R-hydroxyethyl)-1-methyl-2-(N-methyl-4-pyridiniummethylthio)carbapen-2-em carboxylate

Ester 21 (202 mg, 0.43 mmole) is dissolved in 2 ml dichloromethane and cooled to 0°. Methyl fluorosulfonate (38 µl, 0.473 mmol) is added resulting in an orange precipitate. After stirring 30 minutes at 0° the suspension is concentrated to yield crude 22 which is transferred to a hydrogenation vessel with 7.5 ml n-butanol, 3.8 ml ethyl acetate, 7.5 ml water and 3.8 ml of pH 6.8 0.5M N-methylmorpholine hydrochloride buffer. Palladium hydroxide (20% on carbon, 57 mg) is added and the mixture hydrogenated at 45 psi for one hour. Workup and purification is conducted as in Example Ic, above, to yield 17 mg of 23.

uv (water): λmax 293 nm, $NH_2OH$ extinguished λmax 294 nm.

NMR ($D_2O$), δ 1.19 (d, J=7.2 Hz, $C_1$, $CH_3$), 1.27 (d, J=6.3Hz, CH(OH)C$\underline{H}_3$), 3.29 (qd, J=9.6, 7.5Hz, $H_1$), 3.47 (dd, J=6.0, 2.8Hz, $H_6$), 4.10 (dd, J=9.6, 2.8Hz, $H_5$), 4.23 (quint, J=6.3Hz, C$\underline{H}$OH), 4.15 (d, J=15.1Hz, SC$H_{\underline{A}}H_B$), 4.35 (d, J=15.1Hz, SC$H_AH_{\underline{B}}$), 4.35 (S, N-C$\underline{H}_3$), 7.99 (d, J=6.7Hz, ArH), 8.69 (d, J=6.7Hz, ArH).

EXAMPLE 8

Step A:

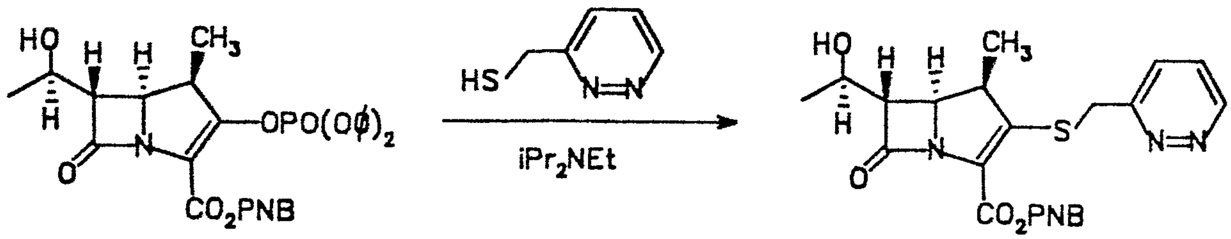


p-Nitrobenzyl (1R,5S,6S)-6-[1(R)-hydroxyethyl]-2-(3-pyridazinylmethylthio)-1-methylcarbapen-2-em 3-carboxylate

A suspension of p-nitrobenzyl(1R,5S,6S)-2-(diphenylphosphono)oxy-6-[1(R)-hydroxyethyl]-1-methyl-carbapen-2-em 3-carboxylate (168 mg, 0.282 mmol) in anhydrous acetonitrile (2 cc) was cooled in an ice-bath under a nitrogen atmosphere and was treated with N,N-diisopropylethylamine (50 µl, 0.282 mmol), followed by the dropwise addition of 3-mercaptomethylpyridazine*(35.6 mg, 0.282 mmol). The solid slowly dissolved and after 30 minutes the solution was diluted with methylene chloride, was washed with 0.1N pH 7 phosphate buffer, dried over magnesium sulfate, filtered and evaporated under vacuum. The residue was chromatographed on a 1 mm x 20 cm x 20 cm silica gel GF plate, using 5% ethanol-methylene chloride as a developing solvent, to give tne title compound (81 mg) as a white solid.

mp 153°C (dec) Thomas Hoover Capillary Melting Point Apparatus (uncorrected)

IR (Nujol) ß-lactam $\nu_{max}$ 1740 $cm^{-1}$

NMR ($CDCl_3$) $\delta$ 1.27 (d, J=6.5 Hz, $C\underline{H}_3CHOH$), 1.33 (d, J=7.5 Hz, HC-$\underline{CH}_3$), 3.29 (dd, J=2.8, 6.5 Hz, H6), 3.90 (dq, J=7.5, 9.1 Hz, H1), 4.12+4.56 (ABq, J=14.8 Hz, $SCH_2$), 4.14 (dd, J=2.8, 9.1 Hz, H5), 4.24 (P, J=6.5 Hz, $CH_3\underline{CH}OH$), 5.20+5.50 (ABq, J=14.0 Hz, $CH_2Ar$), 7.51 (dd, J=5.0, 8.2 Hz, pyridazinyl H5), 7.66 (d, J=8.6 Hz, pyridazinyl H4), 7.66 (d, J=8.8 Hz, 2ArH), 8.24 (d, J=8.8 Hz, 2ArH), 9.14 (d, J=5.0 Hz, pyridazinyl H6).

*3-Mercaptomethylpyridazine

K. Yu. Novitsuii, N. K. Sadovaya, E. F. Kas'Yanova, L. K. Semna, Khimiya Geterotsiklicheskikh Soedinenii Vol 6, No. 3, pp 412-414 (1970).

STEP B:

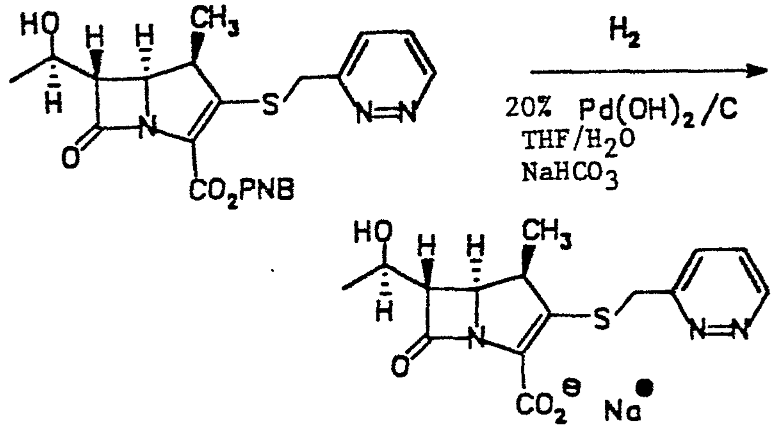


Sodium (1R,5S,6S)-6-[1(R)-hydroxyethyl]-2-[3-pyridazinylmethylthio]-1-methylcarbapen-2-em 3-carboxylate

A suspension of p-nitrobenzyl (1R,5S,6S)-6-[1(R)-hydroxyethyl]-2-(3-pyridazinylmethylthio)-1-methylcarbapen-2-em 3-carboxylate (1.5 g,0.0032 mol) in a mixture of water (0.1 L), containing sodium bicarbonate (0.27 g, 0.0032 mol), and tetrahydrofuran (0.1 L), was hydrogenated for 2 hours at 40 psig in the presence of 20% $Pd(OH)_2/C$ (0.3 g). The mixture was filtered through Solka-Floc and the solution was washed with ethylether. The aqueous phase was concentrated under vacuum to ca. 50 ml and was freeze-dried giving (1.19 g) of the title compound as a light yellow solid.

IR (Nujol) ß-lactam $\nu$max 1740 $cm^{-1}$

UV ($H_2O$) $\lambda$max 300 ($\epsilon$ 6,620) 83% $H_2NOH$ extinguished

NMR ($D_2O$) $\delta$ (ppm) 1.14 (d, J=7.5 Hz, CH<u>$CH_3$</u>), 1.26 (d, J=6.5 Hz, <u>$CH_3$</u>CHOH), 3.40 (dd, J=2.6, 6.5 Hz, H6), 3.40 (m, H2), 4.04 (dd, J=2.6, 9.1 Hz, H5), 4.20 (P, J=6.5 Hz, $CH_3$<u>CH</u>OH), 4.22+4.41 (ABq, J=15.0 Hz, S-$CH_2$), 7.80 (dd, J=5.0, 7.3

STEP C:

Hz, pyridazinyl H5), 7.94 (dd, J=8.8, 1.5 Hz, pyridazinyl H4), 9.08 (dd, J=5.0, 1.1 Hz, pyridazinyl H6).

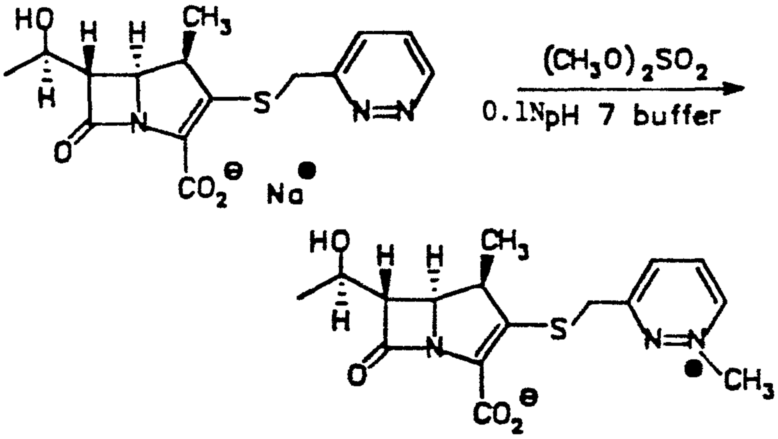


(1R,5S,6S)-6-(1(R)-Hydroxyethyl)-2-(1-methyl-3-pyridaziniummethylthio)-1-methylcarbapen-2-em 3-carboxylate

A solution of sodium (1R,5S,6S)-6-(1(R)-hydroxyethyl)-2-(3-pyridazinylmethylthio)-1-methylcarbapen-2-em 3-carboxylate (1.0 g, 0.0028 mol) in 0.1N pH 7 phosphate buffer (20 cc) was cooled in an ice-bath and treated with dimethylsulfate (1.3 ml, 0.014 mol). The mixture was stirred rapidly in the cold for 150 minutes, while incremental amounts of 1N NaOH were added in order to maintain a pH range of 6.8 to 7.2. The suspension was washed with ethylether and was loaded on a column of Dowex 50W-X4 resin (sodium form, 200-400 mesh, 2.5 cm x 38 cm). The ice-cooled jacketed column was eluted with de-ionized water and 25 cc fractions were collected. Fractions 13-53 were combined, concentrated under vacuum to 50 cc and lyophilized to give the title compound (0.63 g) as a yellow solid.

IR (Nujol) ß-lactam $\nu$ max 1750 $cm^{-1}$

UV ($H_2O$) $\lambda$ max 290 ($\epsilon$7,050) 78% $H_2NOH$ extinguished

NMR ($D_2O$) $\delta$ (ppm) 1.22 (d, J=7.5 Hz, CH$\underline{CH}_3$), 1.28 (d, J=6.5 Hz, $\underline{CH}_3$CHOH), 3.49 (dd, J=3.0, 5.9 Hz, H6), 3.55 (m, H1), 4.21 (dd, J=2.8, 9.1 Hz, H5), 4.29+4.49 (ABq, J=14.8 Hz, S-$CH_2$), 4.64 (s, N-$CH_3$), 8.51 (dd, J=5.0, 8.2 Hz, pyridazinyl H5), 8.57 (d, J=8.8 Hz, pyridazinyl H4), 9.56 (d, J=5.0 Hz, pyridazinyl H6).

EXAMPLE 9

Step A:

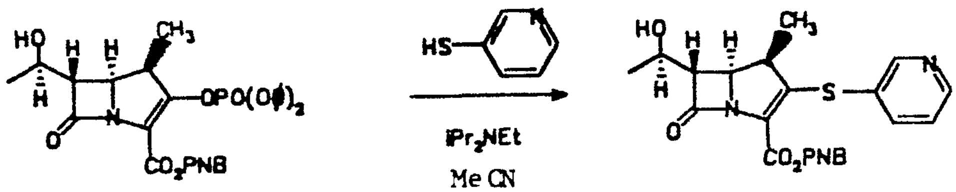


p-Nitrobenzyl (5S,6S)-6-[1(R)-hydroxyethyl]-2-(3-pyridylthio)-1(R)-methylcarbapen-2-em-3-carboxylate

A suspension of p-nitrobenzyl (5S,6S)-2-(diphenylphospheno)oxy-6-[(1(R)-hydroxyethyl]-1(R)-methylcarbapen-2-em-3-carboxylate (446 mg, 0.75 mmol) in anhydrous acetonitrile (3 ml) was cooled to ca. -20°C under a nitrogen atmosphere and treated dropwise over 1 minute with a solution of 3-mercaptopyridine (108 mg, 0.97 mmol) in acetonitrile (1 ml) followed by N,N-diisopropylethylamine (169 µl, 0.97 mmol). The resulting mixture was stirred for 2 hours at -20°C and for 1 hour at 0°C, then diluted with ethyl acetate, washed with brine, 5% aqueous sodium bicarbonate and brine, dried over magnesium sulfate, filtered, and evaporated under vacuum to a pale yellow gum (406 mg). The crude product was chromatographed on EM silica gel 60 (10 g) using 1:1 ethyl acetate-methylene chloride as eluting solvent; 8 ml fractions were collected every 4 minutes. After 17 fractions, the eluting solvent was changed to ethyl acetate. Fractions 14-30 gave a pale yellow gum (306 mg) that crystallized from ethyl acetate-ethyl ether to afford the title compound (88 mg) as a white solid. The mother liquors gave additional product (39 mg) on standing at room temperature.

mp 109-111° (microhot stage);
IR (Nujol) $\nu$max 1770, 1705, 1515, 1335 $cm^{-1}$;
UV (dioxane) $\lambda$max 265 nm ($\epsilon$ 13,400), 323 nm ($\epsilon$ 16,520);
NMR ($CDCl_3$) $\delta$ 0.99 (d, J=7.4 Hz, $C\underline{H}_3CH$), 1.32 (d, J=6.2 Hz, $C\underline{H}_3CHOH$), 3.04 (dq, J=9.6 and 7.4 Hz, $CH_3C\underline{H}$), 3.25 (dd, J=2.7 and 6.4 Hz, H6), 4.22 (dd, J=2.7 and 9.6 Hz, H5), 4.25 (dq, J=6.4 Hz, $CH_3C\underline{H}OH$), 5.30 and 5.57 (two d, J=13.8 Hz, $CH_2Ar$), 7.35 (dd, J=5.0 and 7.8 Hz, pyridyl H5), 7.70 (d, J=8.8 Hz, 2ArH), 7.86 (ddd, J=1.3, 2.0 and 7.8 Hz, pyridyl H4), 8.25 (d, J=8.8 Hz, 2ArH), 8.64 (dd, J=1.3 and 5.0 Hz, pyridyl H6), 8.76 (d, J=2.0 Hz, pyridyl H2);
Mass Spectrum m/e 455 ($M^+$), 411, 369, 301, 111;
Anal. Calc'd for $C_{22}H_{21}N_3O_6S$:
C, 58.02; H, 4.65; N, 9.23; S, 7.04
Found: C, 57.74; H, 4.64; N, 8.97; S, 6.84.

Step B:

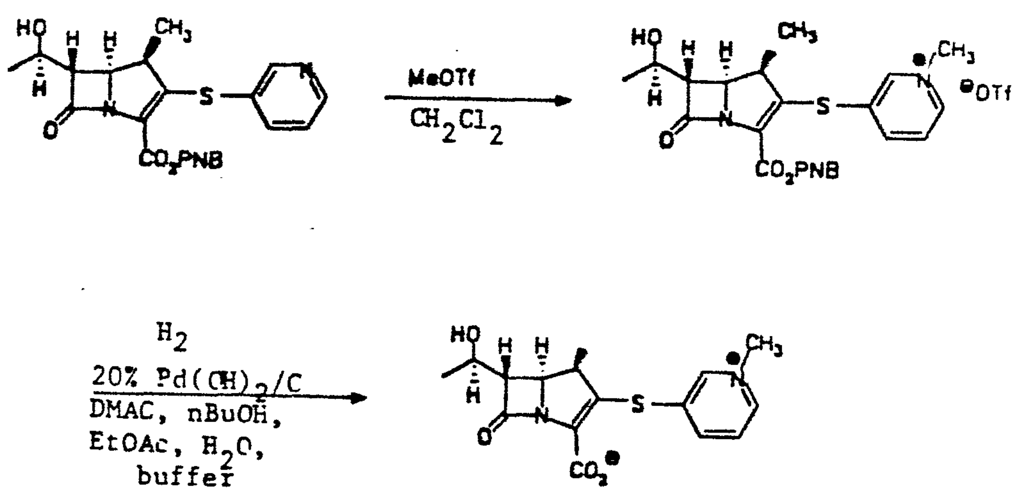

(5S,6S)-6-[1(R)-Hydroxyethyl]-2-[(1-methyl-3-pyridinium)thio]-1(R)-methylcarbapen-2-em-3-carboxylate

Methyl trifluoromethanesulfonate (17 µl, 0.15 mmol) was added to an ice-cold, stirring solution of p-nitrobenzyl (5S,6S)-6-[1(R)-hydroxyethyl]-2-(3-pyridylthio)-1(R)-methylcarbapen-2-em-3-carboxylate (56.9 mg, 0.125 mmol) in anhydrous methylene chloride (1.25 ml), and the resulting mixture was stirred in the cold for 100 minutes. The solvent phase was decanted from the gummy precipitate which was washed with methylene chloride and dried under vacuum to a yellow foam. The foam was dissolved in N,N-dimethylacetamide (1 ml) and the solution was diluted with n-butanol (5 ml), ethyl acetate (2.5 ml), water (5 ml) and 0.5M pH 6.8 N-methylmorpholine hydrochloric acid buffer (2.5 ml), treated with 20% palladium hydroxide on carbon (25 mg), and hydrogenated at 45 psi for 90 minutes. The mixture was filtered through a celite pad using additional water. The aqueous portion of the filtrate was washed with methylene chloride and ether, then concentrated under vacuum to ca. 1 ml and added to a column of Dowex 50W-X4 resin (sodium form, 200-400 mesh, 1.5 x 30 cm). The column was eluted with water in a cold room collected 170 drop fractions every 3.3 minutes. Fractions 10-16 were concentrated under vacuum to a few ml, filtered through a 0.45 µ CR acrodisc, and lyophilized to provide the title compound as a yellow, amorphous powder (24.3 mg).

IR (Nujol) $\nu$max 3350 (br), 1758, 1605 cm$^{-1}$;

UV (0.05 pH 7.0 MOPS buffer)$\lambda$ max 275 nm (sh, $\epsilon$ 7,610), 302 nm ($\epsilon$ 9,115);

UV (buffer + $NH_2OH.HCl$) max 266.5 nm ($\epsilon$ 4,900), 319 nm ($\epsilon$ 2,010), extinguished$\lambda$ max 301.5 nm ($\epsilon$ ext. 7,480);

NMR ($D_2O$) $\delta$ 1.07 (d, J=7.3 Hz, $C\underline{H}_3CH$), 1.27 (d, J=6.4 Hz, $C\underline{H}_3CHOH$), 3.17 (dq, J=9.8 and 7.3 Hz, $CH_3C\underline{H}$), 3.55 (dd, J=2.9 and 5.9 Hz, H6), 4.27 (dq, J 6.2 Hz, $CH_3C\underline{H}OH$), 4.32 (dd, J=2.9 and 9.8 Hz, H5), 4.39 (s, $NCH_3$), 8.01 (dd, J=6 and 8 Hz, pyridyl H5), 8.58 (br d, J=8 Hz, pyridyl H4), 8.71 (br d, J=6 Hz, pyridyl H6), 8.95 (br s, pyridyl H2).

## EXAMPLE 10

Steps A - E:

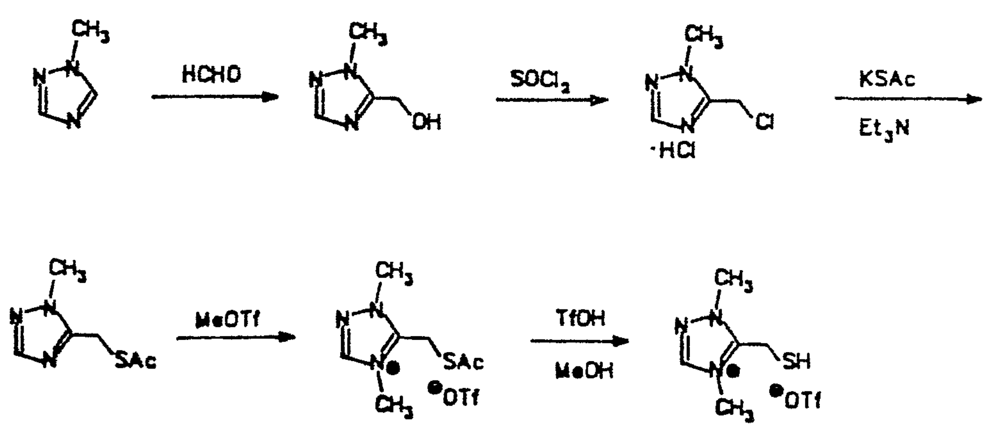

1,4-Dimethyl-5-mercaptomethyl-1,2,4-triazolium trifluoromethanesulfonate

Step A. 5-Hydroxymethyl-1-methyl-1,2,4-triazole

A solution of 1-methyl-1,2,4-triazole (4.16 g, 0.05 mol) in formalin (20 ml) was heated overnight in a sealed tube at 135°C. After cooling, the solvent was evaporated under vacuum to give a clear liquid that partially solidified on standing. This material was distilled to give a white, crystalline solid (4.65 g) bp. ca. 110°C/0.25 mm. The solid product was recrystallized from ethyl acetate-hexane to afford the title compound (3.78 g, 67%) as white crystals.

IR (Nujol) $\nu$max 3180, 1505, 1290, 1200, 1045, 1000 $cm^{-1}$;

NMR ($CDCl_3$) $\delta$ 3.95 (s, $CH_3$), 4.75 (d, J=6.5 Hz, $CH_2$), 5.49 (t, J=6.5 Hz, OH), 7.78 (s, H5);

Anal. Calc'd for $C_4H_7N_3O$:

C, 42.47; H, 6.24; N, 37.15

Found: C, 42.67; H, 6.16; N, 37.35.

Step B. 5-Chloromethyl-1-methyl-1,2,4-triazole hydrochloride

The hydroxymethyl triazole from Step 1 (1.00 g) was added to ice-cold thionyl chloride (4 ml) and the resulting mixture was heated at reflux for 25 minutes. Excess thionyl chloride was evaporated under vacuum. The solid residue was recrystallized from ethanol-ethyl acetate to give the title compound (1.17 g, 79% yield) as white crystals.

IR (Nujol) $\nu$max 1585, 1400, 1265, 1250, 960 $cm^{-1}$;
NMR ($D_2O$) $\delta$ 4.07 (s, $CH_3$), 4.85 (s, HOD), 5.04 (s, $CH_2$), 8.53 (s, H5);
Anal. Calc'd for $C_4H_7Cl_2N_3$:
C, 28.59; H, 4.20; N, 25.01
Found: C, 28.73; H, 4.16; N, 25.00.

Step C. 5-Acetylthiomethyl-1-methyl-1,2,4-triazole

A mixture of the chloromethyltriazole from Step 2 (609 mg, 3.63 mmol) and potassium thiolacetate (497 mg, 4.36 mmol) in anhydrous acetonitrile (7.3 ml) was treated with a speck of dicyclohexano-18-crown-6 and with triethylamine (531 µl, 3.81 mmol). The resulting mixture was stirred at room temperature for 3 hours. The mixture was filtered and the solids washed with acetonitrile. The filtrate and washings were evaporated under vacuum to a residue which was triturated with three portions of ethyl acetate. The ethyl acetate extracts were filtered, washed with brine, dried over magnesium sulfate, filtered, and evaporated under vacuum to afford the title compound (526 mg, 85%) as an orange liquid.
NMR ($CDCl_3$) $\delta$ 2.40 (s, $CH_3CO$), 3.91 (s, $CH_3$), 4.26 (s, $CH_2$), 7.80 (s, H5).

Step D. 5-Acetylthiomethyl-1,4-dimethyl-1,2,4-triazolium trifluoromethanesulfonate

A solution of 3-acetylthiomethyl-2-methyl-1,2,4-triazole (244 mg, 1.43 mmol) in anhydrous methylene chloride (1.4 ml) was cooled in an ice bath under a nitrogen atmosphere and treated with methyl trifluoromethanesulfonate (194 µl, 1.71 mmol). The

resulting mixture was stirred in the cold for 30 minutes, then evaporated under vacuum. The residue was triturated three times with diethyl ether, then dissolved in anhydrous methylene chloride and evaporated under vacuum to afford the title compound (484 mg, 100%) as a viscous orange oil.

NMR ($D_2O$) $\delta$ 2.43 (s, $CH_3CO$), 3.95 (s, $CH_3$), 4.14 (s, $CH_3$), 4.62 (s, $CH_2$), 4.78 (s, HOD), 8.72 (s, H5).

<u>Step E</u>. <u>1,4-Dimethyl-5-mercaptomethyl-1,2,4-triazolium trifluoromethanesulfonate</u>

A solution of the product from the preceding step (484 mg, 1.43 mmol) in anhydrous methanol (1.4 ml) was treated with trifluoromethanesulfonic acid (127 µl, 1.43 mmol) and kept at room temperature for 18.5 hours. The solution was diluted with ethyl ether to precipitate the product as an oil. The oil was washed four times with ethyl ether, diluted with anhydrous methylene chloride, and evaporated under vacuum to provide the title compound (344 mg, 82%) as a pale orange oil.

NMR ($D_2O$) $\delta$ 3.93 (s, $CH_3$), 4.08 (s, $CH_3$), 4.25 (s, $CH_2$), 4.78 (s, HOD), 8.72 (s, H5).

Step F:

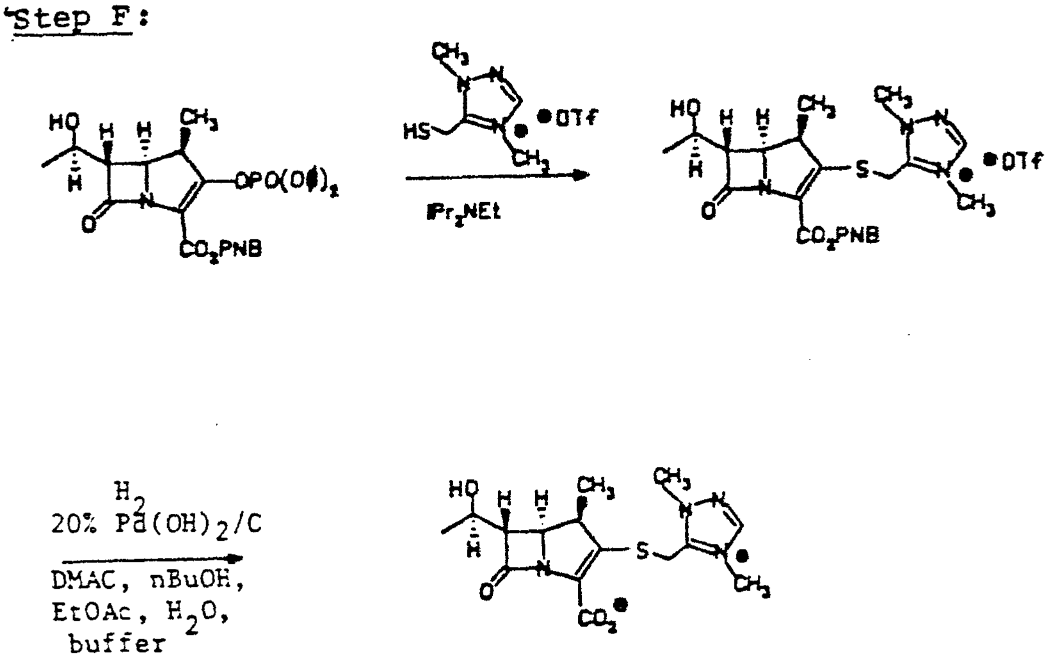


(5S,6S)-2-(1,4-Dimethyl-1,2,4-triazol-5-ium)methylthio-6-[1(R)-hydroxyethyl]-1(R)-methylcarbapen-2-em-3-carboxylate

A solution of 1,4-dimethyl-5-mercaptomethyl-1,2,4-triazolium trifluoromethanesulfonate (108 mg, 0.368 mmol) in anhydrous N,N-dimethylacetamide (2.3 ml) was cooled to -20°C under a nitrogen atmosphere and treated with p-nitrobenzyl (5S,6S)-2-(diphenylphosphono)oxy-6-[1(R)-hydroxyethyl]-1(R)-methylcarbapen-2-em-3-carboxylate (146 mg, 0.245 mmol) followed

by the dropwise addition over 6 minutes of a solution of N,N-diisopropylethylamine (64 µl, 0.368 mmol) in dimethylacetamide (0.15 ml). The resulting solution was stirred at -20°C for 30 minutes, then diluted with n-butanol (12 ml), ethyl acetate (6 ml), water (12 ml), and 0.5M pH 6.8 N-methylmorpholine hydrochloric acid buffer (6 ml), treated with 20% palladium hydroxide on carbon (75 mg), and hydrogenated at 45 psi for 90 minutes. The mixture was filtered through a celite pad to remove the catalyst which was washed with water. The aqueous portion of the filtrate was washed with methylene chloride (3x) and ethyl ether, concentrated under vacuum to ca. 5 ml, and added to a column of Dowex 50W-X4 resin (sodium form, 200-400 mesh, 1.5 x 33 cm). The column was eluted with water in a cold room, collecting 170 drop fractions. Fractions 7-12 were concentrated under vacuum to ca. 5 ml, filtered through a 0.45 µ CR acrodisc, and lyophilized to give the title compound (32.9 mg) as a white, amorphous powder.

IR (Nujol) $\nu$max 3400 (br), 1760, 1610, 1305 $cm^{-1}$;
UV (0.05M pH 7.0 MOPS buffer) $\lambda$ max 294 nm (95% $NH_2OH$ extinguished, $\epsilon$ ext. 5,570);
NMR ($D_2O$) $\delta$ 1.19 (d, J=7.2 Hz, $C\underline{H}_3CH$), 1.29 (d, J=6.4 Hz, $C\underline{H}_3CHOH$), 3.42 (dq, J=9.6 and 7.2 Hz, $CH_3C\underline{H}$), 3.57 (dd, J=3.0 and 5.8 Hz, H6), 3.94 (s, $NCH_3$), 4.09 (s, $NCH_3$), 4.28 (m, H5 and $CH_3C\underline{H}OH$), 8.76 (s, triazolium H).

EXAMPLE 11

Utilizing the procedures of Examples 1-10, the following compounds are prepared:

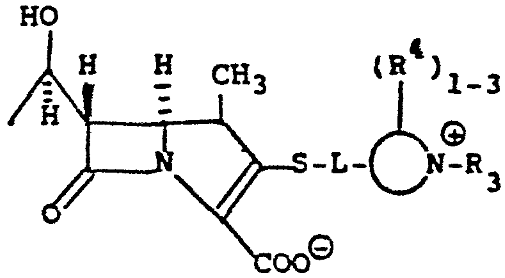

| Compound No. | L | 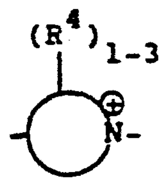 | $R^3$ |
|---|---|---|---|
| 1 | $-CH_2-$ | 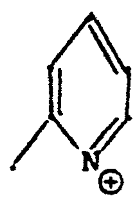 | $CH_2CH_2CH_3$ |
| 2 | $-\underset{CH_2CH_3}{\underset{\vert}{CH}}-$ | " | $CH_2CH_3$ |
| 3 | $-CH_2-$ | 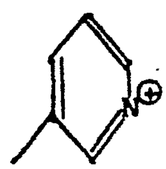 | $CH_2CH_2CH_3$ |
| 4 | " | " | $CH_2CH_3$ |
| 5 | " | 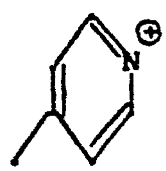 | $CH_2CH_2CH_3$ |

| | | | |
|---|---|---|---|
| 6 | $-CH_2-$ | " | $CH_2CH_3$ |
| 7 | " | 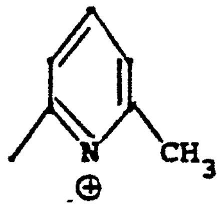 | $CH_3$ |
| 8 | " | 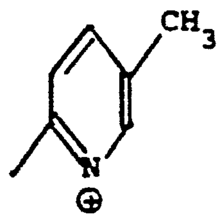 | " |
| 9 | " | 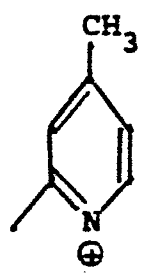 | " |
| 10 | " | 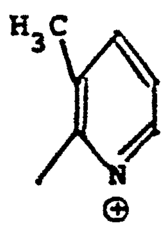 | " |
| 11 | " | 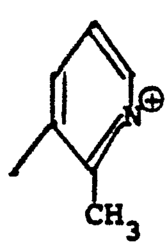 | " |

| | | | |
|---|---|---|---|
| 12 | $-CH_2-$ | 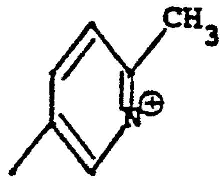 | $CH_3$ . |
| 13 | " | 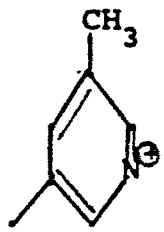 | " |
| 14 | " | 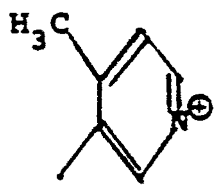 | " |
| 15 | " | 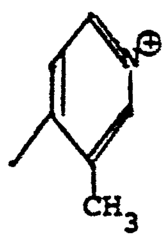 | " |
| 16 | " | 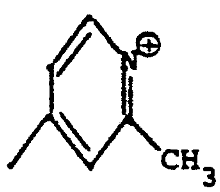 | " |
| 17 | $-CH_2CH_2-$ | 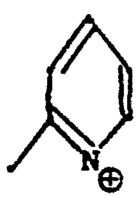 | $CH_2CH_3$ |

| | | | |
|---|---|---|---|
| 18 | $-CH_2CH_2-$ | 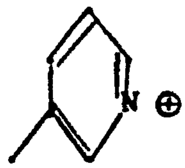 | $CH_3$ |
| 19 | " | 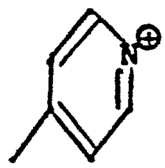 | " |
| 20 | $-CH-$<br>$CH_3$ | 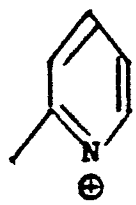 | " |
| 21 | " | 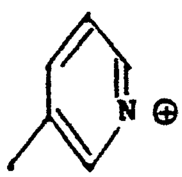 | " |
| 22 | " | 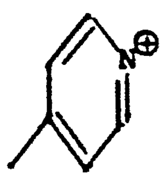 | " |
| 23 | $-CH_2-$ | 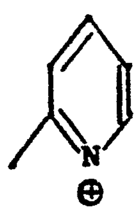 | Ø$CH_2$ |

| | | | |
|---|---|---|---|
| 24 | $-CH_2-$ | 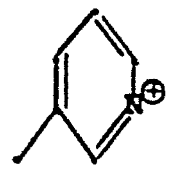 | $\emptyset CH_2$ |
| 25 | " | 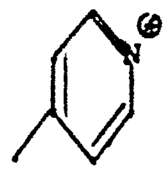 | " |
| 26 | " | 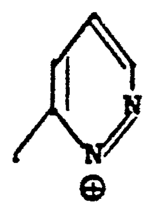 | $CH_3$ |
| 27 | " | 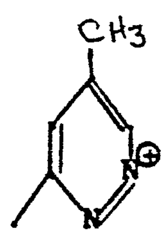 | " |
| 28 | " | 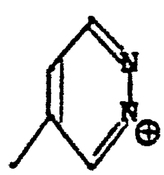 | " |
| 29 | " | 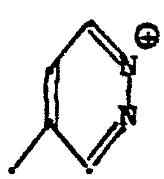 | " |

| | | | |
|---|---|---|---|
| 30 | $-CH_2-$ | 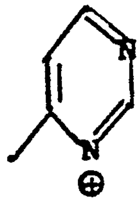 | $CH_3$ |
| 31 | " | 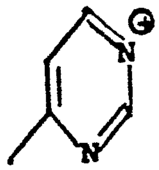 | " |
| 32 | " | 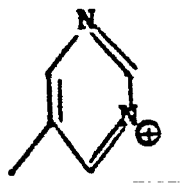 | " |
| 33 | " | 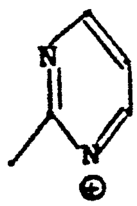 | " |
| 34 | " | 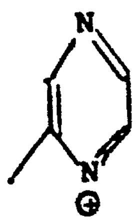 | " |
| 35 | " | 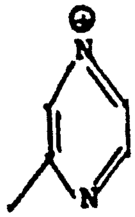 | " |

| | | | |
|---|---|---|---|
| 36 | $-CH_2-$ | 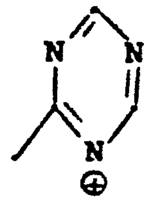 | $CH_3$ |
| 37 | " | 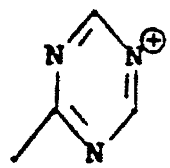 | " |
| 38 | " | 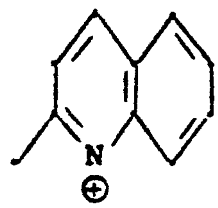 | " |
| 39 | " | 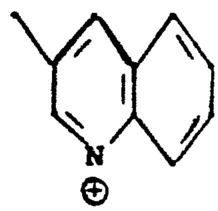 | " |
| 40 | " | 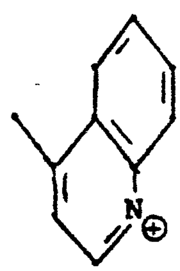 | " |
| 41 | " | 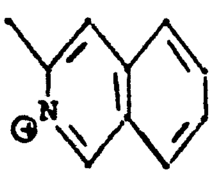 | " |

| | | | |
|---|---|---|---|
| 42 | $-CH_2-$ | 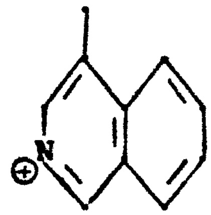 | $CH_3$ |
| 43 | " | 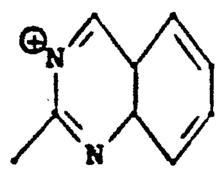 | " |
| 44 | " | 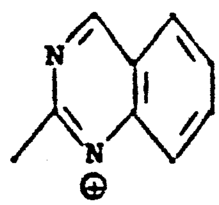 | " |
| 45 | " | 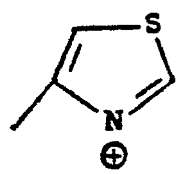 | $CH_2CH_2CH_3$ |
| 46 | " | " | $CH_2CH_3$ |
| 47 | " | 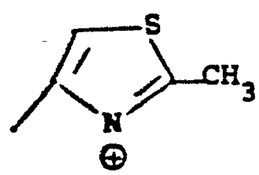 | $CH_3$ |
| 48 | " | 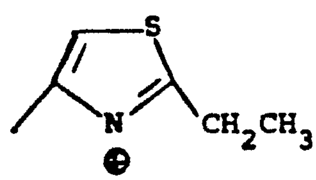 | " |

| | | | |
|---|---|---|---|
| 49 | $-CH_2-$ | 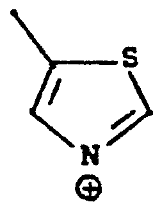 | $CH_3$ |
| 50 | " | 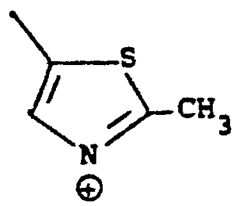 | " |
| 51 | -CH-<br>$CH_3$ | 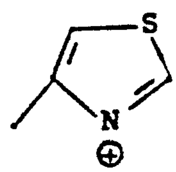 | " |
| 52 | " | 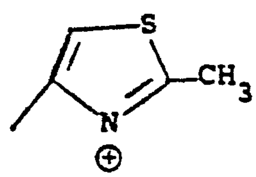 | " |
| 53 | $-CH_2-$ | 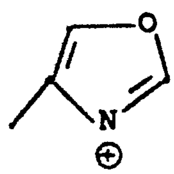 | " |
| 54 | " | " | $CH_2CH_3$ |
| 55 | " | 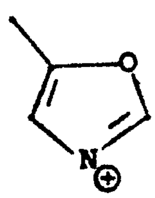 | $CH_3$ |

| | | | |
|---|---|---|---|
| 56 | $-CH_2-$ | 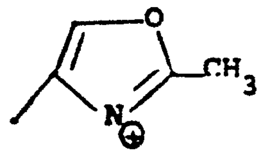 | $CH_3$ |
| 57 | " | 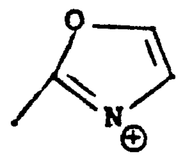 | " |
| 58 | " | 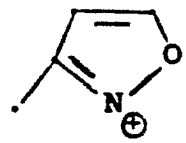 | " |
| 59 | " | 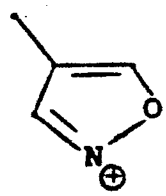 | " |
| 60 | " | 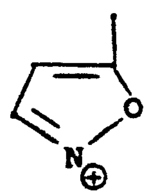 | " |
| 61 | " | 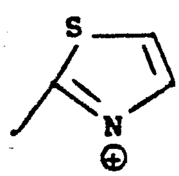 | $CH_2CH_3$ |
| 62 | $-CH_2CH_2-$ | 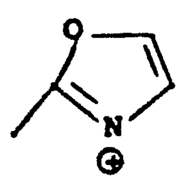 | $CH_3$ |

| | | | |
|---|---|---|---|
| 63 | $-CH_2-$ | 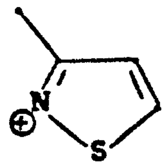 | $CH_3$ |
| 64 | " | 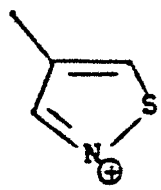 | " |
| 65 | " | 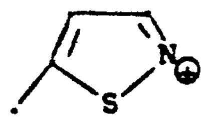 | " |
| 66 | " | 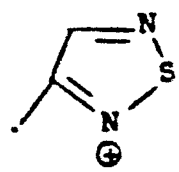 | " |
| 67 | " | 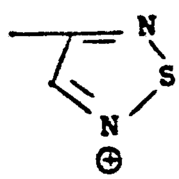 | " |
| 68 | " | 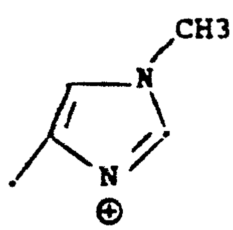 | $CH_3$ |
| 69 | " | " | $-CH_2CH_3$ |

| | | | |
|---|---|---|---|
| 70 | $-CH_2-$ | 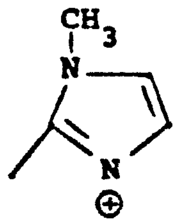 | $-CH_3$ |
| 71 | " | 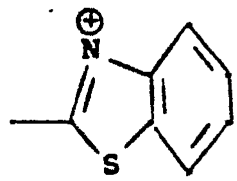 | " |
| 72 | " | 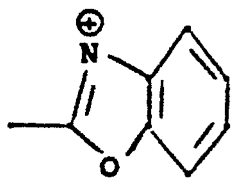 | " |
| 73 | " | 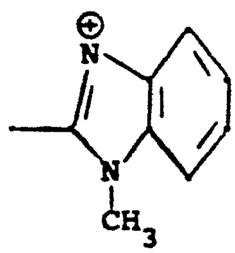 | " |
| 74 | " | 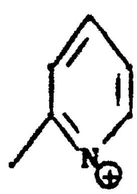 | $CH_2OCH_3$ |
| 75 | " | " | $CH_2CN$ |
| 76 | " | " | $CH_2CO_2H$ |
| 77 | " | " | $CH_2SO_2CH_3$ |

| | | | |
|---|---|---|---|
| 78 | $-CH_2-$ | " | $CH_2\overset{O}{\overset{\uparrow}{P}}(OH)OCH_3$ |
| 79 | " | " | $CH_2SO_3H$ |
| 80 | " | " | $CH_2CONMe_2$ |
| 81 | " | " | $CH_2SOCH_3$ |
| 82 | " | " | $CH_2NMe_2$ |
| 83 | " | " | $CH_2$- 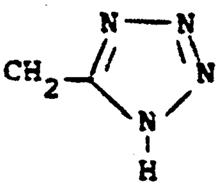 |
| 84 | " | 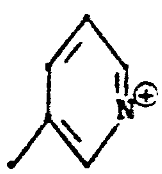 | $CH_2OCH_3$ |
| 85 | " | " | $CH_2SCH_3$ |
| 86 | " | " | $CH_2SOCH_3$ |
| 87 | " | " | $CH_2SO_2CH_3$ |
| 88 | " | " | $CH_2CO_2H$ |
| 89 | " | " | $CH_2CONMe_2$ |

0170073

| | | | |
|---|---|---|---|
| 90 | $-CH_2-$ | " | $CH_2\overset{O}{\overset{\uparrow}{P}}(OH)OCH_3$ |
| 91 | " | " | $CH_2SO_3H$ |
| 92 | " | " | $CH_2CN$ |
| 93 | " | " | $CH_2NMe_2$ |
| 94 | " | " | $CH_2CH_2NMe_2$ |
| 95 | " | 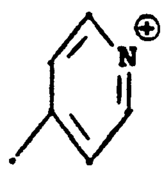 | $CH_2OCH_3$ |
| 96 | " | " | $CH_2NMe_2$ |
| 97 | " | " | $CH_2CH_2NMe_2$ |
| 98 | " | " | $CH_2CN$ |
| 99 | " | " | $CH_2SCH_3$ |
| 100 | " | " | $CH_2SOCH_3$ |
| 101 | " | " | $CH_2SO_2CH_3$ |
| 102 | " | " | $CH_2CO_2H$ |
| 103 | " | " | $CH_2CONMe_2$ |

| | | | |
|---|---|---|---|
| 104 | $-CH_2-$ | " | $CH_2\overset{O}{\overset{\uparrow}{P}}(OH)OCH_3$ |
| 105 | " | " | $CH_2SO_3H$ |
| 106 | " | 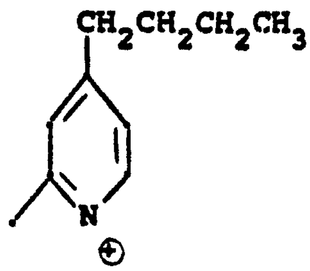 | $CH_3$ |
| 107 | $-CH_2CH_2CH_2-$ | 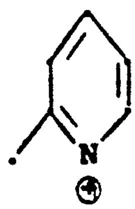 | " |
| 108 | $-CH_2\underset{CH_2OH}{\underset{\vert}{C}}H-$ | 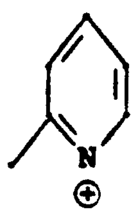 | " |
| 109 | $-CH_2-$ | 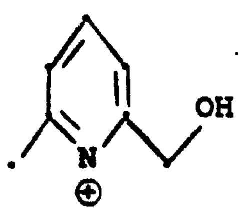 | " |

| | | | |
|---|---|---|---|
| 110 | $-CH_2-$ | 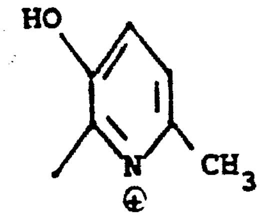 | $CH_3$ |
| 111 | " | 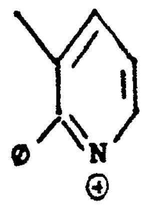 | " |
| 112 | $-CH_2CH_2CH_2-$ | 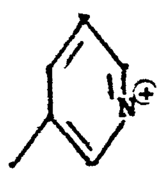 | " |
| 113 | $-CH_2CH_2-$ | 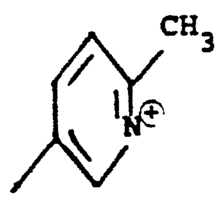 | " |
| 114 | $-CH_2-$ | 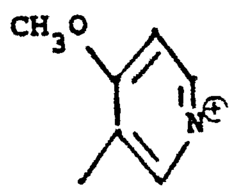 | " |
| 115 | " | 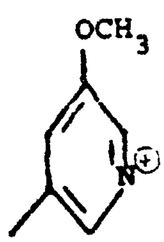 | " |

| | | | |
|---|---|---|---|
| 116 | $-CH_2-$ | 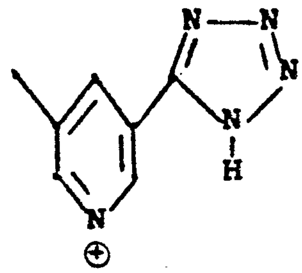 | $CH_3$ |
| 117 | $-CH_2CH_2CH_2-$ | 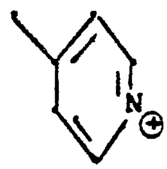 | " |
| 118 | $CH_2$ | 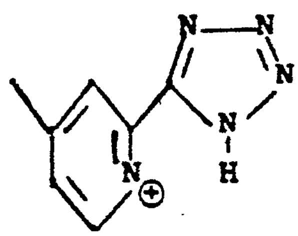 | " |
| 119 | bond | 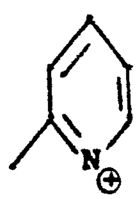 | " |
| 120 | " | 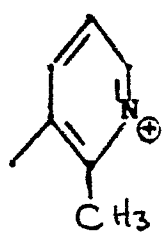 | " |
| 121 | " | 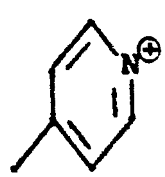 | $CH_3$ |

| | | | |
|---|---|---|---|
| 122 | bond | 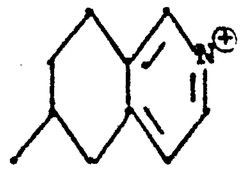 | $CH_3$ |
| 123 | $-CH_2-$ | 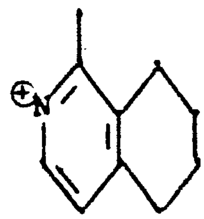 | " |
| 124 | " | 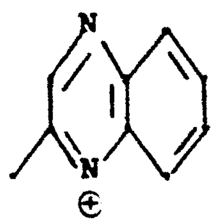 | " |
| 125 | " | 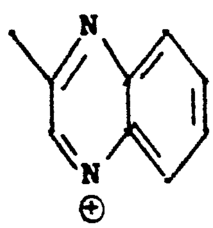 | " |
| 126 | " | 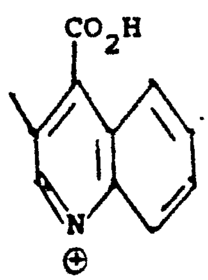 | " |

| | | | |
|---|---|---|---|
| 127 | $-CH_2-$ | 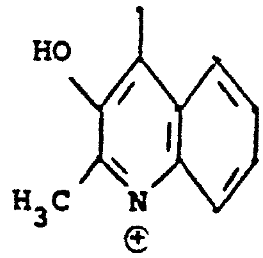 | $CH_3$ |
| 128 | $-CH_2CH_2-$ | 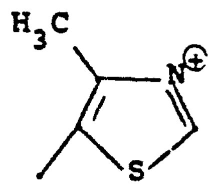 | " |
| 129 | bond | 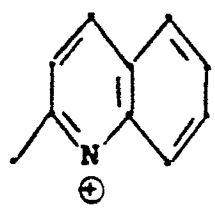 | " |
| 130 | " | 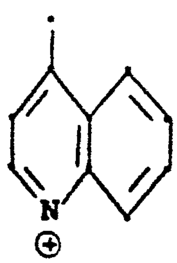 | " |
| 131 | " | 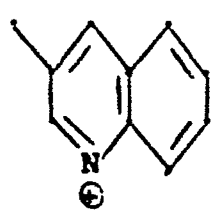 | " |

| | | | |
|---|---|---|---|
| 132 | bond | 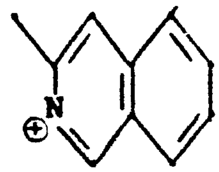 | $CH_3$ |
| 133 | " | 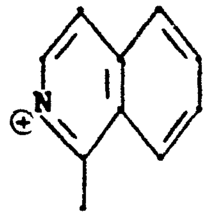 | " |
| 134 | " | 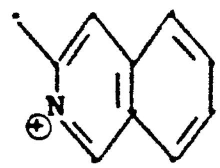 | " |
| 135 | " | 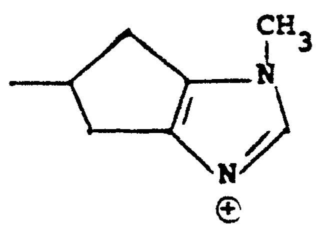 | " |
| 136 | $-\underset{CH_3}{CH}-$ | 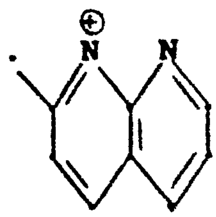 | " |

| | | | |
|---|---|---|---|
| 137 | $-CH_2-$ | 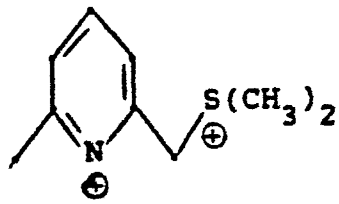 | $CH_3$ |
| 138 | " | 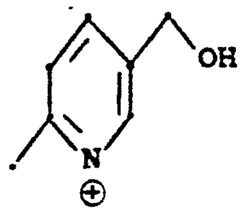 | " |
| 139 | " | 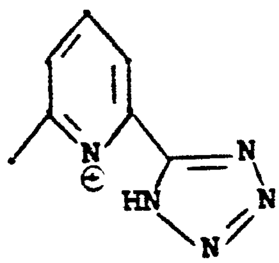 | " |
| 140 | " | 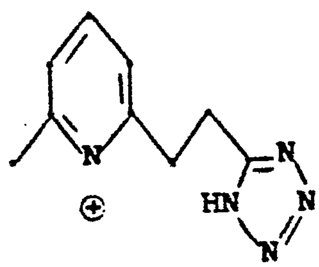 | " |
| 141 | " | 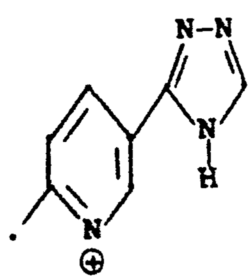 | " |

| | | | |
|---|---|---|---|
| 142 | $CH_2$ | 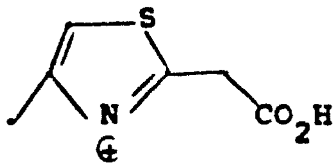 | $CH_3$ |
| 143 | " | 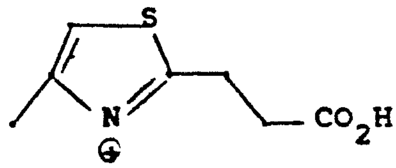 | " |
| 144 | " | 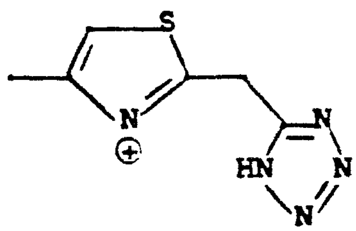 | " |
| 145 | " | 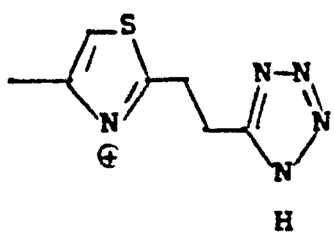 | " |
| 146 | " | 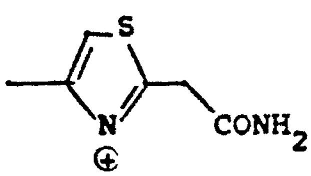 | " |
| 147 | " | 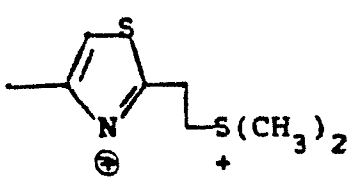 | " |

| | | | |
|---|---|---|---|
| 148 | $-CH_2-$ | 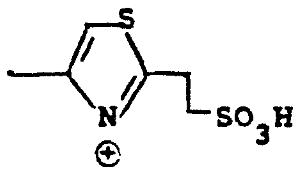 | $CH_3$ |
| 149 | " | 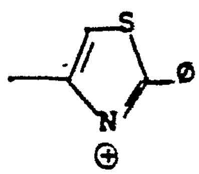 | " |
| 150 | " | 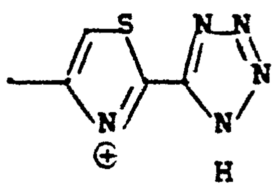 | " |
| 151 | " | 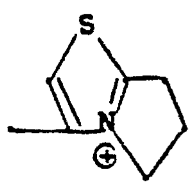 | -- |
| 152 | " | 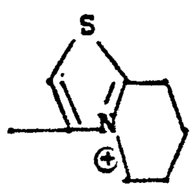 | -- |
| 153 | " | 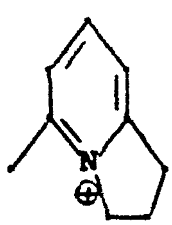 | -- |

| | | | |
|---|---|---|---|
| 154 | $-CH_2-$ | 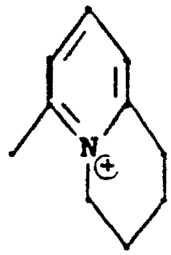 | -- |
| 155 | " | 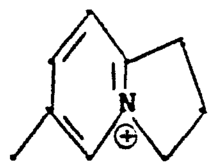 | - |
| 156 | " | 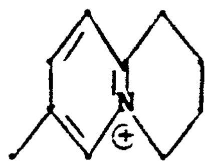 | -- |
| 157 | " | 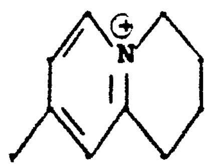 | -- |
| 158 | $-\overset{CH_3}{\overset{\vert}{C}}HCH_2-$ | 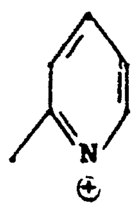 | $CH_3$ |
| 159 | $-CH_2-$ | 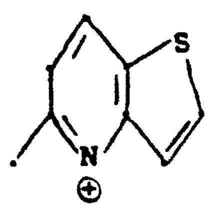 | " |

| | | | |
|---|---|---|---|
| 160 | $-CH_2-$ | 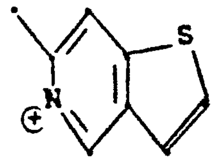 | $CH_3$ |
| 161 | " | 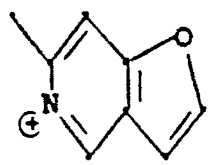 | " |
| 162 | " | 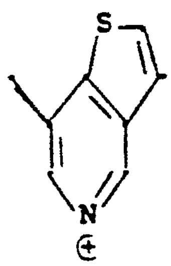 | " |
| 163 | " | 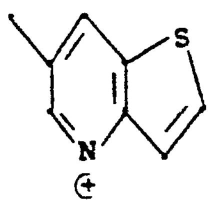 | " |
| 164 | " | 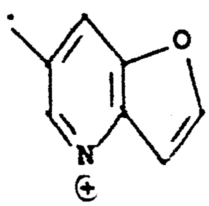 | " |
| 165 | " | 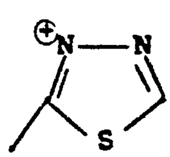 | " |

| | | | |
|---|---|---|---|
| 166 | $-CH_2-$ | 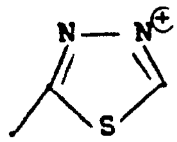 | $CH_3$ |
| 167 | " | 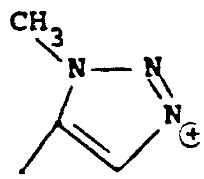 | " |
| 168 | " | 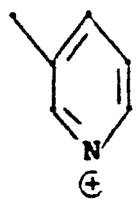 | $CH_2CONH_2$ |
| 169 | " | 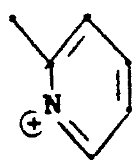 | " |
| 170 | " | 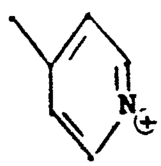 | " |
| 171 | " | 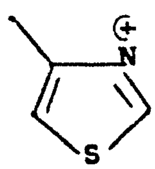 | " |
| 172 | bond | 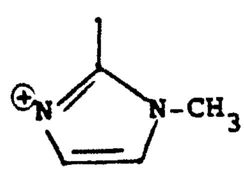 | $CH_3$ |

| | | | |
|---|---|---|---|
| 173 | bond | 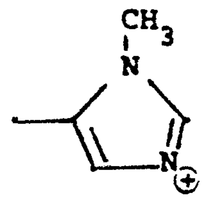 | $CH_3$ |
| 174 | " | 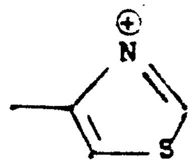 | " |
| 175 | " | 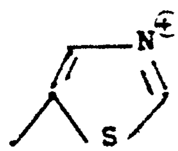 | " |
| 176 | " | 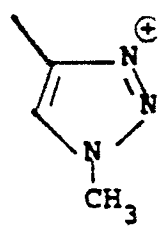 | " |
| 177 | " | 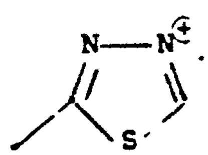 | " |
| 178 | " | 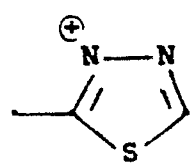 | " |
| 179 | " | 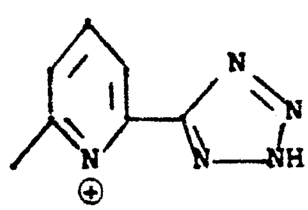 | " |

| | | | |
|---|---|---|---|
| 180 | $-CH_2-$ | 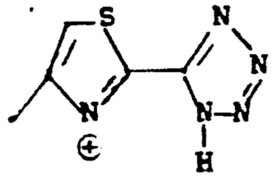 | $CH_3$ |
| 181 | " | 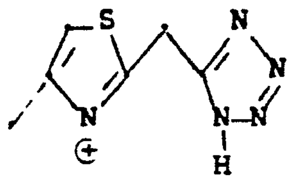 | " |
| 182 | " | 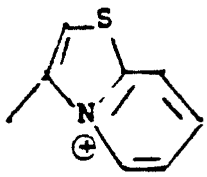 | -- |
| 183 | bond | 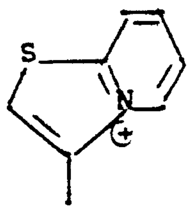 | -- |
| 184 | $CH_2$ | 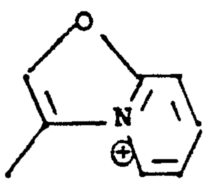 | -- |
| 185 | bond | 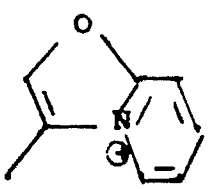 | -- |

EXAMPLE 12

Utilizing the procedures of Examples 1- 10, the following compounds are prepared:

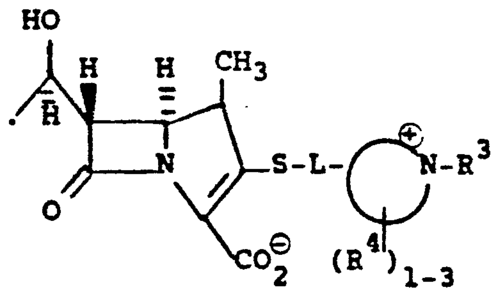

| Compound No. | L | 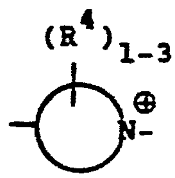 | $R_3$ | $R_4$ |
|---|---|---|---|---|
| 1 | $CH_2$ | 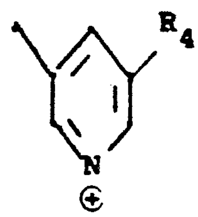 | $CH_3$ | $CO_2H$ |
| 2 | " | " | " | $CONH_2$ |
| 3 | " | " | " | CN |
| 4 | " | " | " | OH |
| 5 | " | " | " | $SO_2NH_2$ |
| 6 | " | " | " | $SO_3H$ |
| 7 | " | " | " | $NMe_2$ |
| 8 | " | " | " | $CONMe_2$ |

| | | | | |
|---|---|---|---|---|
| 9 | " | " | " | $CH_2NMe_2$ |
| 10 | " | " | " | $CH_2CN$ |
| 11 | " | " | " | $CH_2CONH_2$ |
| 12 | " | " | " | $CH_2CO_2H$ |
| 13 | " | " | " | $CH_2SCH_3$ |
| 14 | " | " | " | $CH_2SOCH_3$ |
| 15 | " | " | " | $CH_2SO_2CH_3$ |
| 16 | " | " | " | $SO_2CH_3$ |
| 17 | " | " | " | $SOCH_3$ |
| 18 | " | " | " | 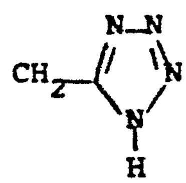 |
| 19 | " | " | " | $CH_2CH_2CO_2H$ |
| 20 | " | " | " | $CH_2SO_3H$ |
| 21 | " | " | " | $CH_2OCH_3$ |
| 22 | " | " | " | 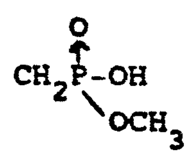 |

| | | | | |
|---|---|---|---|---|
| 23 | " | " | " | $CH_2CH_2SO_3H$ |
| 24 | " | " | " | $CF_3$ |
| 25 | " | " | " | $CH_2O\overset{O}{\overset{\|}{C}}NH_2$ |
| 26 | " | " | " | $CH_2SO_2NH_2$ |
| 27 | " | " | " | Br |
| 28 | " | " | " | Cl |
| 29 | " | " | " | F |
| 30 | " | 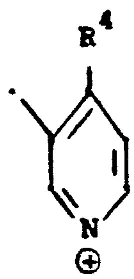 | " | $CO_2H$ |
| 31 | " | " | " | $CONH_2$ |
| 32 | " | " | " | CN |
| 33 | " | " | " | OH |
| 34 | " | " | " | $SONH_2$ |

| | | | | |
|---|---|---|---|---|
| 35 | " | " | " | $SO_3H$ |
| 36 | " | " | " | $NMe_2$ |
| 37 | " | " | " | $CONMe_2$ |
| 38 | " | " | " | $CH_2NMe_2$ |
| 39 | " | " | " | $CH_2CN$ |
| 40 | " | " | " | $CH_2CONH_2$ |
| 41 | " | " | " | $CH_2CO_2H$ |
| 41 | " | " | " | $CH_2SCH_3$ |
| 43 | " | " | " | $CH_2SOCH_3$ |
| 44 | " | " | " | $CH_2SO_2CH_3$ |
| 45 | " | " | " | $SO_2CH_3$ |
| 46 | " | " | " | $SOCH_3$ |
| 47 | " | " | " | 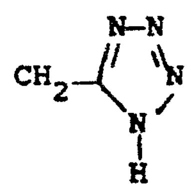 |
| 48 | " | " | " | $CH_2CH_2CO_2H$ |
| 49 | " | " | " | $CH_2SO_3H$ |
| 50 | " | " | " | $CH_2OCH_3$ |

| | | | |
|---|---|---|---|
| 51 | " | " | " 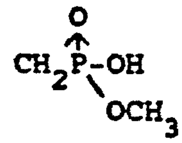 |
| 52 | " | " | " $CH_2CH_2SO_3H$ |
| 53 | " | " | " $CF_3$ |
| 54 | " | " | " 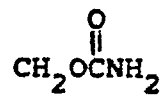 |
| 55 | " | " | " $CH_2SO_2NH_2$ |
| 56 | " | " | " $CH_2SO_2NMe_2$ |
| 57 | " | 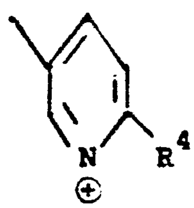 | " $CO_2H$ |
| 58 | " | " | " $CONH_2$ |
| 59 | " | " | " CN |
| 60 | " | " | " $OCH_3$ |
| 61 | " | " | " $SO_2NH_2$ |
| 62 | " | " | " $SO_3H$ |
| 63 | " | " | " $NMe_2$ |

| | | | | |
|---|---|---|---|---|
| 64 | " | " | " | $CONMe_2$ |
| 65 | " | " | " | $CH_2NMe_2$ |
| 66 | " | " | " | $CH_2CN$ |
| 67 | " | " | " | $CH_2CONH_2$ |
| 68 | " | " | " | $CH_2CO_2H$ |
| 69 | " | " | " | $CH_2SCH_3$ |
| 70 | " | " | " | $CH_2SOCH_3$ |
| 71 | " | " | " | $CH_2SO_2CH_3$ |
| 72 | " | " | " | $SO_2CH_3$ |
| 73 | " | " | " | $SOCH_3$ |
| 74 | " | " | " | $CH_2$ 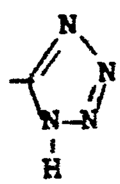 |
| 75 | " | " | " | $CH_2CH_2CO_2H$ |
| 76 | " | " | " | $CH_2SO_3H$ |
| 77 | " | " | " | $CH_2OCH_3$ |

0170073

| | | | | |
|---|---|---|---|---|
| 78 | " | " | " | 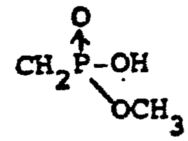 |
| 79 | " | " | " | $CH_2CH_2SO_3H$ |
| 80 | " | " | " | $CF_3$ |
| 81 | " | " | " | 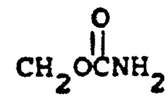 |
| 82 | " | " | " | $CH_2SO_2NH_2$ |
| 83 | " | " | " | $CH_2SO_2NMe_2$ |
| 84 | " | " | " | 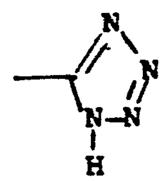 |
| 85 | " | 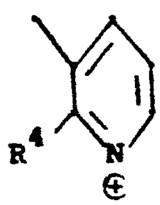 | " | $CO_2H$ |
| 86 | " | " | " | $CONH_2$ |
| 87 | " | " | " | CN |
| 88 | " | " | " | $OCH_3$ |

| | | | | |
|---|---|---|---|---|
| 89 | " | " | " | $SO_2NH_2$ |
| 90 | " | " | " | $SO_3H$ |
| 91 | " | " | " | $NMe_2$ |
| 92 | " | " | " | $CONMe_2$ |
| 93 | " | " | " | $CH_2NMe_2$ |
| 94 | " | " | " | $CH_2CN$ |
| 95 | " | " | " | $CH_2CONH_2$ |
| 96 | " | " | " | $CH_2CO_2H$ |
| 97 | " | " | " | $CH_2SCH_3$ |
| 98 | " | " | " | $CH_2SOCH_3$ |
| 99 | $CH_3$ | " | " | $CH_2SO_2CH_3$ |
| 100 | " | " | " | $SO_2CH_3$ |
| 101 | " | " | " | $SOCH_3$ |
| 102 | " | " | " | 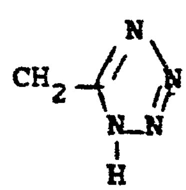 |
| 103 | " | " | " | $CH_2CH_2CO_2H$ |

| | | | | |
|---|---|---|---|---|
| 104 | " | " | " | $CH_2SO_3H$ |
| 105 | " | " | " | $CH_2OCH_3$ |
| 106 | " | " | " | 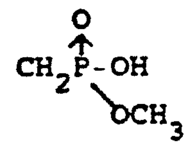 |
| 107 | " | " | " | $CH_2CH_2SO_3H$ |
| 108 | " | " | " | $CF_3$ |
| 109 | " | " | " | 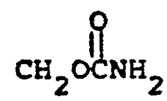 |
| 110 | " | " | " | $CH_2SO_2NH_2$ |
| 111 | " | " | " | $CH_2SO_2NMe_2$ |
| 112 | " | " | " | 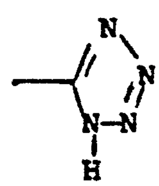 |
| 113 | $-CH_2-$ | 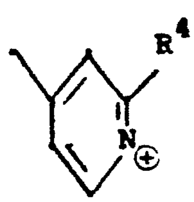 | " | $CO_2H$ |
| 114 | " | " | " | $CONH_2$ |
| 115 | " | " | " | CN |

| | | | | |
|---|---|---|---|---|
| 116 | " | " | " | $OCH_3$ |
| 117 | " | " | " | $SO_2NH_2$ |
| 118 | " | " | " | $SO_3H$ |
| 119 | " | " | " | $NMe_2$ |
| 120 | " | " | " | $CONMe_2$ |
| 121 | " | " | " | $CH_2NMe_2$ |
| 122 | " | " | " | $CH_2CN$ |
| 123 | " | " | " | $CH_2CONH_2$ |
| 124 | " | " | " | $CH_2CO_2H$ |
| 125 | " | " | " | $CH_2SCH_3$ |
| 126 | " | " | " | $CH_2SOCH_3$ |
| 127 | " | " | " | $CH_2SO_2CH_3$ |
| 128 | " | " | " | $SO_2CH_3$ |
| 129 | " | " | " | $SOCH_3$ |
| 130 | " | " | " | 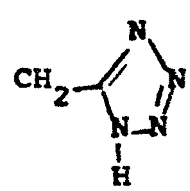 |

| | | | | |
|---|---|---|---|---|
| 131 | " | " | " | $CH_2CH_2CO_2H$ |
| 132 | " | " | " | $CH_2SO_3H$ |
| 133 | " | " | " | $CH_2OCH_3$ |
| 134 | " | " | " | 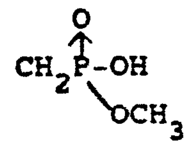 |
| 135 | " | " | " | $CH_2CH_2SO_3H$ |
| 136 | " | " | " | $CF_3$ |
| 137 | " | " | " | 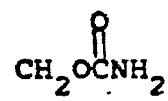 |
| 138 | " | " | " | $CH_2SO_2NH_2$ |
| 139 | " | " | " | $CH_2SO_2NMe_2$ |
| 140 | " | " | " | 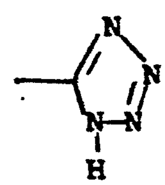 |
| 141 | " | 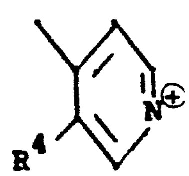 | " | $CO_2H$ |
| 142 | " | " | " | $CONH_2$ |

| | | | | |
|---|---|---|---|---|
| 143 | " | " | " | CN |
| 144 | " | " | " | OH |
| 145 | " | " | " | $OCH_3$ |
| 146 | " | " | " | $SO_2NH_2$ |
| 147 | " | " | " | $SO_3H$ |
| 148 | " | " | " | $NMe_2$ |
| 149 | " | " | " | $CONMe_2$ |
| 150 | " | " | " | $CH_2NMe_2$ |
| 151 | " | " | " | $CH_2CN$ |
| 152 | " | " | " | $CH_2CONH_2$ |
| 153 | " | " | " | $CH_2CO_2H$ |
| 154 | " | " | " | $CH_2SCH_3$ |
| 155 | " | " | " | $CH_2SOCH_3$ |
| 156 | " | " | " | $CH_2SO_2CH_3$ |
| 157 | " | " | " | $SO_2CH_3$ |
| 158 | " | " | " | $SOCH_3$ |

| | | | | |
|---|---|---|---|---|
| 159 | " | " | " | 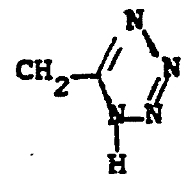 |
| 160 | " | " | " | $CH_2CH_2CO_2H$ |
| 161 | " | " | " | $CH_2SO_3H$ |
| 162 | " | " | " | $CH_2OCH_3$ |
| 163 | " | " | " | 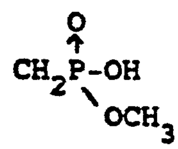 |
| 164 | " | " | " | $CH_2CH_2SO_3H$ |
| 165 | " | " | " | $CF_3$ |
| 166 | " | " | " | 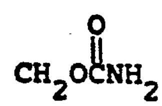 |
| 167 | " | " | " | $CH_2SO_2NH_2$ |
| 168 | " | " | " | $CH_2SO_2NMe_2$ |
| 169 | " | " | " | 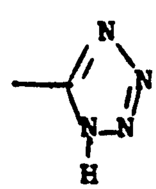 |

| | | | | |
|---|---|---|---|---|
| 170 | " | " | " | F |
| 171 | " | " | " | Cl |
| 172 | " | " | " | Br |
| 173 | " | 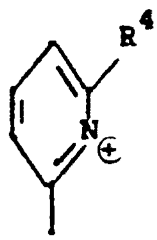 | " | $CO_2H$ |
| 174 | " | " | " | $CONH_2$ |
| 175 | " | " | " | CN |
| 176 | " | " | " | $SO_2NH_2$ |
| 177 | " | " | " | $SO_3H$ |
| 178 | " | " | " | $NMe_2$ |
| 179 | " | " | " | $CONMe_2$ |
| 180 | " | " | " | $CH_2NMe_2$ |
| 181 | " | " | " | $CH_2CN$ |
| 182 | " | " | " | $CH_2CONH_2$ |
| 183 | " | " | " | $CH_2CO_2H$ |

| | | | | |
|---|---|---|---|---|
| 184 | " | " | " | $CH_2SCH_3$ |
| 185 | " | " | " | $CH_2SOCH_3$ |
| 186 | " | " | " | $CH_2SO_2CH_3$ |
| 187 | " | " | " | $SO_2CH_3$ |
| 188 | " | " | " | 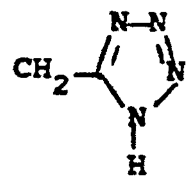 |
| 189 | " | " | " | $CH_2CH_2CO_2H$ |
| 190 | " | " | " | $CH_2SO_3H$ |
| 191 | " | " | " | $CH_2OCH_3$ |
| 192 | " | " | " | 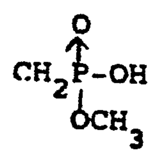 |
| 193 | " | " | " | $CH_2CH_2SO_3H$ |
| 194 | " | " | " | $CF_3$ |
| 195 | " | " | " | 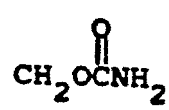 |
| 196 | " | " | " | $CH_2SO_2NH_2$ |

| | | | | |
|---|---|---|---|---|
| 197 | " | 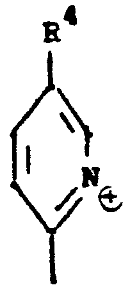 | " | $CO_2H$ |
| 198 | " | " | " | $CONH_2$ |
| 199 | " | " | " | CN |
| 200 | " | " | " | OH |
| 201 | " | " | " | $SO_2NH_2$ |
| 202 | " | " | " | $SO_3H$ |
| 203 | " | " | " | $NMe_2$ |
| 204 | " | " | " | $CONMe_2$ |
| 205 | " | " | " | $CH_2NMe_2$ |
| 206 | " | " | " | $CH_2CN$ |
| 207 | " | " | " | $CH_2CONH_2$ |
| 208 | " | " | " | $CH_2CO_2H$ |
| 209 | " | " | " | $CH_2SCH_3$ |
| 210 | " | " | " | $CH_2SOCH_3$ |

| | | | | |
|---|---|---|---|---|
| 211 | " | " | " | $CH_2SO_2CH_3$ |
| 212 | " | " | " | $SO_2CH_3$ |
| 213 | " | " | " | $SOCH_3$ |
| 214 | " | " | " | 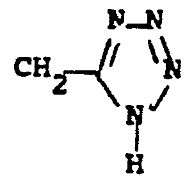 |
| 215 | " | " | " | $CH_2CH_2CO_2H$ |
| 216 | " | " | " | $CH_2SO_3H$ |
| 217 | " | " | " | $CH_2OCH_3$ |
| 218 | " | " | " | 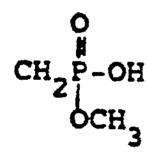 |
| 219 | " | " | " | $CH_2CH_2SO_3H$ |
| 220 | " | " | " | $CF_3$ |
| 221 | " | " | " | 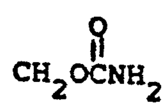 |
| 222 | " | " | " | $CH_2SO_2NH_2$ |
| 223 | " | " | " | Br |

| | | | | |
|---|---|---|---|---|
| 224 | " | " | " | Cl |
| 225 | " | " | " | F |
| 226 | " | 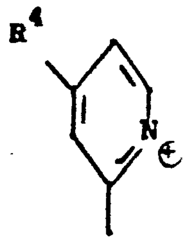 | " | $CO_2H$ |
| 227 | " | " | " | $CONH_2$ |
| 228 | " | " | " | CN |
| 229 | " | " | " | $SO_2NH_2$ |
| 230 | " | " | " | $SO_3H$ |
| 231 | " | " | " | $NMe_2$ |
| 232 | " | " | " | $CONMe_2$ |
| 233 | " | " | " | $CH_2NMe_2$ |
| 234 | " | " | " | $CH_2CN$ |
| 235 | " | " | " | $CH_2CONH_2$ |
| 236 | " | " | " | $CH_2CO_2H$ |
| 237 | " | " | " | $CH_2SCH_3$ |

| | | | | |
|---|---|---|---|---|
| 238 | " | " | " | $CH_2SOCH_3$ |
| 239 | " | " | " | $CH_2SO_2CH_3$ |
| 240 | " | " | " | $SO_2CH_3$ |
| 241 | " | " | " | $SOCH_3$ |
| 242 | " | " | " | 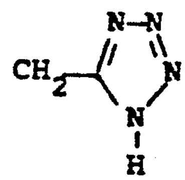 |
| 243 | " | " | " | $CH_2CH_2CO_2H$ |
| 244 | " | " | " | $CH_2SO_3H$ |
| 245 | " | " | " | $CH_2OCH_3$ |
| 246 | " | " | " | 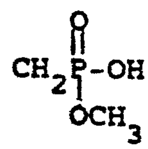 |
| 247 | " | " | " | $CH_2CH_2SO_3H$ |
| 248 | " | " | " | $CF_3$ |
| 249 | " | " | " | 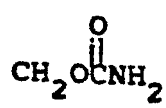 |
| 250 | " | " | " | $CH_2SO_2NH_2$ |

| | | | | |
|---|---|---|---|---|
| 251 | " | 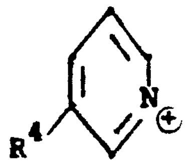 | " | $CO_2H$ |
| 252 | " | " | " | $CONH_2$ |
| 253 | " | " | " | CN |
| 254 | " | " | " | OH |
| 255 | " | " | " | $SO_2NH_2$ |
| 256 | " | " | " | $SO_3H$ |
| 257 | " | " | " | $NMe_2$ |
| 258 | " | " | " | $CONMe_2$ |
| 259 | " | " | " | $CH_2NMe_2$ |
| 260 | " | " | " | $CH_2CN$ |
| 261 | " | " | " | $CH_2CONH_2$ |
| 262 | " | " | " | $CH_2CO_2H$ |
| 263 | " | " | " | $CH_2SCH_3$ |
| 264 | " | " | " | $CH_2SOCH_3$ |

| | | | | |
|---|---|---|---|---|
| 265 | " | " | " | $CH_2SO_2CH_3$ |
| 266 | " | " | " | $SO_2CH_3$ |
| 267 | " | " | " | $SOCH_3$ |
| 268 | " | " | " | 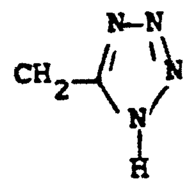 |
| 269 | " | " | " | $CH_2CH_2CO_2H$ |
| 270 | " | " | " | $CH_2SO_3H$ |
| 271 | " | " | " | $CH_2OCH_3$ |
| 272 | " | " | " | 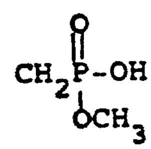 |
| 273 | " | " | " | $CH_2CH_2SO_3H$ |
| 274 | " | " | " | $CF_3$ |
| 275 | " | " | " | 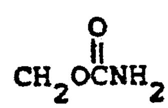 |
| 276 | " | " | " | $CH_2SO_2NH_2$ |

| | | | | |
|---|---|---|---|---|
| 277 | " | " | " | Br |
| 278 | " | " | " | Cl |
| 279 | " | " | " | F |

WHAT IS CLAIMED IS:

1. A compound having the formula:

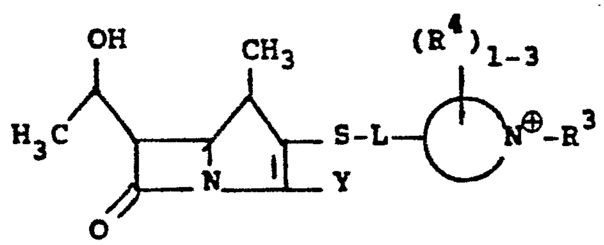

I

wherein:

L is a covalent bond or a bridging group selected from $-(CH_2)_{1-4}S-$; $-(CH_2)_{1-4}-O-$; $-(CH_2)_{1-4}-X-(CH_2)_{1-4}$ where X is O, S, NH, or $N(C_1-C_6)alkyl)$; substituted or unsubstituted $C_1-C_4$ straight, $C_1-C_6$ branched or $C_3-C_7$ cycloalkyl groups wherein the substituents are selected from $C_1-C_6$ alkyl, $O-C_1-C_6$ alkyl, $S-C_1-C_6$ alkyl, halo, OH, $CF_3$, CN, $NH_2$, $NHC_1-C_6$ alkyl, $N(C_1-C_6$ alkyl$)_2$, $CO_2H$, $CONH_2$, CONH $(C_1-C_6$ alkyl), and $CON(C_1-C_6$ alkyl$)_2$;

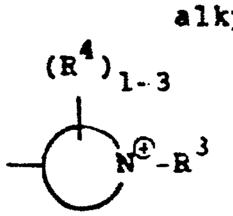 is a mono- or bicyclic heteroarylium group containing from 5-11 ring atoms of which up to 5 are heteroatoms wherein $R^3$ is:

1) an unsubstituted or substituted $C_1-C_6$ alkyl radical;
2) an unsubstituted or substituted $C_1-C_6$ alkenyl radical;

3) an unsubstituted or substituted $C_1$-$C_6$ alkynyl radical;

4) a $C_3$-$C_7$ cycloalkyl radical in which the ring is substituted or unsubstituted and one or more atoms may be replaced by a heteroatom;

5) a $C_3$-$C_7$ cycloalkyl methyl radical in which the ring may be substituted and one or more atoms may be replaced by a heteroatom;

6) an unsubstituted or substituted $C_5$-$C_7$ cycloalkenyl radical;

7) an unsubstituted or substituted bivalent $C_2$-$C_6$ alkylidene radical, optionally interrupted by a heteroatom, and joined to the heteroarylium group to form a ring which is carbocyclic or in which one or more atoms is replaced by a heteroatom. The new ring may contain one or more double bonds;

8) an unsubstituted or substituted phenyl or heteroaryl radical;

9) an unsubstituted or substituted phenyl ($C_1$-$C_4$ alkyl) or heteroaryl ($C_1$-$C_4$ alkyl) radical;

10) a cyano ($C_1$-$C_4$ alkyl) radical;

11) a carbamoyl ($C_1$-$C_4$ alkyl) radical;

12) a hydroxy ($C_1$-$C_4$ alkyl) radical;

13) an amino ($C_1$-$C_4$ alkyl) radical in which the nitrogen atom is unsubstituted or substituted with one to three $C_1$-$C_4$ alkyl groups;

14) an acidic side-chain of the structure $-(CH_2)_n-X-(CH_2)_m-Y-A$ where:

n = 0-4

m = 0-4

X = $CHR^8$, CH=CH, phenylene ($-C_6H_4-$), NH, N(C1-C4 alkyl), O, S, S=O, C=O, $SO_2$, $SO_2NH$, $CO_2$, CONH, $OCO_2$, OC=O, NHC=O;

$R^8$ = H, O(C1-C4 alkyl), $NH_2$, NH(C1-C4 alkyl), N(C1-C4 alkyl)$_2$, CN, $CONH_2$, CON(C1-C4 alkyl)$_2$, $CO_2H$, $SO_2NH_2$, $SO_2$NH(C1-C4 alkyl);

Y = single bond, NH, N(C1-C4 alkyl), O, S;

A = an acidic function such as carboxy ($CO_2H$), phosphono [$P=O(OH)_2$], alkylphosphono {P=O(OH)-[C($C_1$-$C_4$ alkyl)]}, alkylphosphinyl [P=O(OH)-($C_1$-$C_4$ alkyl)], substituted phosphoramido [P=O(OH)NH($C_1$-$C_4$ alkyl) and $P=O(OH)NHR^x$], sulfino ($SO_2H$), sulfo ($SO_3H$), 5-tetrazolyl ($CN_4H$), arylsulfonamido ($SO_2NHR^x$) and acylsulfonamides represented by the structures $CONHSO_2$($C_1$-$C_4$ alkyl), $CONHSO_2N$($C_1$-$C_4$alkyl)$_2$ - $SO_2NHCO$($C_1$-$C_4$ alkyl) and $SO_2NHCOR^x$;

$R^x$ = aryl or heteroaryl as defined above;

wherein the substituents in the above definitions of $R^3$ are independently selected from the group consisting of the definitions of $R^4$ set out below;

$R^4$ is independently selected from:

a) a trifluoromethyl group;

b) a halogen atom;

c) an unsubstituted or substituted $C_1$-$C_4$ alkoxyl radical;

d) a hydroxy group;

e) an unsubstituted or substituted ($C_1$-$C_6$ alkyl) carbonyloxy radical;

f) a carbamoyloxy radical which is unsubstituted or substituted on nitrogen with one or two $C_1$-$C_4$ alkyl groups;

g) a $C_1$-$C_6$ alkylthio radical, $C_1$-$C_6$ alkylsulfinyl radical or $C_1$-$C_6$ alkylsulfonyl radical, each of which is unsubstituted or substituted on the alkyl group;

h) a sulfamoyl group which is unsubstituted or substituted on nitrogen by one or two $C_1$-$C_4$ alkyl groups;

i) an amino group;

j) a mono ($C_1$-$C_4$ alkyl) amino or di($C_1$-$C_4$ alkyl)amino group, each of which is unsubstituted or substituted on the alkyl group;

k) a formylamino group;

l) an unsubstituted or substituted ($C_1$-$C_6$ alkyl)carbonylamino radical;

m) a ($C_1$-$C_4$ alkoxyl) carbonylamino radical;

n) a ureido group in which the terminal nitrogen is unsubstituted or substituted with one or two $C_1$-$C_4$ alkyl groups;

o) a ($C_1$-$C_6$ alkyl)sulfonamido group;

p) a cyano group;

q) a formyl or acetalized formyl radical;

r) an unsubstituted or substituted ($C_1$-$C_6$ alkyl)carbonyl radical wherein the carbonyl is free or acetalized;

s) an unsubstituted or substituted phenylcarbonyl or heteroarylcarbonyl radical;

t) a hydroximinomethyl radical in which the oxygen or carbon atom is optionally substituted by a $C_1$-$C_4$ alkyl group;

u) a ($C_1$-$C_6$ alkoxy)carbonyl radical;

v) a carbamoyl radical which is unsubstituted or substituted on nitrogen by one or two $C_1$-$C_4$ alkyl groups;

w) an N-hydroxycarbamoyl or N($C_1$-$C_4$ alkoxy)carbamoyl radical in which the nitrogen atom may be additionally substituted by a $C_1$-$C_4$ alkyl group;

x) a thiocarbamoyl group;

y) an amidino group 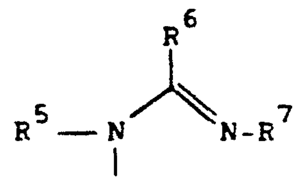

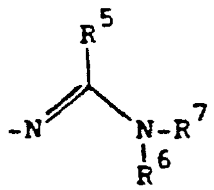

where $R^5$, $R^6$ and $R^7$ are independently hydrogen, $C_1$-$C_4$alkyl or wherein two of the alkyl groups together form a $C_2$-$C_6$alkylidene radical optionally interrupted by a heteroatom and joined together to form a ring;

z) a carboxamidino group 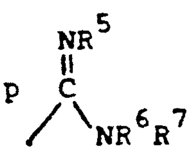 , where $R^5$, $R^6$ and $R^7$ are as defined above;

aa) a guanidinyl group where $R^6$ in ab) above is $NR^8R^9$ and $R^8$ and $R^9$ are as defined for $R^5$ through $R^7$ above.

ab) hydrogen;

ac) an unsubstituted or substituted $C_1$-$C_6$ alkyl radical;

ad) an unsubstituted or substituted $C_1$-$C_6$ alkenyl radical;

ae) an unsubstituted or substituted $C_1$-$C_6$ alkynyl radical;

af) a $C_3$-$C_7$ cycloalkyl radical in which the ring is substituted or unsubstituted and one or more atoms may be replaced by a heteroatom;

ag) a $C_3$-$C_7$ cycloalkyl methyl radical in which the ring may be substituted and one or more atoms may be replaced by a heteroatom;

ah) an unsubstituted or substituted $C_5$-$C_7$ cycloalkenyl radical;

ai) an unsubstituted or substituted phenyl or heteroaryl radical;

aj) an unsubstituted or substituted phenyl ($C_1$-$C_4$ alkyl) or heteroaryl ($C_1$-$C_4$ alkyl) radical; and

ak) an acidic side-chain of the structure

$-A$ or $-(CH_2)_n-X-(CH_2)_m-Y-A$ where:

n = 0-4

m = 0-4

X = $CHR^8$, CH=CH, phenylene ($-C_6H_4-$), NH, N(C1-C4 alkyl), O, S, S=O, C=O, $SO_2$, $SO_2NH$, $CO_2$, CONH, $OCO_2$, OC=O, NHC=O;

$R^8$ = H, O(C1-C4 alkyl), $NH_2$, NH(C1-C4 alkyl), N(C1-C4 alkyl)$_2$, CN, $CONH_2$, CON(C1-C4 alkyl)$_2$, $CO_2H$, $SO_2NH_2$, $SO_2NH$(C1-C4 alkyl);

Y = single bond, NH, N(C1-C4 alkyl), O, S;

A = an acidic function such as carboxy ($CO_2H$), phosphono [$P=O(OH)_2$], alkylphosphono {P=O(OH)-[C($C_1$-$C_4$ alkyl)]}, alkylphosphinyl [P=O(OH)-($C_1$-$C_4$ alkyl)], substituted phosphoramido [P=O(OH)NH($C_1$-$C_4$ alkyl) and $P=O(OH)NHR^X$], sulfino ($SO_2H$), sulfo ($SO_3H$), 5-tetrazolyl ($CN_4H$), arylsulfonamido ($SO_2NHR^X$) and acylsulfonamides represented by the structures $CONHSO_2$($C_1$-$C_4$ alkyl), $CONHSO_2N$($C_1$-$C_4$alkyl)$_2$ - $SO_2NHCO$($C_1$-$C_4$ alkyl) and $SO_2NHCOR^X$;

$R^X$ = aryl or heteroaryl as defined above;

Y is selected from:
i) COOH or a pharmaceutically acceptable ester or salt thereof,
ii) $COOR^1$ wherein $R^1$ is a removable carboxy protecting group,
iii) COOM wherein M is an alkali metal, or
iv) $COO^{\ominus}$; provided that when Y is other than iv) a counterion $Z^{\ominus}$ is provided.

2. A compound of Claim 1 wherein L is substituted or unsubstituted branched or linear $C_1$-$C_4$ alkyl.

3. A compound of Claim 2 wherein 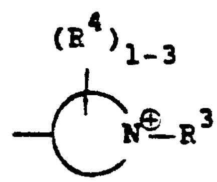 is monocyclic heteroarylium.

4. A compound of Claim 3 wherein 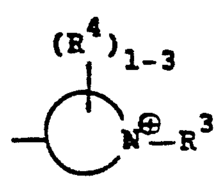 is a pyridinium group.

5. A compound of Claim 4 wherein $R^3$ is an unsubstituted or substituted $C_1$-$C_4$ alkyl group.

6. A compound of Claim 5 wherein L is $-CH_2-$, $-CH(CH_3)-$ or $-(CH_2)_2-$.

7. A compound of Claim 1 wherein the compound is a member selected from the group consisting of:

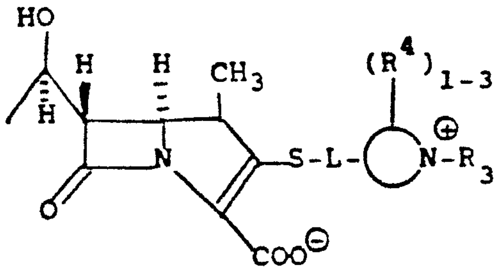

| Compound No. | L | 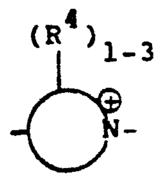 | $R^3$ |
| --- | --- | --- | --- |
| 1 | $-CH_2-$ | 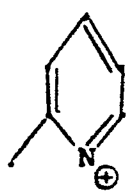 | $CH_2CH_2CH_3$ |
| 2 | $-CH-$<br>$CH_2CH_3$ | " | $CH_2CH_3$ |
| 3 | $-CH_2-$ | 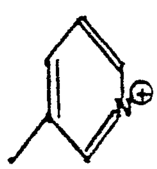 | $CH_2CH_2CH_3$ |
| 4 | " | " | $CH_2CH_3$ |
| 5 | " | 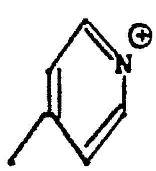 | $CH_2CH_2CH_3$ |

| | | | |
|---|---|---|---|
| 6 | $-CH_2-$ | " | $CH_2CH_3$ |
| 7 | " | 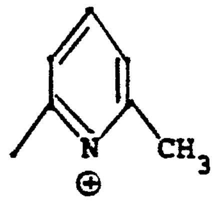 | $CH_3$ |
| 8 | " | 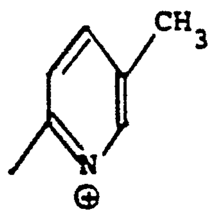 | " |
| 9 | " | 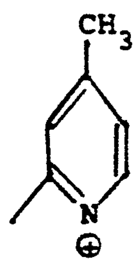 | " |
| 10 | " | 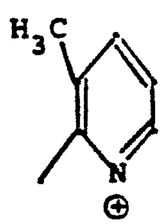 | " |
| 11 | " | 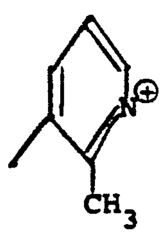 | " |

| | | | |
|---|---|---|---|
| 12 | $-CH_2-$ | 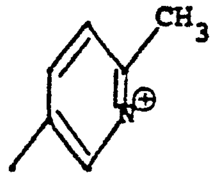 | $CH_3$ |
| 13 | " | 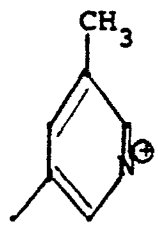 | " |
| 14 | " | 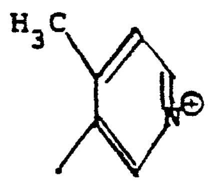 | " |
| 15 | " | 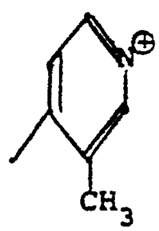 | " |
| 16 | " | 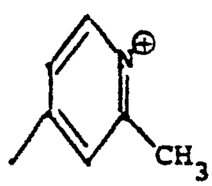 | " |
| 17 | $-CH_2CH_2-$ | 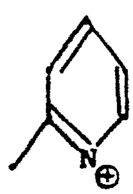 | $CH_2CH_3$ |

| | | | |
|---|---|---|---|
| 18 | $-CH_2CH_2-$ | 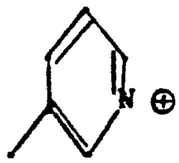 | $CH_3$ |
| 19 | " | 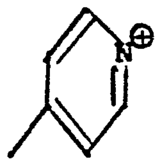 | " |
| 20 | $-\underset{CH_3}{CH}-$ | 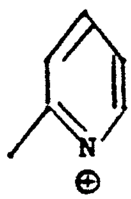 | " |
| 21 | " | 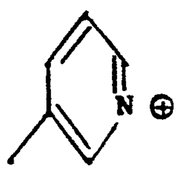 | " |
| 22 | " | 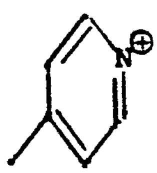 | " |
| 23 | $-CH_2-$ | 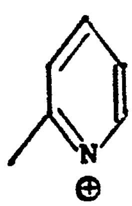 | $ØCH_2$ |

| | | | |
|---|---|---|---|
| 24 | $-CH_2-$ | 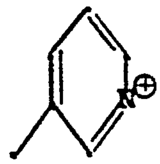 | ØCH$_2$ |
| 25 | " | 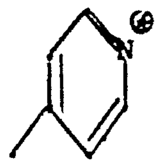 | " |
| 26 | " | 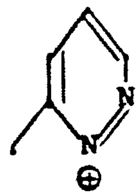 | $CH_3$ |
| 27 | " | 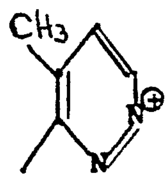 | " |
| 28 | " | 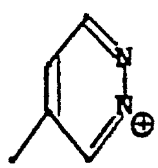 | " |
| 29 | " | 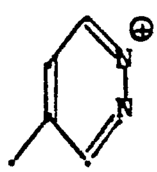 | " |

| | | | |
|---|---|---|---|
| 30 | $-CH_2-$ | 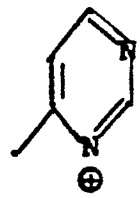 | $CH_3$ |
| 31 | " | 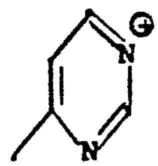 | " |
| 32 | " | 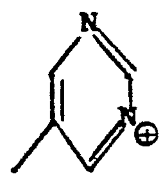 | " |
| 33 | " | 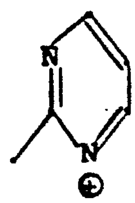 | " |
| 34 | " | 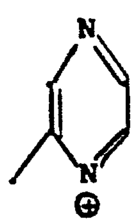 | " |
| 35 | " | 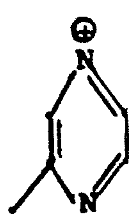 | " |

| | | | |
|---|---|---|---|
| 36 | $-CH_2-$ | 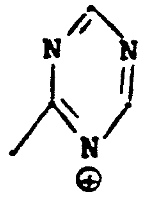 | $CH_3$ |
| 37 | " | 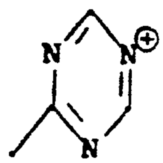 | " |
| 38 | " | 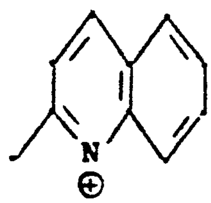 | " |
| 39 | " | 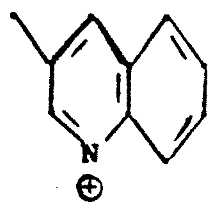 | " |
| 40 | " | 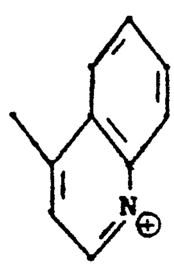 | " |
| 41 | " | 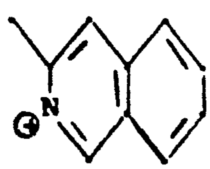 | " |

| | | | |
|---|---|---|---|
| 42 | $-CH_2-$ | 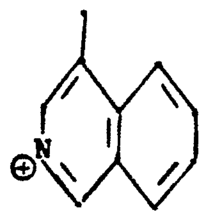 | $CH_3$ |
| 43 | " | 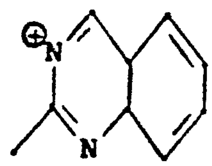 | " |
| 44 | " | 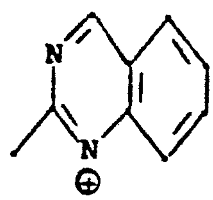 | " |
| 45 | " | 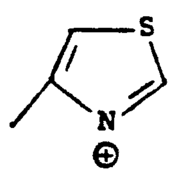 | $CH_2CH_2CH_3$ |
| 46 | " | " | $CH_2CH_3$ |
| 47 | " | 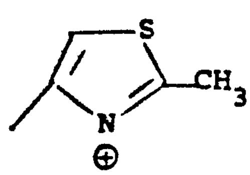 | $CH_3$ |
| 48 | " | 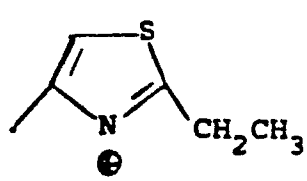 | " |

| | | | |
|---|---|---|---|
| 49 | $-CH_2-$ | 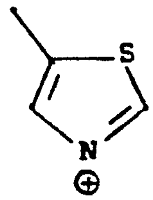 | $CH_3$ |
| 50 | " | 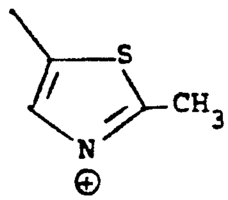 | " |
| 51 | $-\underset{CH_3}{CH}-$ | 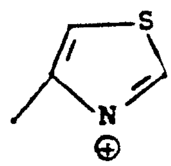 | " |
| 52 | " | 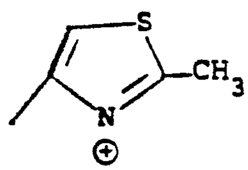 | " |
| 53 | $-CH_2-$ | 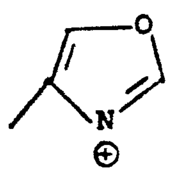 | " |
| 54 | " | " | $CH_2CH_3$ |
| 55 | " | 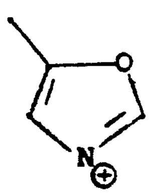 | $CH_3$ |

| | | | |
|---|---|---|---|
| 56 | $-CH_2-$ | 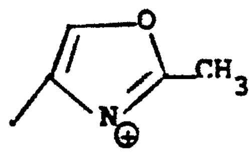 | $CH_3$ |
| 57 | " | 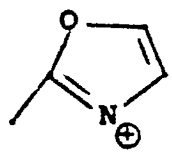 | " |
| 58 | " | 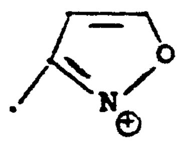 | " |
| 59 | " | 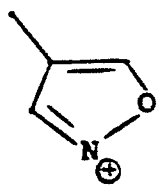 | " |
| 60 | " | 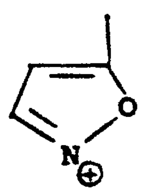 | " |
| 61 | " | 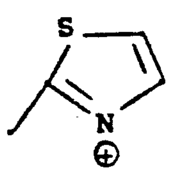 | $CH_2CH_3$ |
| 62 | $-CH_2CH_2-$ | 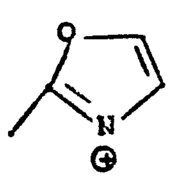 | $CH_3$ |

| | | | |
|---|---|---|---|
| 63 | $-CH_2-$ | 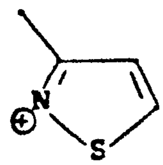 | $CH_3$ |
| 64 | " | 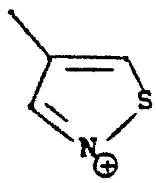 | " |
| 65 | " | 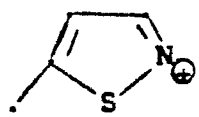 | " |
| 66 | " | 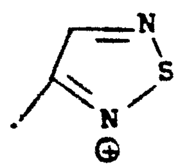 | " |
| 67 | " | 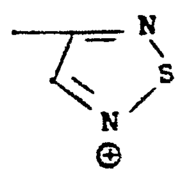 | " |
| 68 | " | 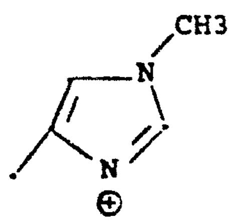 | $CH_3$ |
| 69 | " | " | $-CH_2CH_3$ |

| | | | |
|---|---|---|---|
| 70 | $-CH_2-$ | 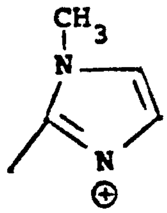 | $-CH_3$ |
| 71 | " | 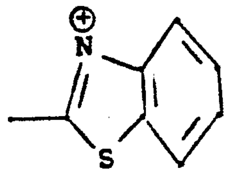 | " |
| 72 | " | 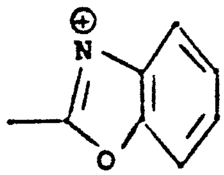 | " |
| 73 | " | 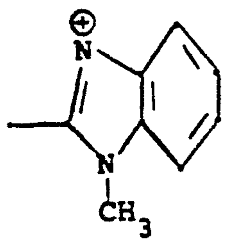 | " |
| 74 | " | 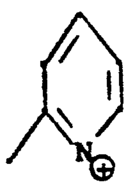 | $CH_2OCH_3$ |
| 75 | " | " | $CH_2CN$ |
| 76 | " | " | $CH_2CO_2H$ |
| 77 | " | " | $CH_2SO_2CH_3$ |

| | | | |
|---|---|---|---|
| 78 | $-CH_2-$ | " | $CH_2\overset{O}{\overset{\uparrow}{P}}(OH)OCH_3$ |
| 79 | " | " | $CH_2SO_3H$ |
| 80 | " | " | $CH_2CONMe_2$ |
| 81 | " | " | $CH_2SOCH_3$ |
| 82 | " | " | $CH_2NMe_2$ |
| 83 | " | " | 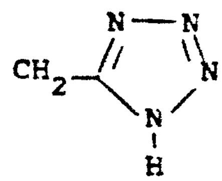 |
| 84 | " | 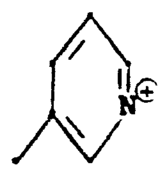 | $CH_2OCH_3$ |
| 85 | " | " | $CH_2SCH_3$ |
| 86 | " | " | $CH_2SOCH_3$ |
| 87 | " | " | $CH_2SO_2CH_3$ |
| 88 | " | " | $CH_2CO_2H$ |
| 89 | " | " | $CH_2CONMe_2$ |

| | | | |
|---|---|---|---|
| 90 | $-CH_2-$ | " | $CH_2\overset{O}{\overset{\uparrow}{P}}(OH)OCH_3$ |
| 91 | " | " | $CH_2SO_3H$ |
| 92 | " | " | $CH_2CN$ |
| 93 | " | " | $CH_2NMe_2$ |
| 94 | " | " | $CH_2CH_2NMe_2$ |
| 95 | " | 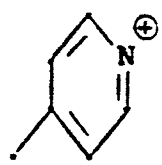 | $CH_2OCH_3$ |
| 96 | " | " | $CH_2NMe_2$ |
| 97 | " | " | $CH_2CH_2NMe_2$ |
| 98 | " | " | $CH_2CN$ |
| 99 | " | " | $CH_2SCH_3$ |
| 100 | " | " | $CH_2SOCH_3$ |
| 101 | " | " | $CH_2SO_2CH_3$ |
| 102 | " | " | $CH_2CO_2H$ |
| 103 | " | " | $CH_2CONMe_2$ |

| | | | |
|---|---|---|---|
| 104 | $-CH_2-$ | " | $CH_2\overset{O}{\overset{\uparrow}{P}}(OH)OCH_3$ |
| 105 | " | " | $CH_2SO_3H$ |
| 106 | " | 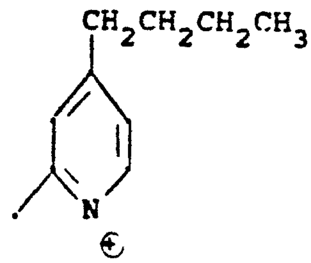 | $CH_3$ |
| 107 | $-CH_2CH_2CH_2-$ | 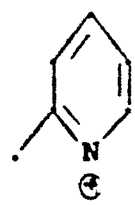 | " |
| 108 | $-CH_2\underset{CH_2OH}{\underset{\vert}{C}}H-$ | 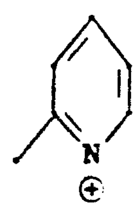 | " |
| 109 | $-CH_2-$ | 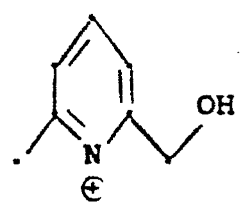 | " |

| | | | |
|---|---|---|---|
| 110 | $-CH_2-$ | 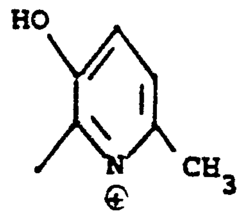 | $CH_3$ |
| 111 | " | 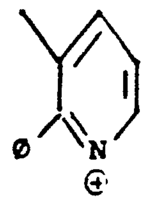 | " |
| 112 | $-CH_2CH_2CH_2-$ | 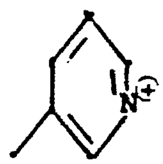 | " |
| 113 | $-CH_2CH_2-$ | 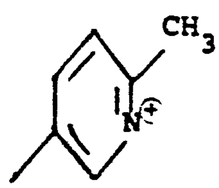 | " |
| 114 | $-CH_2-$ | 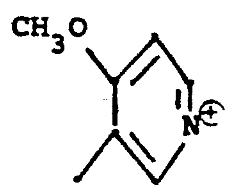 | " |
| 115 | " | 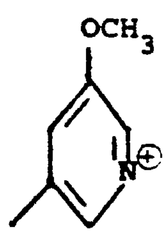 | " |

| | | | |
|---|---|---|---|
| 116 | $-CH_2-$ | 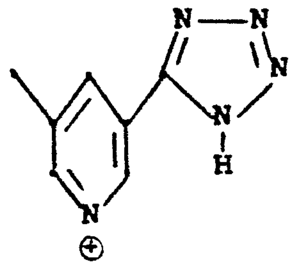 | $CH_3$ |
| 117 | $-CH_2CH_2CH_2-$ | 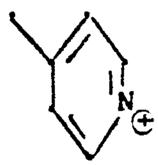 | " |
| 118 | $CH_2$ | 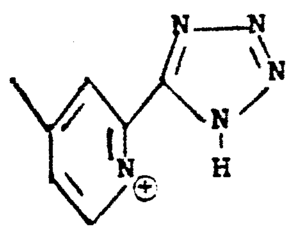 | " |
| 119 | bond | 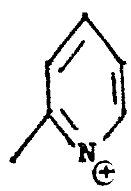 | " |
| 120 | " | 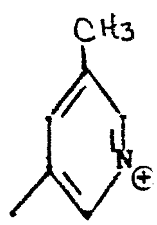 | " |
| 121 | " | 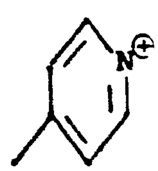 | $CH_3$ |

| | | | |
|---|---|---|---|
| 122 | bond | 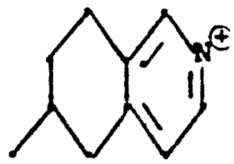 | $CH_3$ |
| 123 | $-CH_2-$ | 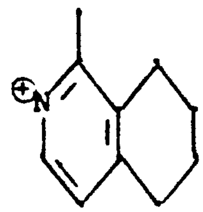 | " |
| 124 | " | 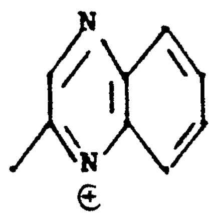 | " |
| 125 | " | 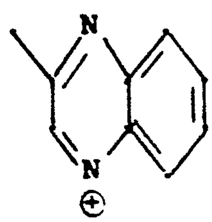 | " |
| 126 | " | 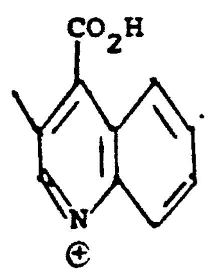 | " |

| | | | |
|---|---|---|---|
| 127 | $-CH_2-$ | 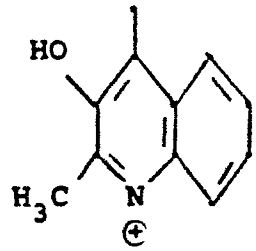 | $CH_3$ |
| 128 | $-CH_2CH_2-$ | 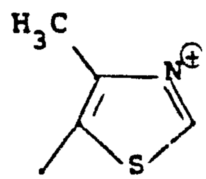 | " |
| 129 | bond | 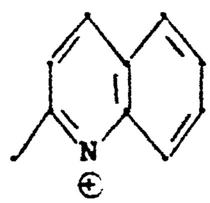 | " |
| 130 | " | 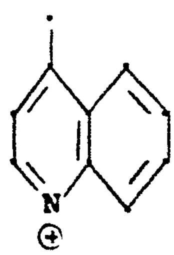 | " |
| 131 | " | 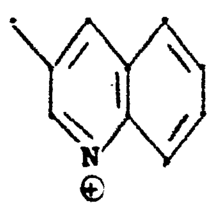 | " |

| | | | |
|---|---|---|---|
| 132 | bond | 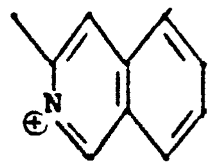 | $CH_3$ |
| 133 | " | 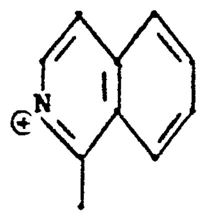 | " |
| 134 | " | 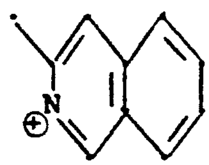 | " |
| 135 | " | 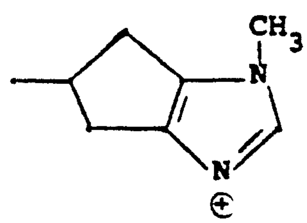 | " |
| 136 | -CH-<br>$CH_3$ | 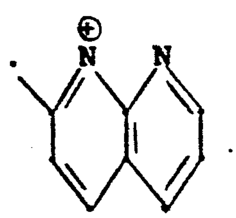 | " |

| | | | |
|---|---|---|---|
| 137 | -$CH_2$- | 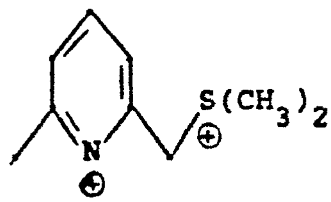 | $CH_3$ |
| 138 | " | 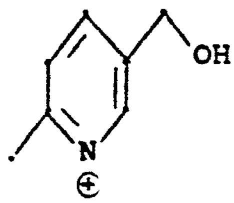 | " |
| 139 | " | 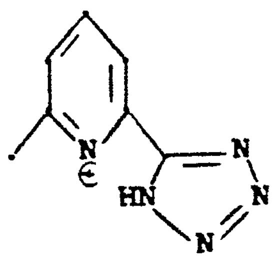 | " |
| 140 | " | 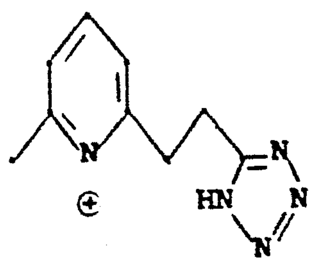 | " |
| 141 | " | 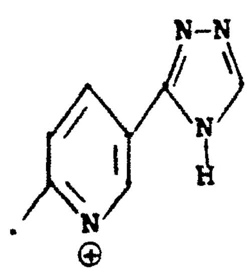 | " |

| | | | |
|---|---|---|---|
| 142 | $CH_2$ | 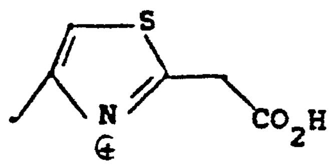 | $CH_3$ |
| 143 | " | 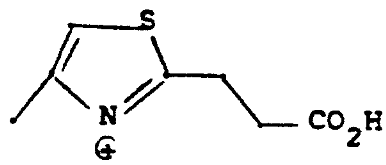 | " |
| 144 | " | 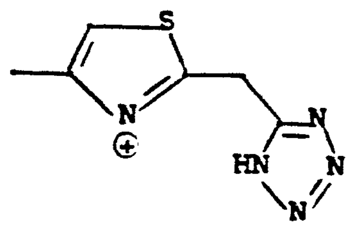 | " |
| 145 | " | 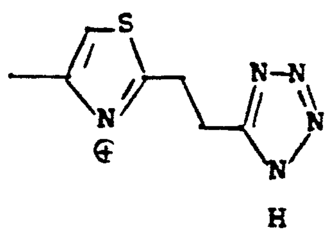 | " |
| 146 | " | 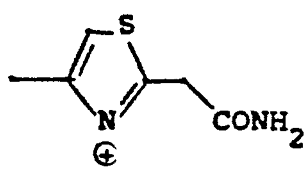 | " |
| 147 | " | 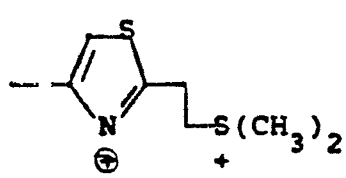 | " |

| | | | |
|---|---|---|---|
| 148 | $-CH_2-$ | 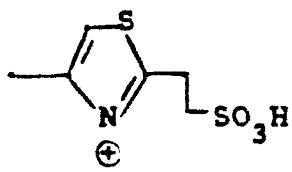 | $CH_3$ |
| 149 | " | 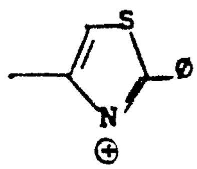 | " |
| 150 | " | 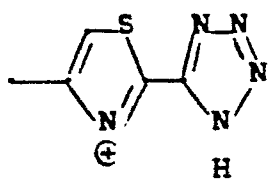 | " |
| 151 | " | 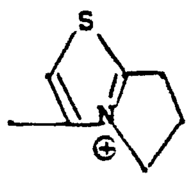 | -- |
| 152 | " | 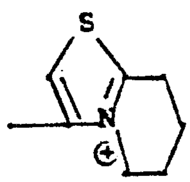 | -- |
| 153 | " | 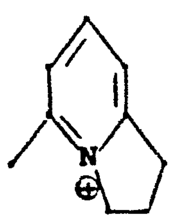 | -- |

| | | | |
|---|---|---|---|
| 154 | $-CH_2-$ | 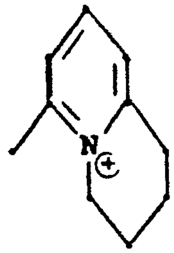 | -- |
| 155 | " | 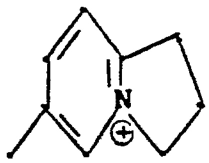 | - |
| 156 | " | 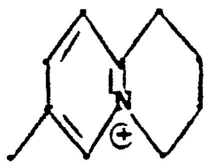 | -- |
| 157 | " | 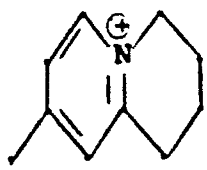 | -- |
| 158 | $-CH(CH_3)CH_2-$ | 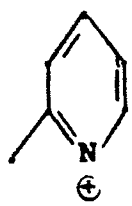 | $CH_3$ |
| 159 | $-CH_2-$ | 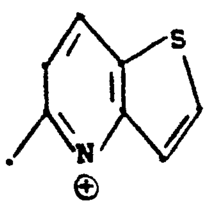 | " |

| | | | |
|---|---|---|---|
| 160 | $-CH_2-$ | 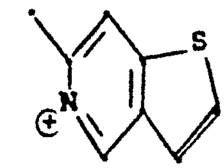 | $CH_3$ |
| 161 | " | 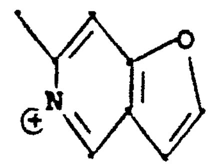 | " |
| 162 | " | 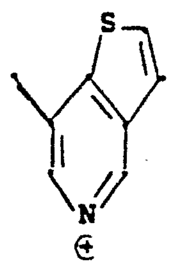 | " |
| 163 | " | 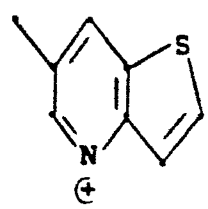 | " |
| 164 | " | 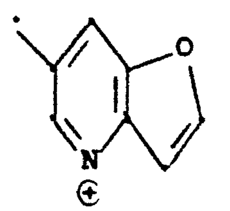 | " |
| 165 | " | 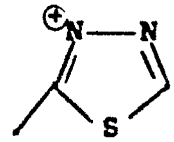 | " |

| | | | |
|---|---|---|---|
| 166 | $-CH_2-$ | 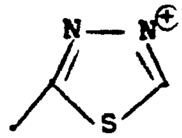 | $CH_3$ |
| 167 | " | 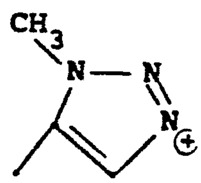 | " |
| 168 | " | 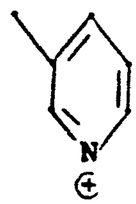 | $CH_2CONH_2$ |
| 169 | " | 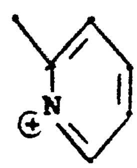 | " |
| 170 | " | 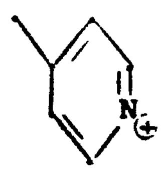 | " |
| 171 | " | 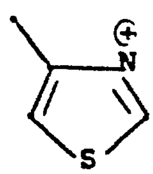 | " |
| 172 | bond | 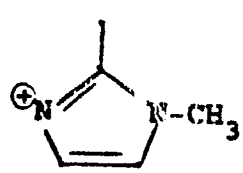 | $CH_3$ |

| | | | |
|---|---|---|---|
| 173 | bond | 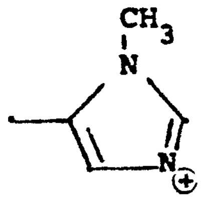 | $CH_3$ |
| 174 | " | 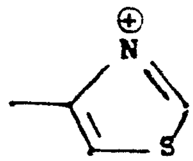 | " |
| 175 | " | 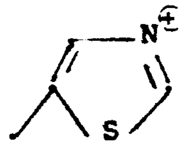 | " |
| 176 | " | 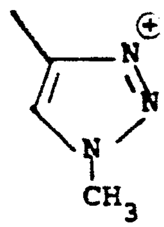 | " |
| 177 | " | 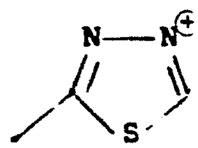 | " |
| 178 | " | 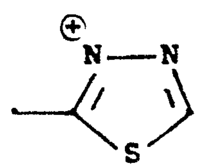 | " |
| 179 | " | 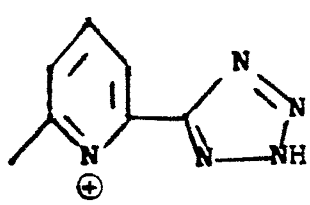 | " |

| | | | |
|---|---|---|---|
| 180 | $-CH_2-$ | 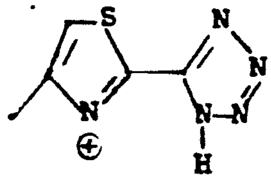 | $CH_3$ |
| 181 | " | 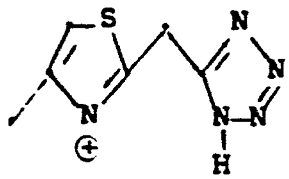 | " |
| 182 | " | 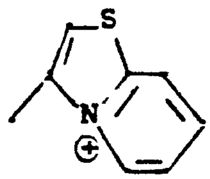 | -- |
| 183 | bond | 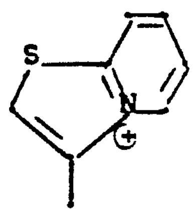 | -- |
| 184 | $CH_2$ | 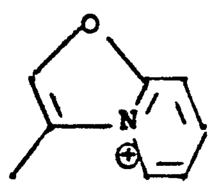 | -- |
| 185 | bond | 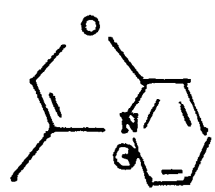 | -- |

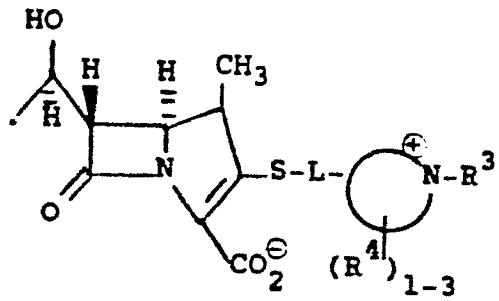

| Compound No. | L | 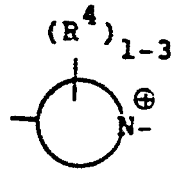 | $R_3$ | $R_4$ |
|---|---|---|---|---|
| 1 | $CH_2$ | 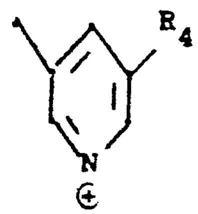 | $CH_3$ | $CO_2H$ |
| 2 | " | " | " | $CONH_2$ |
| 3 | " | " | " | CN |
| 4 | " | " | " | OH |
| 5 | " | " | " | $SO_2NH_2$ |
| 6 | " | " | " | $SO_3H$ |
| 7 | " | " | " | $NMe_2$ |
| 8 | " | " | " | $CONMe_2$ |

| | | | | |
|---|---|---|---|---|
| 9 | " | " | " | $CH_2NMe_2$ |
| 10 | " | " | " | $CH_2CN$ |
| 11 | " | " | " | $CH_2CONH_2$ |
| 12 | " | " | " | $CH_2CO_2H$ |
| 13 | " | " | " | $CH_2SCH_3$ |
| 14 | " | " | " | $CH_2SOCH_3$ |
| 15 | " | " | " | $CH_2SO_2CH_3$ |
| 16 | " | " | " | $SO_2CH_3$ |
| 17 | " | " | " | $SOCH_3$ |
| 18 | " | " | " | 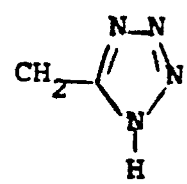 |
| 19 | " | " | " | $CH_2CH_2CO_2H$ |
| 20 | " | " | " | $CH_2SO_3H$ |
| 21 | " | " | " | $CH_2OCH_3$ |
| 22 | " | " | " | 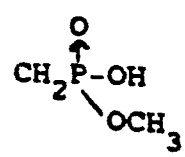 |

| | | | | |
|---|---|---|---|---|
| 23 | " | " | " | $CH_2CH_2SO_3H$ |
| 24 | " | " | " | $CF_3$ |
| 25 | " | " | " | $CH_2O\overset{O}{\overset{\|}{C}}NH_2$ |
| 26 | " | " | " | $CH_2SO_2NH_2$ |
| 27 | " | " | " | Br |
| 28 | " | " | " | Cl |
| 29 | " | " | " | F |
| 30 | " | 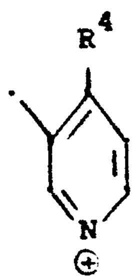 | " | $CO_2H$ |
| 31 | " | " | " | $CONH_2$ |
| 32 | " | " | " | CN |
| 33 | " | " | " | OH |
| 34 | " | " | " | $SONH_2$ |

| | | | | |
|---|---|---|---|---|
| 35 | " | " | " | $SO_3H$ |
| 36 | " | " | " | $NMe_2$ |
| 37 | " | " | " | $CONMe_2$ |
| 38 | " | " | " | $CH_2NMe_2$ |
| 39 | " | " | " | $CH_2CN$ |
| 40 | " | " | " | $CH_2CONH_2$ |
| 41 | " | " | " | $CH_2CO_2H$ |
| 41 | " | " | " | $CH_2SCH_3$ |
| 43 | " | " | " | $CH_2SOCH_3$ |
| 44 | " | " | " | $CH_2SO_2CH_3$ |
| 45 | " | " | " | $SO_2CH_3$ |
| 46 | " | " | " | $SOCH_3$ |
| 47 | " | " | " | 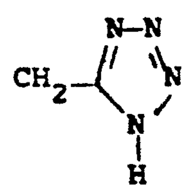 |
| 48 | " | " | " | $CH_2CH_2CO_2H$ |
| 49 | " | " | " | $CH_2SO_3H$ |
| 50 | " | " | " | $CH_2OCH_3$ |

| | | | | |
|---|---|---|---|---|
| 51 | " | " | " | 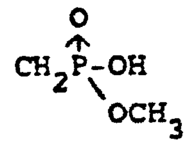 |
| 52 | " | " | " | $CH_2CH_2SO_3H$ |
| 53 | " | " | " | $CF_3$ |
| 54 | " | " | " | 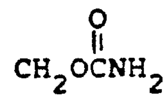 |
| 55 | " | " | " | $CH_2SO_2NH_2$ |
| 56 | " | " | " | $CH_2SO_2NMe_2$ |
| 57 | " | 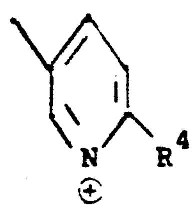 | " | $CO_2H$ |
| 58 | " | " | " | $CONH_2$ |
| 59 | " | " | " | CN |
| 60 | " | " | " | $OCH_3$ |
| 61 | " | " | " | $SO_2NH_2$ |
| 62 | " | " | " | $SO_3H$ |
| 63 | " | " | " | $NMe_2$ |

| | | | | |
|---|---|---|---|---|
| 64 | " | " | " | $CONMe_2$ |
| 65 | " | " | " | $CH_2NMe_2$ |
| 66 | " | " | " | $CH_2CN$ |
| 67 | " | " | " | $CH_2CONH_2$ |
| 68 | " | " | " | $CH_2CO_2H$ |
| 69 | " | " | " | $CH_2SCH_3$ |
| 70 | " | " | " | $CH_2SOCH_3$ |
| 71 | " | " | " | $CH_2SO_2CH_3$ |
| 72 | " | " | " | $SO_2CH_3$ |
| 73 | " | " | " | $SOCH_3$ |
| 74 | " | " | " | $CH_2$ 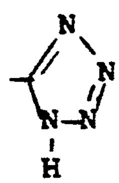 |
| 75 | " | " | " | $CH_2CH_2CO_2H$ |
| 76 | " | " | " | $CH_2SO_3H$ |
| 77 | " | " | " | $CH_2OCH_3$ |

| | | | | |
|---|---|---|---|---|
| 78 | " | " | " | 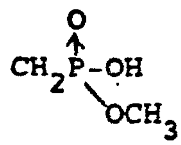 |
| 79 | " | " | " | $CH_2CH_2SO_3H$ |
| 80 | " | " | " | $CF_3$ |
| 81 | " | " | " | 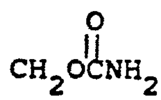 |
| 82 | " | " | " | $CH_2SO_2NH_2$ |
| 83 | " | " | " | $CH_2SO_2NMe_2$ |
| 84 | " | " | " | 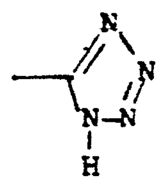 |
| 85 | " | 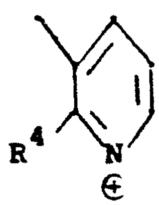 | " | $CO_2H$ |
| 86 | " | " | " | $CONH_2$ |
| 87 | " | " | " | CN |
| 88 | " | " | " | $OCH_3$ |

| | | | | |
|---|---|---|---|---|
| 89 | " | " | " | $SO_2NH_2$ |
| 90 | " | " | " | $SO_3H$ |
| 91 | " | " | " | $NMe_2$ |
| 92 | " | " | " | $CONMe_2$ |
| 93 | " | " | " | $CH_2NMe_2$ |
| 94 | " | " | " | $CH_2CN$ |
| 95 | " | " | " | $CH_2CONH_2$ |
| 96 | " | " | " | $CH_2CO_2H$ |
| 97 | " | " | " | $CH_2SCH_3$ |
| 98 | " | " | " | $CH_2SOCH_3$ |
| 99 | $CH_3$ | " | " | $CH_2SO_2CH_3$ |
| 100 | " | " | " | $SO_2CH_3$ |
| 101 | " | " | " | $SOCH_3$ |
| 102 | " | " | " | $CH_2$ 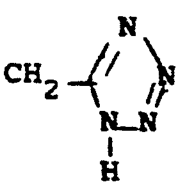 |
| 103 | " | " | " | $CH_2CH_2CO_2H$ |

| | | | | |
|---|---|---|---|---|
| 104 | " | " | " | $CH_2SO_3H$ |
| 105 | " | " | " | $CH_2OCH_3$ |
| 106 | " | " | " | 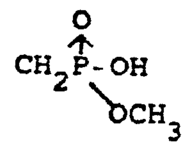 |
| 107 | " | " | " | $CH_2CH_2SO_3H$ |
| 108 | " | " | " | $CF_3$ |
| 109 | " | " | " | 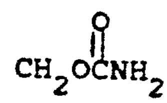 |
| 110 | " | " | " | $CH_2SO_2NH_2$ |
| 111 | " | " | " | $CH_2SO_2NMe_2$ |
| 112 | " | " | " | 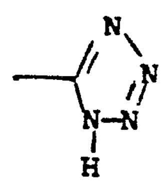 |
| 113 | $-CH_2-$ | 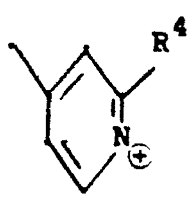 | " | $CO_2H$ |
| 114 | " | " | " | $CONH_2$ |
| 115 | " | " | " | CN |

| | | | | |
|---|---|---|---|---|
| 116 | " | " | " | $OCH_3$ |
| 117 | " | " | " | $SO_2NH_2$ |
| 118 | " | " | " | $SO_3H$ |
| 119 | " | " | " | $NMe_2$ |
| 120 | " | " | " | $CONMe_2$ |
| 121 | " | " | " | $CH_2NMe_2$ |
| 122 | " | " | " | $CH_2CN$ |
| 123 | " | " | " | $CH_2CONH_2$ |
| 124 | " | " | " | $CH_2CO_2H$ |
| 125 | " | " | " | $CH_2SCH_3$ |
| 126 | " | " | " | $CH_2SOCH_3$ |
| 127 | " | " | " | $CH_2SO_2CH_3$ |
| 128 | " | " | " | $SO_2CH_3$ |
| 129 | " | " | " | $SOCH_3$ |
| 130 | " | " | " | 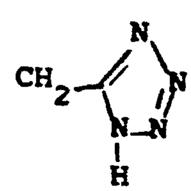 |

| | | | | |
|---|---|---|---|---|
| 131 | " | " | " | $CH_2CH_2CO_2H$ |
| 132 | " | " | " | $CH_2SO_3H$ |
| 133 | " | " | " | $CH_2OCH_3$ |
| 134 | " | " | " | 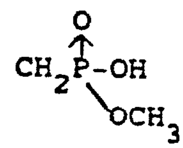 |
| 135 | " | " | " | $CH_2CH_2SO_3H$ |
| 136 | " | " | " | $CF_3$ |
| 137 | " | " | " | 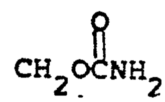 |
| 138 | " | " | " | $CH_2SO_2NH_2$ |
| 139 | " | " | " | $CH_2SO_2NMe_2$ |
| 140 | " | " | " | 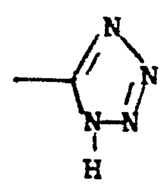 |
| 141 | " | 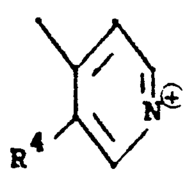 | " | $CO_2H$ |
| 142 | " | " | " | $CONH_2$ |

| | | | | |
|---|---|---|---|---|
| 143 | " | " | " | CN |
| 144 | " | " | " | OH |
| 145 | " | " | " | $OCH_3$ |
| 146 | " | " | " | $SO_2NH_2$ |
| 147 | " | " | " | $SO_3H$ |
| 148 | " | " | " | $NMe_2$ |
| 149 | " | " | " | $CONMe_2$ |
| 150 | " | " | " | $CH_2NMe_2$ |
| 151 | " | " | " | $CH_2CN$ |
| 152 | " | " | " | $CH_2CONH_2$ |
| 153 | " | " | " | $CH_2CO_2H$ |
| 154 | " | " | " | $CH_2SCH_3$ |
| 155 | " | " | " | $CH_2SOCH_3$ |
| 156 | " | " | " | $CH_2SO_2CH_3$ |
| 157 | " | " | " | $SO_2CH_3$ |
| 158 | " | " | " | $SOCH_3$ |

| | | | | |
|---|---|---|---|---|
| 159 | " | " | " | 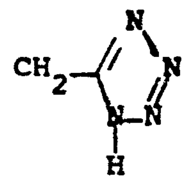 |
| 160 | " | " | " | $CH_2CH_2CO_2H$ |
| 161 | " | " | " | $CH_2SO_3H$ |
| 162 | " | " | " | $CH_2OCH_3$ |
| 163 | " | " | " | 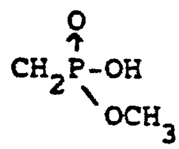 |
| 164 | " | " | " | $CH_2CH_2SO_3H$ |
| 165 | " | " | " | $CF_3$ |
| 166 | " | " | " | 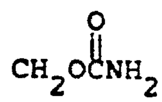 |
| 167 | " | " | " | $CH_2SO_2NH_2$ |
| 168 | " | " | " | $CH_2SO_2NMe_2$ |
| 169 | " | " | " | 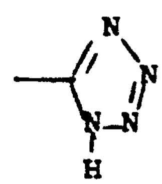 |

| | | | | |
|---|---|---|---|---|
| 170 | " | " | " | F |
| 171 | " | " | " | Cl |
| 172 | " | " | " | Br |
| 173 | " | 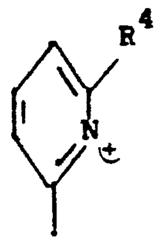 | " | $CO_2H$ |
| 174 | " | " | " | $CONH_2$ |
| 175 | " | " | " | CN |
| 176 | " | " | " | $SO_2NH_2$ |
| 177 | " | " | " | $SO_3H$ |
| 178 | " | " | " | $NMe_2$ |
| 179 | " | " | " | $CONMe_2$ |
| 180 | " | " | " | $CH_2NMe_2$ |
| 181 | " | " | " | $CH_2CN$ |
| 182 | " | " | " | $CH_2CONH_2$ |
| 183 | " | " | " | $CH_2CO_2H$ |

| | | | | |
|---|---|---|---|---|
| 184 | " | " | " | $CH_2SCH_3$ |
| 185 | " | " | " | $CH_2SOCH_3$ |
| 186 | " | " | " | $CH_2SO_2CH_3$ |
| 187 | " | " | " | $SO_2CH_3$ |
| 188 | " | " | " | 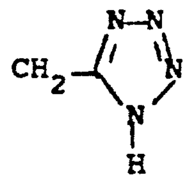 |
| 189 | " | " | " | $CH_2CH_2CO_2H$ |
| 190 | " | " | " | $CH_2SO_3H$ |
| 191 | " | " | " | $CH_2OCH_3$ |
| 192 | " | " | " | 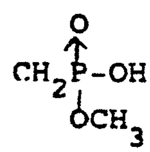 |
| 193 | " | " | " | $CH_2CH_2SO_3H$ |
| 194 | " | " | " | $CF_3$ |
| 195 | " | " | " | 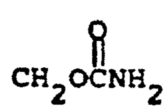 |
| 196 | " | " | " | $CH_2SO_2NH_2$ |

| | | | | |
|---|---|---|---|---|
| 197 | " | 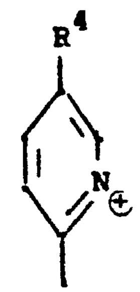 | " | $CO_2H$ |
| 198 | " | " | " | $CONH_2$ |
| 199 | " | " | " | CN |
| 200 | " | " | " | OH |
| 201 | " | " | " | $SO_2NH_2$ |
| 202 | " | " | " | $SO_3H$ |
| 203 | " | " | " | $NMe_2$ |
| 204 | " | " | " | $CONMe_2$ |
| 205 | " | " | " | $CH_2NMe_2$ |
| 206 | " | " | " | $CH_2CN$ |
| 207 | " | " | " | $CH_2CONH_2$ |
| 208 | " | " | " | $CH_2CO_2H$ |
| 209 | " | " | " | $CH_2SCH_3$ |
| 210 | " | " | " | $CH_2SOCH_3$ |

| | | | | |
|---|---|---|---|---|
| 211 | " | " | " | $CH_2SO_2CH_3$ |
| 212 | " | " | " | $SO_2CH_3$ |
| 213 | " | " | " | $SOCH_3$ |
| 214 | " | " | " | 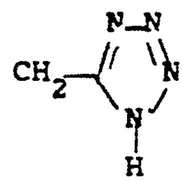 |
| 215 | " | " | " | $CH_2CH_2CO_2H$ |
| 216 | " | " | " | $CH_2SO_3H$ |
| 217 | " | " | " | $CH_2OCH_3$ |
| 218 | " | " | " | 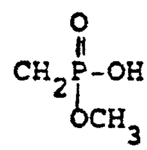 |
| 219 | " | " | " | $CH_2CH_2SO_3H$ |
| 220 | " | " | " | $CF_3$ |
| 221 | " | " | " | 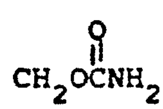 |
| 222 | " | " | " | $CH_2SO_2NH_2$ |
| 223 | " | " | " | Br |

| | | | | |
|---|---|---|---|---|
| 224 | " | " | " | Cl |
| 225 | " | " | " | F |
| 226 | " | 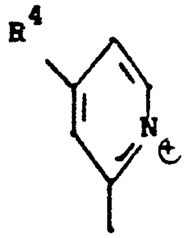 | " | $CO_2H$ |
| 227 | " | " | " | $CONH_2$ |
| 228 | " | " | " | CN |
| 229 | " | " | " | $SO_2NH_2$ |
| 230 | " | " | " | $SO_3H$ |
| 231 | " | " | " | $NMe_2$ |
| 232 | " | " | " | $CONMe_2$ |
| 233 | " | " | " | $CH_2NMe_2$ |
| 234 | " | " | " | $CH_2CN$ |
| 235 | " | " | " | $CH_2CONH_2$ |
| 236 | " | " | " | $CH_2CO_2H$ |
| 237 | " | " | " | $CH_2SCH_3$ |

| | | | | |
|---|---|---|---|---|
| 238 | " | " | " | $CH_2SOCH_3$ |
| 239 | " | " | " | $CH_2SO_2CH_3$ |
| 240 | " | " | " | $SO_2CH_3$ |
| 241 | " | " | " | $SOCH_3$ |
| 242 | " | " | " | 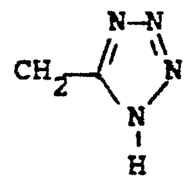 |
| 243 | " | " | " | $CH_2CH_2CO_2H$ |
| 244 | " | " | " | $CH_2SO_3H$ |
| 245 | " | " | " | $CH_2OCH_3$ |
| 246 | " | " | " | 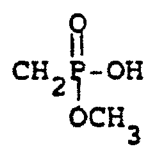 |
| 247 | " | " | " | $CH_2CH_2SO_3H$ |
| 248 | " | " | " | $CF_3$ |
| 249 | " | " | " | 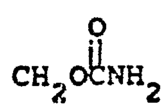 |
| 250 | " | " | " | $CH_2SO_2NH_2$ |

| | | | | |
|---|---|---|---|---|
| 251 | " | 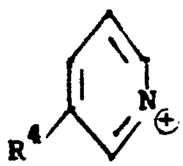 | " | $CO_2H$ |
| 252 | " | " | " | $CONH_2$ |
| 253 | " | " | " | CN |
| 254 | " | " | " | OH |
| 255 | " | " | " | $SO_2NH_2$ |
| 256 | " | " | " | $SO_3H$ |
| 257 | " | " | " | $NMe_2$ |
| 258 | " | " | " | $CONMe_2$ |
| 259 | " | " | " | $CH_2NMe_2$ |
| 260 | " | " | " | $CH_2CN$ |
| 261 | " | " | " | $CH_2CONH_2$ |
| 262 | " | " | " | $CH_2CO_2H$ |
| 263 | " | " | " | $CH_2SCH_3$ |
| 264 | " | " | " | $CH_2SOCH_3$ |

| | | | | |
|---|---|---|---|---|
| 265 | " | " | " | $CH_2SO_2CH_3$ |
| 266 | " | " | " | $SO_2CH_3$ |
| 267 | " | " | " | $SOCH_3$ |
| 268 | " | " | " | 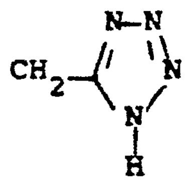 |
| 269 | " | " | " | $CH_2CH_2CO_2H$ |
| 270 | " | " | " | $CH_2SO_3H$ |
| 271 | " | " | " | $CH_2OCH_3$ |
| 272 | " | " | " | 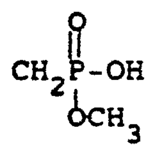 |
| 273 | " | " | " | $CH_2CH_2SO_3H$ |
| 274 | " | " | " | $CF_3$ |
| 275 | " | " | " | 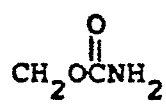 |
| 276 | " | " | " | $CH_2SO_2NH_2$ |

| | | | | |
|---|---|---|---|---|
| 277 | " | " | " | Br |
| 278 | " | " | " | Cl |
| 279 | " | " | " | F |

8. A compound of Claim 1 wherein the compound is a member selected from the group consisting of:

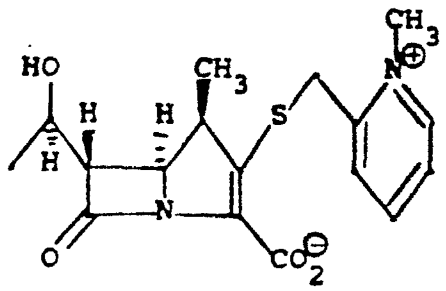

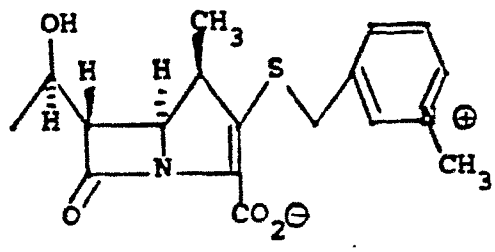

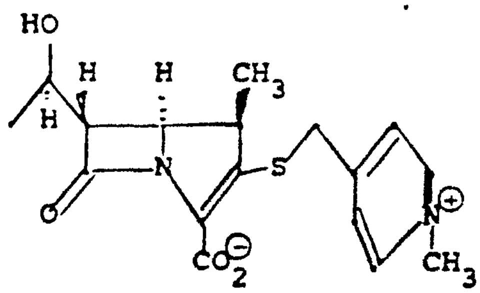

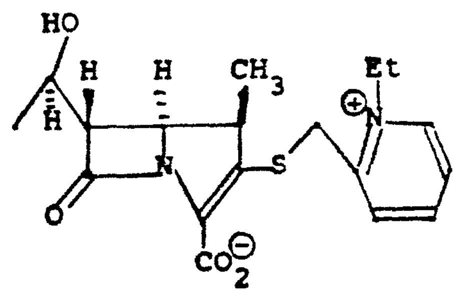

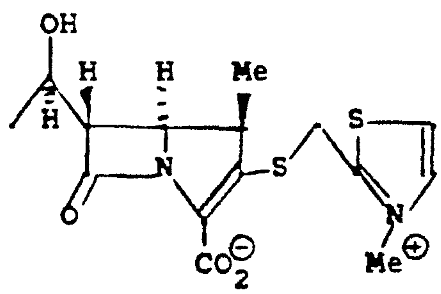

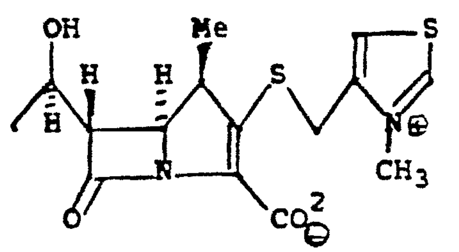

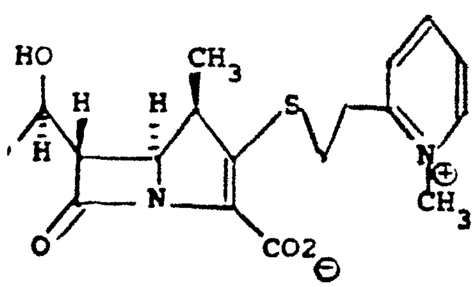

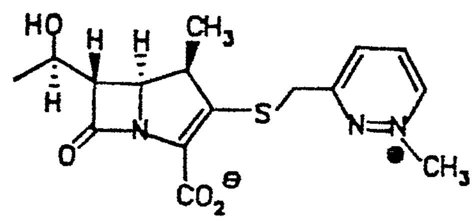

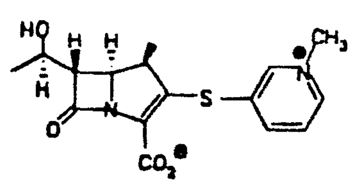

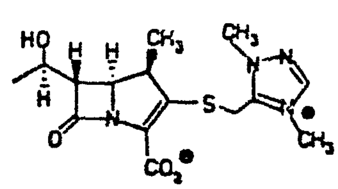

9. The combination of a compound of Claim 1 and a DHP inhibitor.

10. A combination of Claim 10 wherein the DHP inhibitor is 7-(L-2-amino-2-carboxyethylthio)-2-(2,2-dimethylcyclopropanecarboxamide)-2-heptenoic acid.

11. A pharmaceutical composition for antibiotic use comprising an antibacterially effective amount of a compound of Claim 1, an inhibitorily effective amount of a DHP inhibitor, and, optionally, a pharmaceutically acceptable carrier.

12. A pharmaceutical composition according to Claim 12 wherein the DHP inhibitor is 7-(L-2-amino-2-carboxyethylthio)-2-(2,2-dimethylcyclopropanecarboxamide)-2-heptenoic acid.

13. A compound of Claim 1 of the structure:

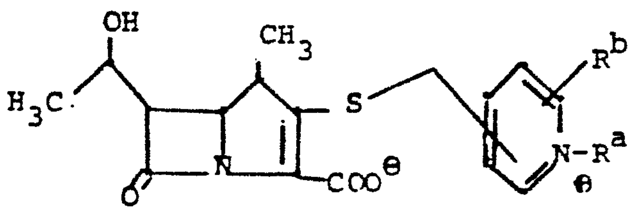

wherein

$R^a$ is $C_{1-4}$ alkyl; or an acidic side-chain of the structure $-(CH_2)_n-X-(CH_2)_m-Y-A$ where:

n = 0-4

m = 0-4

X = $CHR^8$, CH=CH, phenylene ($-C_6H_4-$), NH, N(C1-C4 alkyl), O, S, S=O, C=O, $SO_2$, $SO_2NH$, $CO_2$, CONH, $OCO_2$, OC=O, NHC=O;

$R^8$ = H, O(C1-C4 alkyl), $NH_2$, NH(C1-C4 alkyl), $N(C1\text{-}C4\ alkyl)_2$, CN, $CONH_2$, $CON(C1\text{-}C4\ alkyl)_2$, $CO_2H$, $SO_2NH_2$, $SO_2NH$(C1-C4 alkyl);

Y = single bond, NH, N(C1-C4 alkyl), O, S; and

A = an acidic function .

$R^b$ is hydrogen; cyano; or an acidic side-chain of the structure -A or $-(CH_2)_n-X-(CH_2)_m-Y-A$ where

n = 0-4

m = 0-4

X = $CHR^8$, CH=CH, phenylene ($-C_6H_4-$), NH, N(C1-C4 alkyl), O, S, S=O, C=O, $SO_2$, $SO_2NH$, $CO_2$, CONH, $OCO_2$, OC=O, NHC=O;

$R^8$ = H, O(C1-C4 alkyl), $NH_2$, NH(C1-C4 alkyl), $N(C1\text{-}C4\ alkyl)_2$, CN, $CONH_2$, $CON(C1\text{-}C4\ alkyl)_2$, $CO_2H$, $SO_2NH_2$, $SO_2NH$(C1-C4 alkyl);

Y = single bond, NH, N(C1-C4 alkyl), O, S; and

A = an acidic function.

provided that $R^a$ or $R^b$ must be an acidic side-chain.

14. A compound of Claim 13 wherein the acidic function -A is a member selected from the group consisting essentially of carboxy ($CO_2H$), phosphono [$P=O(OH)_2$], alkylphosphono {P=O(OH)[O(C1-C4 alkyl)]}, alkylphosphinyl [P=O(OH)(C1-C4 alkyl)], substituted phosphoramido [P=O(OH)NH(C1-C4 alkyl) and $P=O(OH)NHR^x$], sulfino ($SO_2H$), sulfo ($SO_3H$), 5-tetrazolyl ($CN_4H$), arylsulfonamido ($SO_2NHR^x$) and acylsulfonamides represented by the structures $CONHSO_2$(C1-C4 alkyl), $CONHSO_2N(C1\text{-}C4\ alkyl)_2$, $SO_2NHCO$(C1-C4 alkyl) and $SO_2NHCOR^x$ wherein $R^x$ = aryl or heteroaryl.

15. A pharmaceutical composition for antibiotic use comprising an antibacterially effective amount of a compound of Claim 14, an inhibitorily effective amount of a DHP inhibitor, and, optionally, a pharmaceutical carrier.

European Patent Office

# EUROPEAN SEARCH REPORT

0170073
Application number

EP 85108132.3

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | DE - A1 - 3 334 937 (BRISTOL-MYERS CO.) * Claims 1,146,159-163,167 * | 1-8,13, 14 | C 07 D 487/04<br>A 61 K 31/40<br>C 07 D 519/00 |
| Y | * Claim 167 * -- | 9-12,15 | |
| D,Y | EP - A1 - 0 007 614 (MERCK & CO. INC.) * Claims 1,7; page 17, lines 5, 6 * -- | 9-12,15 | |
| D,Y | EP - A1 - 0 072 014 (MERCK & CO. INC.) * Claims 1,7,14 * -- | 9-12,15 | |
| A | EP - A1 - 0 021 082 (MERCK & CO. INC.) * Claims 1,3,5 * ---- | 1-8,11, 13-15 | |

TECHNICAL FIELDS SEARCHED (Int Cl 4)

C 07 D 487/00
A 61 K 31/00
C 07 D 519/00

The present search report has been drawn up for all claims

| Place of search | Date of completion of the search | Examiner |
|---|---|---|
| VIENNA | 25-09-1985 | PETROUSEK |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82